# EUROPEAN PATENT SPECIFICATION

(11) **EP 4 101 437 B1**
(45) Date of publication and mention of the grant of the patent: **11.06.2025**
(21) Application number: 21753951.9
(22) Date of filing: 05.02.2021
(51) Int. Cl.: A61K 8/36, A61K 8/362, A61K 8/368, A61K 8/34, A61K 31/19, A61K 31/192, A61P 15/02, A61P 31/00, A61P 31/04, A61K 47/10, A61K 47/12, A61K 47/36

(54) **BACTERIOSTATIC COMPOSITION, PREPARATION METHOD THEREFOR AND USE THEREOF**
BAKTERIOSTATISCHE ZUSAMMENSETZUNG, HERSTELLUNGSVERFAHREN DAFÜR UND VERWENDUNG DAVON
COMPOSITION BACTÉRIOSTATIQUE, SON PROCÉDÉ DE PRÉPARATION ET SON UTILISATION

(30) Priority: 12.02.2020 CN 202010101402; 15.11.2020 CN 202011324292
(43) Date of publication of application: 14.12.2022
(73) Proprietor: Shenzhen Eulikan Biotechnology Co., Ltd., Shenzhen, Guangdong 518100 (CN); Singapore ZE&Z International Pte. Ltd., Singapore 079903 (SG)
(72) Inventor: ZENG, Zhongming, Shenzhen, Guangdong 518000 (CN)
(74) Representative: Ipside
(86) International application number: PCT/CN2021/075655
(87) International publication number: WO 2021/160049

(56) References cited:
- EP-A1- 3 520 785
- WO-A1-2011/041938
- WO-A1-2015/078713
- WO-A1-2015/135470
- WO-A1-2019/147875
- CN-A- 103 648 482
- CN-A- 110 368 324
- JP-A- 2002 322 090
- US-A- 5 670 160
- US-A1- 2006 051 384
- BORGES SANDRA, SILVA JOANA, TEIXEIRA PAULA: "The role of lactobacilli and probiotics in maintaining vaginal health", ARCHIVES OF GYNECOLOGY AND OBSTETRICS, vol. 289, no. 3, 1 March 2014 (2014-03-01), DE, pages 479 - 489, XP055835465, ISSN: 0932-0067, DOI: 10.1007/s00404-013-3064-9

## Description

### Technical Field

The present invention relates to a bacteriostatic composition and a method for inhibiting harmful microorganisms outside the body. The present invention also relates to the combination of fatty acids and dicarboxylic acids and/or salts thereof in a bacteriostatic composition for use in the treatment and/or prevention of vaginal infections by modulating vaginal flora, and a preparation method of the bacteriostatic composition.

### Background

Exposed to the external environment, human skin and mucosa are easily contaminated with pathogens, which may lead to pathogen colonization and infection of skin and mucosa, or lead to the carry and spread of pathogens. Cleaning skin and/or mucosa with bacteriostatic products can effectively reduce the pathogen load on skin and/or mucosa and even kill pathogens, thus helping to prevent the infection and spread of pathogens.

To prevent the spoilage of various personal cleaning and care products, such as skin care products, cosmetics, bath products, and shampoo products, as well as various medical products and pharmaceutical products, as a result of microbial contamination during their storage and use, it is essential to inhibit the growth of contaminated microorganisms and even to kill them, such as inhibiting or killing molds, yeast, *Escherichia coli*, *Pseudomonas aeruginosa*, and *Staphylococcus aureus.*

In particular, the surface of the vaginal mucosa is inhabited by a large number of bacteria, fungi, and other microorganisms. As the most abundant bacteria in vagina of a healthy female, lactobacilli metabolize glycogens in vaginal mucosal epithelial cells, produce acids, maintain the vaginal pH in the range between 3.5-4.5, and produce inhibitory substances such as hydrogen peroxide and bacteriocin against pathogens and opportunistic pathogens. They are therefore beneficial and are known as "normal vaginal flora".

The vagina is also inhabited by *Gardnerella*, *Prevotella*, *Mobiluncus*, *Escherichia coli*, *Staphylococcus*, *Candida*, and the like, which are not pathogenic when in small numbers, but may produce harmful metabolites, toxins, etc. when in large numbers thus resulting in pathological changes and diseases in human body. These microorganisms are therefore called "opportunistic pathogens". Among Staphylococcus, the pathogenicity of *Staphylococcus aureus* is relatively high and representative. Among Candida infections, *Candida albicans* infection is the most common and representative, accounting for more than 70% of total vaginal and/or vulvar *Candida* infections.

When the vaginal flora is abnormal and lactobacilli exist in low numbers, the vagina is not only inhabited by a higher number of opportunistic pathogens such as *Gardnerella*, but also has a decreased resistance to pathogens of high toxicity and pathogenicity. The risk of communicable diseases of the reproductive tract, such as those caused by gonococci, trichomonas, chlamydia, mycoplasmas, and viruses such as HIV and HPV, and the like, then increases.

Currently, the antibacterial therapy remains the primary treatment for vaginal microbial diseases. Although antibacterial therapy can inhibit or kill pathogenic bacteria, it often inhibits or kills beneficial lactobacilli also and results in the reduction of vaginal resistance to infections. Thus, recurrent or persistent vaginal infections occur. It is a hot topic in medical research how to protect the beneficial bacteria in vagina during the course of antibacterial therapy and improve the efficacy of the prevention and treatment.

Therefore, there is a huge practical demand for a safe and effective bacteriostatic product, which is not only suitable for use in cleaning, hygiene, and bacteriostasis of human skin and mucosa, in the antisepsis of personal cleaning and care products such as bath products, skin care products, and cosmetics, and in the antisepsis of medical products, pharmaceutical products, and the like, but also can be used to inhibit abnormal vaginal flora, to restore and/or maintain vaginal lactobacilli, to restore and/or maintain normal vaginal flora, and to restore and/or maintain normal vaginal microecology. Documents EP3520785 and WO2015078713 are related to bacteriostatic compositions directed to female care including a fatty acid or lactic acid, an aromatic alcohol, and sodium benzoate. Documents US2006/051384 and US5670160 are related to bacteriostatic compositions not directed to female care.

The information in the Background section is intended only to describe the general context of the present invention and shall not be considered as an acknowledgment or in any way as an implication that such information constitutes the prior art known to those of ordinary skill in the art.

### Summary of the Invention

The present invention is set out in the appended set of claims. The references to the methods of treatments in this description, the in-vivo experiments I to VI and clinical observation I in the examples of the application are to be interpreted as references to compounds, pharmaceutical compositions and medicaments of the present invention for use in those methods.

An objective of the present invention is to provide a bacteriostatic composition.

Another objective of the present invention is to provide a method for inhibiting harmful microorganisms outside the body.

A further objective of the present invention is to provide a composition for use in modulating vaginal flora, referring to inhibiting abnormal vaginal flora, and/or restoring and/or maintaining vaginal lactobacilli.

And a further objective of the present invention is to provide a method for preparing the bacteriostatic composition.

The present invention provides a bacteriostatic composition, wherein the composition comprises the following ingredients:
(1) one or more of the fatty acids and/or salts thereof selected from the group consisting of acetic acid, propionic acid, butyric acid, isobutyric acid, valeric acid, hexanoic acid, heptanoic acid, caprylic acid, nonanoic acid, capric acid, undecylic acid, undecylenic acid, lauric acid, and salts thereof, preferably selected from the group consisting of propionic acid, butyric acid, hexanoic acid, caprylic acid, capric acid, undecylic acid, and salts thereof; the total content of the ingredients (1) as described, calculated as fatty acid, is in the range of 0.001-3.00% (w/w);
(2) one or more of the dicarboxylic acids and/or salts thereof selected from the group consisting of glutaric acid, adipic acid, pimelic acid, and salts thereof, preferably adipic acid, and salt thereof; he total content of the ingredients (2) as described, calculated as dicarboxylic acid, is in the range of 0.05-5.00% (w/w);
(3) one or more of the aromatic alcohols selected from the group consisting of benzyl alcohol, phenethyl alcohol, phenoxyethanol, and cinnamyl alcohol, preferably selected from the group consisting of phenethyl alcohol and cinnamyl alcohol; the total content of the ingredients (3) as described, is in the range of 0.03-1.00% (w/w);
(4) one or more of the aromatic carboxylic acids and/or salts thereof selected from the group consisting of benzoic acid, p-hydroxybenzoic acid, p-methoxybenzoic acid, salicylic acid, cinnamic acid, gentianic acid, caffeic acid, and salts thereof, preferably selected from the group consisting of benzoic acid, cinnamic acid, p-hydroxybenzoic acid, and salts thereof; the total content of the ingredients (4) as described, calculated as aromatic carboxylic acid, is in the range of 0.03-1.00% (w/w);

The bacteriostatic composition is in one of the following dosage forms: aqueous solutions, water-soluble gels, foams, sprays, ointments, powders, films, capsules, suppositories, tablets, preferably aqueous solutions, water-soluble gels, foams, sprays, or ointments.

In some embodiments, the total content of one or more of the fatty acids and/or salts thereof, calculated as fatty acid, is preferably in the range of 0.001-2.00% (w/w), more preferably in the range of 0.001-1.50% (w/w), and the most preferably in the range of 0.002%-1.25% (w/w); and/or the total content of one or more of the dicarboxylic acids and/or salts thereof, calculated as dicarboxylic acid, is preferably in the range of 0.10-3.50% (w/w), more preferably in the range of 0.50-2.50% (w/w), and the most preferably in the range of 0.50-1.50% (w/w); and/or the total content of one or more of the aromatic alcohols is preferably in the range of 0.03-0.70% (w/w), more preferably in the range of 0.05-0.60% (w/w), and the most preferably in the range of 0.08-0.50% (w/w); and/or the total content of one or more of the aromatic carboxylic acids and/or salts thereof, calculated as aromatic carboxylic acids, is preferably in the range of 0.03-0.50% (w/w), more preferably in the range of 0.05-0.25% (w/w), and the most preferably in the range of 0.08-0.20% (w/w).

In some embodiments, the bacteriostatic composition can further comprise one or more of the dicarboxylic acids, polybasic carboxylic acids, and salts thereof selected from the group consisting of malic acid, citric acid, succinic acid, tartaric acid, maleic acid, isocitric acid, suberic acid, azelaic acid, sebacic acid, and salts thereof, preferably selected from the group consisting of malic acid, citric acid, succinic acid, and salts thereof; the total content of one or more of the dicarboxylic acids, polybasic carboxylic acids, and salts thereof as described is in the range of 0.10-2.50% (w/w). Wherein the acids and/or salts thereof have a buffering effect, which improves the pH stability of the bacteriostatic composition in the present disclosure, and further strengthens the modulating effect of the bacteriostatic composition on the pH of human skin and/or mucosa.

In some embodiments, the bacteriostatic composition can optionally include one or more of the monosaccharides and/or oligosaccharides and/or polysaccharides selected from the group consisting of glucose, fructose, mannose, galactose, maltose, isomaltose, sucrose, isomaltulose, lactose, lactulose, trehalose, cellobiose, melibiose, gentiobiose, 1-kestose, nystose, 1F-fructofuranosylnystose, isomaltotriose, isomaltotetraose, isomaltopentaose, gentiooligosaccharide, raffinose, panose, maltooligosaccharide, palatinose-oligosaccharide, oligofructose, glucomannan, galactooligosaccharide, dextrin, starch, and glycogen; the total content of which is in the range of 0.01-20.00% (w/w). The preferable saccharides are selected from the group consisting of isomaltulose, lactose, lactulose, maltose, isomaltose, trehalose, glycogen, and mixtures thereof; the total content of which is in the range of 0.10-2.00% (w/w). The saccharide-containing bacteriostatic composition in the present disclosure can not only inhibit *Candida, Staphylococcus aureus, Escherichia coli, Pseudomonas aeruginosa, Gardnerella, Prevotella*, *Mobiluncus*, *Aspergillus niger*, abnormal flora of skin and/or mucosa, and the like, but also can restore and/or maintain lactic acid bacteria on skin and/or mucosa, and restore and/or maintain the acidity of skin and/or mucosa. Therefore, it is suitable for use in the prevention and/or treatment of the decrease of lactic acid bacteria on skin and/or mucosa and the imbalance of skin and/or mucosa flora, such as the use in the prevention and/or treatment of bacterial vaginosis, aerobic vaginitis, or atrophic vaginitis, or the use in the adjuvant treatment of vaginitis, or the use in the recovery after vaginitis treatment, or the use in the treatment of reproductive tract infections, and the like.

In some embodiments, the bacteriostatic composition can optionally include one or more of the amino acids and/or salts thereof selected from the group consisting of L-glutamic acid, glutamine, L-aspartic acid, asparagine, leucine, isoleucine, phenylalanine, valine, proline, threonine, and salts thereof; the total content of which is in the range of 0.10-6.00% (w/w); the preferable amino acids are glutamic acid, aspartic acid, salts thereof, and mixtures thereof; the total content of which is in the range of 0.50-3.00% (w/w). The bacteriostatic composition in the present disclosure containing amino acids and/or salts thereof can reduce acids production from lactic acid bacteria, thus decreasing the acidity of skin and/or mucosa. It is suitable for use in the modulation of the microenvironment of skin and/or mucosa, such as the use in the treatment or adjuvant treatment of cytolytic vaginosis, vulvovaginal candidiasis, and the like.

In some embodiments, the bacteriostatic composition can optionally include one or more of monoterpene and/or sesquiterpene compounds, including but not limited to citronellol, linalool, geraniol, nerol, eucalyptol, terpineol, carveol, menthol, and lavandulol; the total content of the monoterpene and/or sesquiterpene compounds as described is in the range of 0.001-0.50% (w/w). The monoterpene and/or sesquiterpene compounds may enhance the bacteriostatic effect of the bacteriostatic composition as well as provide a fragrance for the composition.

In some embodiments, the bacteriostatic composition can optionally include one or more of plant aromatic oils, including but not limited to rose essential oil, clove oil, red thyme oil, lavender oil, peppermint oil, artemisia leaf oil, eucalyptus oil, sassafras oil, litsea cubeba oil, cinnamon essential oil, laurel leaf oil, and thyme oil; the total content of the plant aromatic oils as described is in the range of 0.0001-0.05% (w/w). The plant aromatic oils can enhance the bacteriostatic effect of the composition, as well as provide a fragrance for the composition.

In some embodiments, the bacteriostatic composition can optionally include one or more of the vitamins selected from the group consisting of vitamin A, vitamin C, vitamin D, and vitamin E; the total content of which is in the range of 0.001-0.50% (w/w). The vitamins can be highly pure vitamins or the vitamins contained in plant extracts. The vitamins as described have effects of antioxidation, promoting the growth of skin cells, modulating the immune function, or maintaining the integrity of mucosal epithelial cells, etc., and can enhance the stability of the bacteriostatic composition, or protect human skin and/or mucosa.

In some embodiments, the bacteriostatic composition can optionally include one or more of the ingredients selected from the group consisting of dehydroacetic acid, sodium dehydroacetate, sorbic acid, potassium sorbate, sodium sorbate, natamycin, bergenin, tropolone, cinnamaldehyde, pseudolaric acid, chlorogenic acid, 1,2-pentanediol, 1,2-hexanediol, 1,6-hexanediol, 1,2-octanediol, 1,2-decanediol, p-hydroxyacetophenone, 2,4-dihydroxyacetophenone, methyl p-hydroxybenzoate, ethyl p-hydroxybenzoate, propyl p-hydroxybenzoate, lysozyme, glycerol monocaprylate, glycerol monodecanoate, and glycerol monolaurate. The ingredients as described are used to further enhance the bacteriostatic or antibacterial effect of the bacteriostatic composition in the present disclosure against *Candida, Staphylococcus aureus, Escherichia coli, Pseudomonas aeruginosa, Gardnerella, Prevotella, Mobiluncus, Aspergillus niger,* abnormal flora of skin and/or mucosa, and the like, and other harmful microorganisms including but not limited to viruses such as HIV and HPV.

In some embodiments, the bacteriostatic composition further comprises one or more of antibacterial drugs, including but not limited to nitroimidazoles such as metronidazole, tinidazole, and ornidazole; aminoglycosides such as gentamicin, tobramycin, amikacin, sisomicin, and netilmicin; quinolones such as ciprofloxacin, ofloxacin, and levofloxacin; furanes such as nifuratel, nifuroxime, furacilin, furazolidone, and furantoin; sulfonamides such as silver sulfadiazine and sodium sulfacetamide; pyrroles such as clotrimazole, fluconazole, miconazole, and ketoconazole; acrylamides such as naftifine and terbinafine; polyenes such as amphotericin B, nystatin, levorin, and natamycin; preferably metronidazole, nifuratel, clotrimazole, or mixtures thereof. The bacteriostatic composition in the present disclosure containing antibacterial drugs is suitable for skin and/or mucosa use, such as for the prevention and/or treatment of bacterial and/or fungal infections of skin, the prevention and/or treatment of bacterial or fungal infections of the oral mucosa, and the prevention and/or treatment of vaginal mucosa infections such as bacterial vaginosis, aerobic vaginitis, and vulvovaginal candidiasis.

In some embodiments, the bacteriostatic composition as described is in one of the following dosage forms: aqueous solutions, water-soluble gels, foams, sprays, or ointments; the pH value of the aqueous solutions, water-soluble gels, foams, sprays, or ointments is in the range of 3.1-4.8, preferably in the range of 3.6-4.6, and more preferably in the range of 3.8-4.4.

In some embodiments, the bacteriostatic composition is a water-soluble gel. The water-soluble gel comprises one or more of non-flowable, viscous, and water-soluble colloidal excipients, including but not limited to xanthan gum, carbomer, polycarbophil, dextran, glucomannan, tragacanth gum, gummitragacanthae, methyl cellulose (MC), carboxymethyl cellulose (CMC), hydroxyethyl cellulose (HEC), hydroxyethyl methyl cellulose (HEMC), and hydroxypropyl methyl cellulose (HPMC), the preferred are xanthan gum and carbomer.

The bacteriostatic composition in the present disclosure has an inhibitory effect on harmful microorganisms, which is referred to but not limited to *Candida*, *Staphylococcus aureus*, *Escherichia coli*, *Pseudomonas aeruginosa*, *Gardnerella*, *Prevotella*, *Mobiluncus*, *Aspergillus niger*, abnormal flora of skin and/or mucosa, as well as various other pathogenic or opportunistically pathogenic aerobic bacteria, facultative bacteria, anaerobic bacteria, molds, and viruses such as HPV and HIV.

Therefore, the bacteriostatic composition in the present disclosure can be used for the cleaning and/or bacteriostasis and/or disinfection of human skin and/or mucosa such as vaginal mucosa, for the antisepsis of various personal cleaning and care products such as bath products, shampoo products, skin care products and cosmetics, or for the antisepsis of medical products, pharmaceutical products, and the like.

In some embodiments, the bacteriostatic composition in the present disclosure can be a therapeutic product, an active ingredient thereof, and an antiseptic thereof; the therapeutic product form includes but are not limited to drugs, disinfectants, topical microbicides, antibacterial agents, bacteriostatic agents, microecological modulators, flora modulators, microenvironment modulators, microbial modulators, disposable medical supplies, and the like, or the components of medical devices, the components of pharmaceutical devices, the components of disinfection devices, and the components of devices for vagina use.

In some embodiments, the bacteriostatic composition in the present disclosure can be a non-therapeutic product, an active ingredient thereof, and an antiseptic thereof; the non-therapeutic product form is one of the following group: health care products, hygiene products, personal cleaning and care products, cosmetics, disposable hygiene products, cleaning products, daily necessities, microecological care products, deodorants, lubricants, humectants, lotions, cleaning agents, body care products, antipruritic agents, refreshing agents, and the components of sanitary napkins, sanitary pads, and tampons.

In some embodiments, the bacteriostatic composition of the present invention is a vaginal bacteriostatic composition, wherein the vaginal bacteriostatic composition comprises:
(1) one or more of the fatty acids and/or salts thereof selected from the group consisting of acetic acid, propionic acid, butyric acid, valeric acid, hexanoic acid, heptanoic acid, caprylic acid, nonanoic acid, capric acid, undecylic acid, undecylenic acid, lauric acid, and salts thereof; the total content of which, calculated as fatty acid, is in the range of 0.001-2.00% (w/w). The preferred fatty acids and/or salts are selected from the group consisting of propionic acid, butyric acid, hexanoic acid, caprylic acid, capric acid, undecylic acid, salts thereof, and mixtures thereof; the total content of which, calculated as fatty acid, is in the range of 0.001-1.50% (w/w), more preferably in the range of 0.002-1.25% (w/w);
(2) one or more of the dicarboxylic acids and/or salts thereof selected from the group consisting of glutaric acid, adipic acid, pimelic acid, and salts thereof; the total content of which, calculated as dicarboxylic acids, is in the range of 0.50-2.50% (w/w). The preferred is adipic acid and/or salt thereof; the total content of which, calculated as adipic acid, is in the range of 0.50-1.50% (w/w);
(3) one or more of the aromatic alcohols selected from the group consisting of benzyl alcohol, phenethyl alcohol, phenoxyethanol, and cinnamyl alcohol; the total content of which is in the range of 0.05-0.60% (w/w). The preferred aromatic alcohols are phenethyl alcohol, cinnamic alcohol, and mixtures thereof; the total content of which is in the range of 0.08-0.50% (w/w);
(4) one or more of the aromatic carboxylic acids and/or salts thereof selected from the group consisting of benzoic acid, p-hydroxybenzoic acid, p-methoxybenzoic acid, salicylic acid, cinnamic acid, gentianic acid, caffeic acid, and salts thereof; the total content of which, calculated as aromatic carboxylic acid, is in the range of 0.05-0.25% (w/w). The preferred aromatic carboxylic acids and/or salts thereof are selected from the group consisting of benzoic acid, cinnamic acid, p-hydroxybenzoic acid, salts thereof, and mixtures thereof; the total content of which, calculated as aromatic carboxylic acid, is in the range of 0.08-0.20% (w/w);

The vaginal bacteriostatic composition as described is in one of the following dosage forms: aqueous solutions, water-soluble gels, foams, sprays, ointments, powders, films, capsules, suppositories, or tablets, preferably aqueous solutions, water-soluble gels, foams, sprays, or ointments.

In some embodiments, the vaginal bacteriostatic composition in the present disclosure further comprises one or more of the estrogens selected from the group consisting of diethylstilbestrol, hexoestrol, estradiol, estrone, estriol, nilestriol, ethinyloestradiol, quinestrol, mestranol, and promestriene, preferably, estriol and promestriene; the total content of which is in the range of 0.001-1.00% (w/w). The vaginal bacteriostatic composition in the present disclosure containing estrogens can promote the glycogen synthesis in vaginal mucosal epithelial cells, and promote the growth of beneficial lactobacilli. It is especially suitable for use in menopause, postmenopausal, and postpartum.

In some embodiments, the vaginal bacteriostatic composition in the present disclosure can further compriseone or more of the phytoestrogens selected from the group consisting of daidzin, daidzein, glycitein, puerarin, coumestrol, genistein, equol, apigenin, genistin, genisteol, biochanin, coumestrol, formononetin, resveratrol, secoisolariciresinol, and lignan; the total content of which is in the range of 0.001-1.00% (w/w). The vaginal bacteriostatic composition in the present disclosure containing phytoestrogens can promote glycogen synthesis in vaginal mucosal epithelial cells, and promote the growth of beneficial lactobacilli. It is especially suitable for use in menopause, postmenopausal, and postpartum.

According to practical needs, the vaginal bacteriostatic composition in the present disclosure can optionally contain different excipients for different dosage forms such as water, xanthan gum, or carbomer. It can also optionally include one or more of the aforementioned ingredients further, such as the dicarboxylic acid(s), polybasic carboxylic acid(s), and salt(s) thereof, e.g., malic acid, citric acid, succinic acid, and salts thereof, in a total content of 0.10-2.50% (w/w); and/or the antibacterial or bacteriostatic agent(s) such as dehydroacetic acid and glycerol monocaprylate; and/or the saccharide(s) such as isomaltulose, lactose, and maltose, in a total content of 0.01-20.00% (w/w); and/or amino acid(s) such as glutamic acid, aspartic acid, and salts thereof, in a total content of 0.10-6.00% (w/w); and/or the monoterpene or sesquiterpene compound(s) such as citronellol and linalool, in a total content of 0.001-0.50% (w/w); and/or the plant aromatic oil(s) such as rose essential oil and clove oil, in a total content of 0.0001-0.05% (w/w); and/or the vitamin(s) such as vitamin A, and vitamin C, in a total content of 0.001-0.50% (w/w); and/or antibacterial drug(s) such as metronidazole, nifuratel, and clotrimazole.

Having an inhibitory effect on abnormal vaginal flora but an effect of restoring and/or maintaining and/or promoting vaginal lactobacilli, the vaginal bacteriostatic composition in the present disclosure can be used to restore and/or maintain normal vaginal flora, to restore and/or maintain normal vaginal microecology, and to restore and/or maintain normal vaginal acidity.

Therefore, the vaginal bacteriostatic composition in the present disclosure can be used to clean and take care of vagina and/or vulva, and/or to reduce and/or eliminate vaginal pruritus, and/or soreness, and/or dryness, and/or irritation, and/or dyspareunia, and/or to reduce and/or eliminate abnormal vaginal discharge, and/or unpleasant odor of vaginal discharge. It can also be used for the prevention and/or treatment and/or adjuvant treatment of the imbalance of vaginal flora, and/or bacterial vaginosis, and/or aerobic vaginitis, and/or cytolytic vaginosis, and/or vulvovaginal Candidiasis, and/or atrophic vaginitis, etc.

The present invention also provides a method for inhibiting harmful microorganisms outside the body, wherein the method includes the steps of using a bacteriostatic composition according to the invention. Therefore, the bacteriostatic composition as described in the method for inhibiting harmful microorganisms outside the body in the present disclosure can be used for the antisepsis of various personal cleaning and care products such as bath products, shampoo products, skin care products, and cosmetics, or for the antisepsis of medical products, pharmaceutical products, and the like.

In some embodiments, the bacteriostatic composition as described in the method for inhibiting harmful microorganisms outside the body in the present disclosure can be a non-therapeutic product, an active ingredient thereof, or an antiseptic thereof; wherein the non-therapeutic product form includes but are not limited to health care products, personal cleaning and care products, cosmetics, hygiene products, disposable hygiene products, cleaning products, daily necessities, microecological care products, deodorants, lubricants, humectants, lotions, cleaning agents, body care products, antipruritic agents, refreshing agents, or can be the components of hygiene products or cleaning care products such as sanitary napkins, sanitary pads, or tampons.

When the bacteriostatic composition as described in the present disclosure for inhibiting harmful microorganisms outside the body is used for antisepsis, the content of each ingredient of the bacteriostatic composition in the present disclosure contained in bath products, shampoo products, skin care products, cosmetics, pharmaceutical products, and other medical products, and the like, i.e. the total content of one or more of the fatty acids and/or salts thereof calculated as fatty acid, the total content of one or more of the dicarboxylic acids and/or salts thereof calculated as dicarboxylic acid, the total content of one or more of the aromatic alcohols, the total content of one or more of the aromatic carboxylic acids and/or salts thereof calculated as aromatic carboxylic acid, shall be within the total content ranges of the four ingredients in the bacteriostatic composition in the present disclosure.

The present invention also provides a vaginal bacterial composition for use in modulating vaginal flora, wherein the modulating vaginal flora refers to at least one of the following: inhibiting abnormal vaginal flora, restoring and/or maintaining vaginal lactobacilli. Wherein the vaginal bacteriostatic composition comprises one or more of the fatty acids and/or salts thereof selected from the group consisting of butyric acid, valeric acid, hexanoic acid, heptanoic acid, caprylic acid, nonanoic acid, capric acid, undecylic acid, undecylenic acid, lauric acid, and salts thereof; the total content of which, calculated as fatty acid, is in the range of 0.001-2.00% (w/w), preferably in the range of 0.001-1.50% (w/w). The more preferable fatty acids and/or salts thereof are selected from the group consisting of butyric acid, hexanoic acid, caprylic acid, capric acid, undecylic acid, salts thereof, and mixtures thereof; the total content of which is in the range of 0.002-1.25% (w/w);

Wherein the vaginal bacteriostatic composition is in one of the following dosage forms: aqueous solutions, water-soluble gels, foams, sprays, ointments, powders, films, capsules, suppositories, or tablets, preferably aqueous solutions, water-soluble gels, foams, sprays, or ointments.

In some embodiments, the vaginal bacteriostatic composition as described for use in modulating vaginal flora in the present invention also comprises one or more of the dicarboxylic acids and/or salts thereof selected from the group consisting of glutaric acid, adipic acid, pimelic acid, and salts thereof; the total content of which, calculated as dicarboxylic acid, is in the range of 0.50-2.50% (w/w). The preferabe dicarboxylic acid and/or salt thereof is adipic acid and/or salt thereof, the total content of which is in the range of 0.50-1.50% (w/w). The combination of dicarboxylic acids such as adipic acid, salts thereof as described and fatty acids such as butyric acid, salts thereof in the present disclosure can enhance the bacteriostatic effect of the fatty acids and/or salts thereof as described against *Candida albicans.*

In some embodiments, the vaginal bacteriostatic composition as described for use in modulating vaginal flora in the present invention may also comprise one or more of the aromatic alcohols selected from the group consisting of benzyl alcohol, phenethyl alcohol, phenoxyethanol, and cinnamyl alcohol; the total content of which is in the range of 0.05-0.60% (w/w). The preferable aromatic alcohols are phenethyl alcohol, cinnamyl alcohol, or mixtures thereof; the total content of which is in the range of 0.08-0.50% (w/w). The combination of aromatic alcohols such as phenethyl alcohol as described and fatty acids such as butyric acid, and salts thereof in the present disclosure can enhance the bacteriostatic effect of the fatty acids and/or salts thereof as described against *Candida albicans*, *Staphylococcus aureus*, *Escherichia coli*, abnormal vaginal flora, and the like.

In some embodiments, the vaginal bacteriostatic composition for use in modulating vaginal flora in the present invention may also comprise one or more of the aromatic carboxylic acids and/or salts thereof selected from the group consisting of benzoic acid, p-hydroxybenzoic acid, p-methoxybenzoic acid, salicylic acid, cinnamic acid, gentianic acid, caffeic acid, and salts thereof; the total content of which, calculated as aromatic carboxylic acid, is in the range of 0.05-0.25% (w/w). The preferable aromatic carboxylic acids and/or salts thereof are selected from the group consisting of benzoic acid, cinnamic acid, p-hydroxybenzoic acid, salts thereof, and mixtures thereof; the total content of which is in the range of 0.08-0.20% (w/w). The combination of aromatic carboxylic acids such as benzoic acid, salts thereof as described and fatty acids such as butyric acid, salts thereof in the present disclosure can enhance the bacteriostatic effect of the fatty acids and/or salts thereof as described against *Candida albicans*, *Staphylococcus aureus*, *Escherichia coli*, abnormal vaginal flora, and the like.

In some embodiments, the vaginal bacteriostatic composition for use in modulating vaginal flora in the present invention comprises: (1) one or more of the fatty acids and/or salts thereof selected from the group consisting of butyric acid, valeric acid, hexanoic acid, heptanoic acid, caprylic acid, nonanoic acid, capric acid, undecylic acid, undecylenic acid, lauric acid, and salts thereof; the total content of which, calculated as fatty acid, is in the range of 0.001-2.00% (w/w), preferably in the range of 0.001-1.50% (w/w). The preferable fatty acids and salts thereof are selected from the group consisting of butyric acid, hexanoic acid, caprylic acid, capric acid, undecylic acid, salts thereof, and mixtures thereof; the total content of which is in the range of 0.002-1.25% (w/w); (2) one or more of the dicarboxylic acids and/or salts thereof selected from the group consisting of glutaric acid, adipic acid, pimelic acid, and salts thereof; the total content of which, calculated as dicarboxylic acid, is in the range of 0.50-2.50% (w/w); preferably adipic acid and/or salt thereof, the total content of which is in the range of 0.50-1.50% (w/w); (3) one or more of the aromatic alcohols selected from the group consisting of benzyl alcohol, phenethyl alcohol, phenoxyethanol, and cinnamyl alcohol; the total content of which is in the range of 0.05-0.60% (w/w); the preferable aromatic alcohols are phenethyl alcohol, cinnamyl alcohol, or mixtures thereof; the total content of which is in the range of 0.08-0.50% (w/w).

The combination of dicarboxylic acids such as adipic acid and/or salts thereof, aromatic alcohols such as phenethyl alcohol, and fatty acids such as butyric acid and/or salts thereof in the present disclosure has a synergetic bacteriostatic effect against *Candida albicans, Staphylococcus aureus,* and *Escherichia coli,* and has an inhibitory effect on abnormal vaginal flora.

In some embodiments, the vaginal bacteriostatic composition for use in modulating vaginal flora in the present disclosure comprises: (1) one or more of the fatty acids and/or salts thereof selected from the group consisting of butyric acid, valeric acid, hexanoic acid, heptanoic acid, caprylic acid, nonanoic acid, capric acid, undecylic acid, undecylenic acid, lauric acid, and salts thereof; the total content of which, calculated as fatty acid, is in the range of 0.001-2.00% (w/w), preferably in the range of 0.001-1.50% (w/w). The preferable fatty acids and/or salts thereof are selected from the group consisting of butyric acid, hexanoic acid, caprylic acid, capric acid, undecylic acid, salts thereof, and mixtures thereof; the total content of which is in the range of 0.002-1.25% (w/w); (2) one or more of the dicarboxylic acids and/or salts thereof selected from the group consisting of glutaric acid, adipic acid, pimelic acid, and salts thereof; the total content of which, calculated as dicarboxylic acid, is in the range of 0.50-2.50% (w/w); preferably adipic acid and/or salt thereof in the total content of 0.50-1.50% (w/w); (3) one or more of the aromatic carboxylic acids and/or salts thereof selected from the group consisting of benzoic acid, p-hydroxybenzoic acid, p-methoxybenzoic acid, salicylic acid, cinnamic acid, gentianic acid, caffeic acid, and salts thereof; the total content of which, calculated as aromatic carboxylic acid, is in the range of 0.05-0.25% (w/w). The preferable aromatic carboxylic acids and/or salts thereof are benzoic acid, cinnamic acid, p-hydroxybenzoic acid, salts thereof, and mixtures thereof; the total content of which is in the range of 0.08-0.20% (w/w).

The combination of dicarboxylic acids such as adipic acid and/or salts thereof, aromatic carboxylic acids such as benzoic acid and/or salts thereof, and fatty acids such as butyric acid and/or salts thereof in the present disclosure has a synergetic bacteriostatic effect against *Candida albicans*, *Staphylococcus aureus*, and *Escherichia coli*, and has an inhibitory effect on abnormal vaginal flora.

In some embodiments, the vaginal bacteriostatic composition for use in modulating vaginal flora in the present disclosure may comprise: (1) one or more of the fatty acids and/or salts thereof selected from the group consisting of butyric acid, valeric acid, hexanoic acid, heptanoic acid, caprylic acid, nonanoic acid, capric acid, undecylic acid, undecylenic acid, lauric acid, and salts thereof; the total content of which, calculated as fatty acid, is in the range of 0.001-2.00% (w/w), preferably in the range of 0.001-1.50% (w/w). The preferable fatty acids and/or salts thereof are selected from the group consisting of butyric acid, hexanoic acid, caprylic acid, capric acid, undecylic acid, salts thereof, and mixtures thereof; the total content of which is in the range of 0.002-1.25% (w/w); (2) one or more of the aromatic alcohols selected from the group consisting of benzyl alcohol, phenethyl alcohol, phenoxyethanol, and cinnamyl alcohol; the total content of which is in the range of 0.05-0.60% (w/w). The preferable aromatic alcohols are phenethyl alcohol, cinnamyl alcohol, or mixtures thereof; the total content of which is in the range of0.08-0.50% (w/w); (3) one or more of the aromatic carboxylic acids and/or salts thereof selected from the group consisting of benzoic acid, p-hydroxybenzoic acid, p-methoxybenzoic acid, salicylic acid, cinnamic acid, gentianic acid, caffeic acid, salts thereof; the total content of which, calculated as aromatic carboxylic acid, is in the range of 0.05-0.25% (w/w). The preferable aromatic carboxylic acids and/or salts thereof are selected from the group consisting of benzoic acid, cinnamic acid, p-hydroxybenzoic acid, salts thereof, and mixtures thereof; the total content of which is in the range of 0.08-0.20% (w/w).

The combination of aromatic alcohols such as phenethyl alcohol, aromatic carboxylic acids such as benzoic acid and/or salts thereof, and fatty acids such as butyric acid and/or salts thereof in the present disclosure has a synergetic bacteriostatic effect against *Candida albicans, Staphylococcus aureus,* and *Escherichia coli,* and has an inhibitory effect on abnormal vaginal flora.

In some embodiments, the vaginal bacteriostatic composition for use in modulating vaginal flora in the present disclosure may comprise: (1) one or more of the fatty acids and/or salts thereof selected from the group consisting of butyric acid, valeric acid, hexanoic acid, heptanoic acid, caprylic acid, nonanoic acid, capric acid, undecylic acid, undecylenic acid, lauric acid, and salts thereof; the total content of which, calculated as fatty acid, is in the range of 0.001-2.00% (w/w), preferably in the range of 0.001-1.50% (w/w). The preferable fatty acids and/or salts thereof are selected from the group consisting of butyric acid, hexanoic acid, caprylic acid, capric acid, undecylic acid, salts thereof, and mixtures thereof; the total content of which is in the range of 0.002-1.25% (w/w); (2) one or more of the dicarboxylic acids and/or salts thereof selected from the group consisting of glutaric acid, adipic acid, pimelic acid, and salts thereof; the total content of which, calculated as dicarboxylic acid, is in the range of 0.50-2.50% (w/w); preferably adipic acid and/or salt thereof in the total content of 0.50-1.50% (w/w); (3) one or more of the aromatic alcohols selected from the group consisting of benzyl alcohol, phenethyl alcohol, phenoxyethanol, and cinnamyl alcohol; the total content of which is in the range of 0.05-0.60% (w/w). The preferable aromatic alcohols are phenethyl alcohol, cinnamyl alcohol, or mixtures thereof; the total content of which is in the range of 0.08-0.50% (w/w); (4) one or more of the aromatic carboxylic acids and/or salts thereof selected from the group consisting of benzoic acid, p-hydroxybenzoic acid, p-methoxybenzoic acid, salicylic acid, cinnamic acid, gentianic acid, caffeic acid, salts thereof; the total content of which, calculated as aromatic carboxylic acid, is in the range of 0.05-0.25% (w/w). The preferable aromatic carboxylic acids and/or salts thereof are selected from the group consisting of benzoic acid, cinnamic acid, p-hydroxybenzoic acid, salts thereof, and mixtures thereof; the total content of which is in the range of 0.08-0.20% (w/w).

The combination of dicarboxylic acids such as adipic acid and/or salts thereof, aromatic alcohols such as phenethyl alcohol, aromatic carboxylic acids such as benzoic acid and/or salts thereof, and fatty acids such as butyric acid and/or salts thereof in the present disclosure has a synergetic bacteriostatic effect against *Candida albicans*, *Staphylococcus aureus*, and *Escherichia coli,* and has an inhibitory effect on abnormal vaginal flora. After vaginal application of the bacteriostatic composition, there was a significant decrease in abnormal vaginal flora and a significant increase in lactobacilli. Therefore, the bacteriostatic composition as described has the effects of inhibiting abnormal vaginal flora, restoring and/or maintaining and/or promoting vaginal lactobacilli, and restoring and/or maintaining normal vaginal acidity.

In some embodiments, the vaginal bacteriostatic composition as described for use in modulating vaginal flora in the present disclosure may further comprise one or more of the estrogens selected from the group consisting of diethylstilbestrol, hexoestrol, estradiol, estrone, estriol, nilestriol, ethinyloestradiol, quinestrol, mestranol, and promestriene; the total content of which is in the range of 0.001-1.00% (w/w), preferably estriol and promestriene. As estrogens can promote the glycogen synthesis of vaginal mucosal epithelial cells and promote the growth of beneficial lactobacilli, the bacteriostatic composition in the present disclosure containing estrogens is especially suitable for use in menopause, postmenopausal, or postpartum.

In some embodiments, the vaginal bacteriostatic composition for use in modulating vaginal flora in the present disclosure may further comprises one or more of the phytoestrogens selected from the group consisting of daidzin, daidzein, glycitein, puerarin, coumestrol, genistein, equol, apigenin, genistin, genisteol, biochanin, coumestrol, formononetin, resveratrol, secoisolariciresinol, and lignan; the total content of which is in the range of 0.001-1.00% (w/w). As phytoestrogens can promote the glycogen synthesis of vaginal mucosal epithelial cells and promote the growth of beneficial lactobacilli, the bacteriostatic composition in the present disclosure containing phytoestrogens is especially suitable for use in menopause, postmenopausal, or postpartum.

The vaginal bacteriostatic composition of the invention may be used to restore and/or maintain normal vaginal flora, and/or to restore and/or maintain normal vaginal microecology, and/or to restore and/or maintain normal vaginal acidity, and/or to clean and take care of the vagina and/or vulva, and/or to reduce and/or eliminate vaginal pruritus, soreness, dryness, irritation, and dyspareunia, and/or to reduce and/or eliminate abnormal vaginal discharge and unpleasant odor of vaginal discharge.

The vaginal bacteriostatic composition of the invention may be used to prevent and/or treat the imbalance of vaginal flora, bacterial vaginosis, aerobic vaginitis, cytolytic vaginosis, vulvovaginal Candidiasis, and/or atrophic vaginitis.

The bacteriostatic composition of the invention can be prepared by the following method, wherein the preparation method comprises the following steps: to add the following ingredients to an excipient, wherein the excipient is used for aqueous solutions, water-soluble gels, foams, suppositories, or tablets:
(1) one or more of the fatty acids and/or salts thereof selected from the group consisting of acetic acid, propionic acid, butyric acid, isobutyric acid, hydroxybutyric acid, valeric acid, hexanoic acid, heptanoic acid, caprylic acid, nonanoic acid, capric acid, undecylic acid, undecylenic acid, lauric acid, and salts thereof; the total content of which, calculated as fatty acid, is in the range of 0.001-3.00% (w/w); preferably propionic acid, butyric acid, hexanoic acid, caprylic acid, capric acid, undecylic acid, salts thereof, and mixtures thereof;
(2) one or more of the dicarboxylic acids and/or salts thereof selected from the group consisting of glutaric acid, adipic acid, pimelic acid, and salts thereof; the total content of which, calculated as dicarboxylic acid, is in the range of 0.05-5.00% (w/w); preferably adipic acid, and salt thereof;
(3) one or more of the aromatic alcohols selected from the group consisting of benzyl alcohol, , phenethyl alcohol, phenoxyethanol, and cinnamyl alcohol; the total content of which is in the range of 0.03-1.00% (w/w); preferably phenethyl alcohol, cinnamic alcohol, or mixtures thereof;
(4) one or more of the aromatic carboxylic acids and/or salts thereof selected from the group consisting of benzoic acid, p-hydroxybenzoic acid, p-methoxybenzoic acid, salicylic acid, cinnamic acid, gentianic acid, caffeic acid, and salts thereof; the total content of which, calculated as aromatic carboxylic acid, is in the range of 0.03-1.00% (w/w); preferably benzoic acid, cinnamic acid, p-hydroxybenzoic acid, salts thereof, and mixtures thereof.

The composition as described is in one of the following dosage forms: aqueous solutions, water-soluble gels, foams, sprays, ointments, powders, films, capsules, suppositories, or tablets, preferably, aqueous solutions, or water-soluble gels, or foams, or sprays, or ointments.

In some embodiments, the preparation method comprises the addition of one or more of the fatty acids and/or salts thereof as described, the total content of which, calculated as fatty acid, is preferably in the range of 0.001-2.00% (w/w), more preferably in the range of 0.001-1.50% (w/w), and the most preferably in the range of 0.002%-1.25% (w/w); and/or the addition of one or more of the dicarboxylic acids and/or salts thereof as described, the total content of which, calculated as dicarboxylic acid, is preferably in the range of 0.10-3.50% (w/w), more preferably in the range of 0.50-2.50% (w/w), and the most preferably in the range of 0.50-1.50% (w/w); and/or the addition of one or more of the aromatic alcohols, the total content of which is preferably in the range of 0.03-0.70% (w/w), more preferably in the range of 0.05-0.60% (w/w), and the most preferably in the range of 0.08-0.50% (w/w); and/or the addition of one or more of the aromatic carboxylic acids, the total content of which is preferably in the range of 0.03-0.50% (w/w), more preferably in the range of 0.05-0.25% (w/w), and the most preferably in the range of 0.08-0.20% (w/w).

In the preparation of water-soluble gels, a non-flowable, viscous, and water-soluble colloidal excipient is needed, which enables the composition to homogeneously contact with the vaginal mucosa and to stay for a longer time to take effect. In some embodiments, wherein the excipient is xanthan gum, carbomer, polycarbophil, dextran, glucomannan, tragacanth gum, gummitragacanthae, methyl cellulose (MC), carboxymethyl cellulose (CMC), hydroxyethyl cellulose (HEC), hydroxyethyl methyl cellulose (HEMC), and hydroxypropyl methyl cellulose (HPMC), preferably xanthan gum, and carbomer.

In some embodiments, wherein the composition can be prepared according to the following technological process: weigh and take (1) one or more of the fatty acids and/or salts thereof such as propionic acid and/or sodium salt thereof, (2) one or more of the dicarboxylic acids and/or salts thereof such as adipic acid and/or salts thereof, (3) one or more of the aromatic alcohols such as phenethyl alcohol, (4) one or more of the aromatic carboxylic acids and/or salts thereof such as benzoic acid and/or sodium salts thereof, (5) one or more of the colloidal substrates such as xanthan gum, and other ingredients, mix well, add purified water quantitatively, stir and mix well, so that each ingredient is dissolved and the colloidal substrate swells into homogeneous colloid; adjust the pH value of the composition with acid and/or alkali to the range of 3.1-4.8, preferably 3.6-4.6, and more preferably 3.8-4.4. In some embodiments, sterilization can be further carried out, wherein the sterilization technology may be selected from the following: radiation sterilization, high-temperature sterilization (for example, sterilizing at 115.6°C for 15-20 minutes; or sterilizing at 100°C for > 30 minutes), intermittent sterilization (for example, sterilizing at 80°C for 30 minutes, then placing at 36°C for 5-10 hours, then once again at 80°C for 30 minutes, then at 36°C for 5-10 hours, and finally at 80°C for 30 minutes). Alternatively, in some embodiments, wherein ingredients such as benzoic acid and/or sodium salt thereof are dissolved separately into solutions, sterilized by filtration, and then added into a sterilized water-soluble gel.

In some embodiments, when a solution is prepared, all of the above ingredients except xanthan gum can be mixed well, dissolved with water, sterilized, and packaged; or dissolved, filtered, and packaged.

In some embodiments, when an emulsion-type ointment is prepared, the selection of excipient and the specific preparation process can be referred to methods known to those skilled in the art, such as the method introduced in *Pharmacy* (see Reference 1) edited by Fang Liang.

In some embodiments, a tablet can be prepared by referring to methods known to those skilled in the art, such as the method introduced in *Pharmacy* (see Reference 2) edited by Fang Liang, where quantified fatty acid and/or salt thereof, dicarboxylic acid and/or salt thereof, aromatic alcohol, aromatic carboxylic acid and/or salt thereof, and other ingredients are thoroughly mixed with excipients, then directly tableting. Optionally, excipients can be added, e.g., lubricants such as magnesium stearate or disintegrants such as sodium carboxymethyl starch can be added, homogeneously mixed, then tableting. In some embodiments, the prepared tablets can also be packaged into administration apparatus, disinfection devices, medical devices, or pharmaceutical devices.

In some embodiments, a film or suppository can be prepared by referring to methods known to those skilled in the art, such as the method introduced in *Pharmacy* (see References 3 and 4) edited by Fang Liang.

In some embodiments, when sanitary napkins, sanitary pads, or tampons containing the bacteriostatic composition in the present disclosure are prepared, ingredients such as fatty acid and/or salt thereof, dicarboxylic acid and/or salt thereof, aromatic alcohol, aromatic carboxylic acid and/or salt thereof in the present disclosure, e.g., sodium benzoate, sodium propionate, hexanoic acid, phenethyl alcohol, adipic acid, and other ingredients, can be weighed and taken, then added with corresponding excipients, and prepared into powders, films, tablets, or capsules, which are then placed inside the sanitary napkins, sanitary pads, or tampons through an appropriate method or process. Optionally, ingredients such as fatty acid and/or salt thereof, dicarboxylic acid and/or salt thereof, aromatic alcohol, aromatic carboxylic acid and/or salt thereof in the present disclosure, such as sodium propionate, hexanoic acid, phenethyl alcohol, sodium benzoate, adipic acid, and other ingredients, and appropriate excipients, are attached to the inner-layer materials of sanitary napkins, sanitary pads, or tampons via an appropriate process.

In some embodiments, when the dosage forms such as capsules, suppositories, or tablets are prepared, each unit dosage form comprises a specific content range of the ingredients of the composition in the present disclosure. For example, each unit dosage form comprises one or more of the fatty acids and/or salts thereof, the total content of which, calculated as fatty acid, is in the range of 0.00005-0.15 g; one or more of the dicarboxylic acids and/or salts thereof, the total content of which, calculated as dicarboxylic acid and/or polybasic carboxylic acid, is in the range of 0.0025-0.25 g; one or more of the aromatic alcohols, the total content of which is in the range of 0.0015-0.050 g; and one or more of the aromatic carboxylic acids and/or salts thereof, the total content of which, calculated as aromatic carboxylic acid, is in the range of 0.0015-0.05 g. Preferably, each unit dosage form comprises one or more of the fatty acids and/or salts thereof, the total content of which, calculated as fatty acid, is in the range of 0.00005-0.100 g; one or more of the dicarboxylic acids and/or salts thereof, the total content of which, calculated as dicarboxylic acid and/or polybasic carboxylic acid, is in the range of 0.005-0.175 g; one or more of the aromatic alcohols, the total content of which is in the range of 0.0015-0.035 g; and one or more of the aromatic carboxylic acids and/or salts thereof, the total content of which, calculated as aromatic carboxylic acid, is in the range of 0.0015-0.025 g.

Various organic acids and/or salts thereof used in the preparation method in the present disclosure, such as fatty acid and/or salt thereof, dicarboxylic acid and/or salt thereof, or aromatic carboxylic acid and/or salt thereof, are dissolved in water into non-ionized molecules and ionized ions. The ratio of the non-ionized molecules to the ionized ions depends on the pH value of the solutions and the ionization constant (pKa) of the organic acid. Evidently, both organic acids and salts thereof are organic acid molecules or organic acid radical ions after dissolution in water. From this perspective, there is no substantive difference between the two.

### Detailed Description

Multiple exemplary embodiments of the present invention and of examples having permitted to validate the invention are described in detail below. Furthermore, wherein a range of values is provided in this invention, it should be understood that the upper and lower limits of that range, and each intermediate value between them, are also specifically disclosed. Each smaller range between any stated value or intermediate value in a stated range and any other stated value or intermediate value in that stated range is included in the invention. The upper and lower limits of these smaller ranges may independently be included or excluded in the range.

Unless defined otherwise, all technical and scientific terms used herein have the same meaning as commonly understood by those of ordinary skill in the art to which this invention belongs. In case of conflict with any incorporated literature, this Specification shall prevail. Unless otherwise stated, "%" is a percentage by weight.

Bacteriostatic agents, commonly used for the cleaning, bacteriostasis, or disinfection of skin and/or mucosa such as povidone iodine and chlorhexidine, are mostly broad-spectrum bacteriostatic agents that inhibit not only *Escherichia coli*, *Staphylococcus aureus*, and fungi, but also lactic acid bacteria such as lactobacilli. Antiseptics, commonly used in skin care products, cosmetics, bath products, as well as medical products and pharmaceutical products such as chlorobutanol and benzalkonium chloride, usually also have an inhibitory effect on lactic acid bacteria such as lactobacilli. There is a lack of highly selective bacteriostatic agents or bacteriostatic compositions that strongly inhibit harmful bacteria, but weakly inhibit beneficial bacteria such as lactobacilli.

The prior art (e.g., PCT/CN2017/105296, ZL201080036139, and US8765819) discloses compositions formulated with the combination of low-concentration bacteriostatic agents, such as the low-concentration of phenethyl alcohol, propionic acid and/or salt thereof, and/or benzoic acid and/or salt thereof, which can inhibit *Escherichia coli* and *Staphylococcus aureus,* with an inhibition rate against *Escherichia coli and Staphylococcus aureus* of up to more than 50% and a low inhibition rate against *Candida albicans* of <50% according to the test methods and judgment criteria of results in the National Standards of the People's Republic of China *Hygienic Standard for Disposable Sanitary Products* (GB15979-2002).

Increasing the concentration of the bacteriostatic agent can enhance the bacteriostatic effect against *Candida albicans.* Studies have shown that an appropriate increase in the concentration of each ingredient in the bacteriostatic combination of "propionic acid and/or salt thereof + benzoic acid and/or salt thereof + phenethyl alcohol" can not only enhance the bacteriostatic effect of the combination of bacteriostatic agents, therefor more effectively inhibit *Escherichia coli, Staphylococcus aureus,* and the like, but also inhibit *Candida albicans* by an inhibition rate of more than 50%. However, the higher the concentration of the bacteriostatic agent is, the stronger the inhibition against lactobacilli and other lactic acid bacteria becomes. The experimental example I disclosed in PCT/CN2017/105296 showed that when the concentration of sodium propionate was 0.40% (w/v) or 0.50% (w/v), the growth of lactobacilli was not significantly inhibited; when the concentration of sodium propionate was 0.70% (w/v), the growth of lactobacilli was inhibited. When the concentration of phenethyl alcohol was 0.40% (w/v), the growth of lactobacilli was not significantly inhibited; when the concentration of phenethyl alcohol was 0.50% (w/v), the growth of lactobacilli and acid production was inhibited.

The inventor has continuously carried out an in-depth study to further develop bacteriostatic compositions that effectively inhibit harmful microorganisms but not beneficial bacteria such as lactobacilli, and that can be used for the cleaning, bacteriostasis, or disinfection of skin and/or mucosa, and can be used as antiseptics for skin care products, cosmetics, and bath products, as well as medical products and pharmaceutical products. The inventor has found that the selective combination of appropriate fatty acids and/or salts thereof with dicarboxylic acids, aromatic alcohols, and aromatic carboxylic acids has a synergetic bacteriostatic effect against not only *Staphylococcus aureus* and *Escherichia coli,* but also *Candida albicans,* as well as significantly inhibits the growth of *Pseudomonas aeruginosa* and *Aspergillus niger.* The vaginal use of bacteriostatic composition prepared accordingly can result in a significant decrease in abnormal vaginal flora and a significant increase in lactobacilli, thus effectively modulate vaginal flora and vaginal acidity. Therefore, the bacteriostatic composition in the present disclosure can be used for the cleaning, bacteriostasis, or disinfection of skin and/or mucosa, and for antisepsis of bath products, skin care products, and cosmetics, as well as medical products, and pharmaceutical products.

In order to facilitate the accurate understanding of relevant nouns or phrases or expressions or standards mentioned herein, the meanings thereof are hereby clarified and defined as follows:
"Lactobacillus" refers to the bacteria of the genus *Lactobacillus,* a kind of gram-positive, rod-shaped, non-spore-forming bacteria capable of producing a large amount of lactic acid as a byproduct of glucose metabolism, comprising hundreds of species and subspecies.

"Lactic acid bacteria" is a general term of bacteria capable of metabolizing fermentable carbohydrates to produce a large amount of lactic acid, and refer to bacteria of more than 200 species of 18 genera. *Lactobacillus* is also a kind of lactic acid bacteria.

"Normal vaginal flora" refers to a Nugent score of 1-3. The vaginal bacteria are dominated by Lactobacillus species and with a small number of other bacteria. Wherein the "other bacteria" refer to gram-positive cocci such as *Staphylococcus* and *Streptococcus,* gram-negative bacilli such as *Gardnerella* and *Escherichia coli,* gram-negative cocci such as *Veillonella parvula,* and obligate anaerobes such as *Prevotella* and *Mobiluncus.*

"Abnormal vaginal flora" refers to a Nugent score of 5-10. The vaginal bacteria are dominated by other bacteria and with a small number of *Lactobacillus* species. Wherein the "other bacteria" refer to gram-positive cocci such as *Staphylococcus* and *Streptococcus,* gram-negative bacilli such as *Gardnerella* and *Escherichia coli,* gram-negative cocci such as *Veillonella parvula,* and obligate anaerobes such as *Prevotella* and *Mobiluncus.*

"Harmful microorganisms" generally refer to various highly pathogenic microorganisms, or various opportunistically pathogenic microorganisms that cause disease when human immunity is weakened, or microorganisms that cause the spoilage of food, pharmaceutical products, cosmetics, and hygiene products, including but not limited to the following group of microorganisms: *Candida, Staphylococcus aureus, Escherichia coli, Pseudomonas aeruginosa, Gardnerella, Prevotella, Mobiluncus, Aspergillus niger,* the abnormal flora of skin and/or mucosa, as well as viruses such as HPV and HIV.

"Ingredient" refers to various ingredients in the composition, including bacteriostatic agents and other ingredients without bacteriostatic activity.

"Bacteriostatic agent" refers to various ingredients with bacteriostatic effects, or combinations thereof, not limited to conventional bacteriostatic agents or combinations thereof recognized by those skilled in the art.

"Inhibition rate" is the inhibition rate of bacteriostatic agents against *Escherichia coli, Staphylococcus aureus, Candida albicans,* or other microorganisms obtained by referring to the test methods and judgment criteria in Appendix C of GB 15979-2002 *Hygienic Standard for Disposable Sanitary Products.*

"Bacteriostatic effect" refers to the inhibition of bacteria, fungi, or other microorganisms by bacteriostatic agents. The strength of inhibition is judged herein based on the inhibition rate, specifically as follows: inhibition rate >50%, indicating "possessing bacteriostatic effect "; inhibition rate >90%, indicating "strong bacteriostatic effect".

"Low-concentration bacteriostatic agent" is a relatively low concentration bacteriostatic agent with an inhibition rate of <50% against *Candida albicans.*

"High-concentration bacteriostatic agent" is a relatively high concentration bacteriostatic agent with an inhibition rate of >50% against *Candida albicans.*

"No difference in inhibition rates" or "no influence on bacteriostatic effect" indicates that the difference between two inhibition rates is <15%.

"A difference in inhibition rates" or "influence on bacteriostatic effect" indicates that the difference between two inhibition rates is 15-30%.

"A significant difference in inhibition rates" or "significant influence on bacteriostatic effect" indicates that the difference between two inhibition rates is >30%.

"Synergistic bacteriostatic effect" refers to the combination of two or more bacteriostatic agents acting against *Escherichia coli, Staphylococcus aureus,* or *Candida albicans,* which has an inhibition rate that is more than 15% higher than the sum of the inhibition rates of each bacteriostatic agent acting alone.

### Embodiment 1

This embodiment provides a plurality of exemplary ingredients of the bacteriostatic composition. Unless specifically stated otherwise, exemplary ingredients of the composition, described hereunder, are the following substances:
Propionic acid CAS: 79-09-4, adipic acid CAS: 124-04-9, phenethyl alcohol (2-phenylethanol) CAS: 60-12-8, butyric acid (n-butyric acid) CAS: 107-92-6, hexanoic acid (n-hexanoic acid) CAS: 142-62-1, pimelic acid CAS: 111-16-0, malic acid (L-hydroxysuccinic acid) CAS: 97-67-6, cinnamic acid (trans-cinnamic acid) CAS: 140-10-3, salicylic acid (2-hydroxybenzoic acid) CAS: 69-72-7, lauric acid (dodecanoic acid) CAS: 143-07-7, valeric acid (n-valeric acid) CAS: 109-52-4, heptanoic acid (enanthic acid) CAS: 111-14-8, caprylic acid (n-caprylic acid) CAS: 124-07-2, nonanoic acid (n-nonanoic acid) CAS: 112-05-0, capric acid (n-capric acid) CAS: 334-48-5, undecylic acid (undecanoic acid) CAS: 112-37-8, cinnamyl alcohol (3-phenyl-2-propen-1-ol) CAS: 104-54-1, succinic acid (butanedioic acid) CAS: 110-15-6, tartaric acid (L-tartaric acid) CAS: 87-69-4, maleic acid (cis-butenedioic acid) CAS: 110-16-7, citric acid CAS: 77-92-9, fumaric acid (trans-butenedioic acid) CAS: 110-17-8, undecylenic acid (10-undecylenic acid) CAS: 112-38-9, glutaric acid CAS: 110-94-1.

### Example 1:

Add 1.50 g of adipic acid, 2.00 g of propionic acid, 0.25 g of phenethyl alcohol, 1.00 g of citric acid, and 2.50 g of maltose to 80 g of purified water and stir to dissolve them, then add 2.15 g of xanthan gum, and complement purified water to render a total weight of 100 g. Stir for the xanthan gum to swell into a homogeneous viscous gel, then adjust pH to 3.1 with 1.0 mol/L of sodium hydroxide solution, and sterilize at 115.6°C for 15 minutes, to obtain the water-soluble gel composition in the present disclosure.

### Example 2:

Raw materials were weighed and taken according to the following formula, to prepare 100 g of composition by basically following the method in example 1.

| | |
|---|---|
| Adipic acid | 1.50 g |
| Propionic acid | 1.25 g |
| Cinnamic acid | 0.08 g |
| Benzyl alcohol | 0.50 g |
| Maltose | 1.40 g |
| Xanthan gum | 2.50 g |
| Add purified water to reach the total weight of Adjust pH to 3.8 | 100 g |

### Example 3:

Raw materials were weighed and taken according to the following formula, to prepare 100 g of composition by basically following the method in example 1.

| | |
|---|---|
| Pimelic acid | 0.50 g |
| Cinnamic acid | 0.03 g |
| Phenoxyethanol | 1.00 g |
| Butyric acid | 0.60 g |
| Xanthan gum | 2.15 g |
| Add purified water to reach the total weight of Adjust pH to 4.4 | 100 g |

### Example 4:

Raw materials were weighed and taken according to the following formula, to prepare 100 g of composition by basically following the method in example 1.

| | |
|---|---|
| Glutaric acid | 5.00 g |
| Propionic acid | 1.50 g |
| Xanthan gum | 2.15 g |
| Add purified water to reach the total weight of Adjust pH to 3.8 | 100 g |

### Example 5 (only for illustrative purposes):

Raw materials were weighed and taken according to the following formula, to prepare 100 g of composition by basically following the method in example 1.

| | |
|---|---|
| Glutaric acid | 3.50 g |
| Salicylic acid | 0.05 g |
| Levulinic acid | 1.25 g |
| Isomaltulose | 1.50 g |
| Xanthan gum | 2.15 g |
| Add purified water to reach the total weight of Adjust pH to 3.8 | 100 g |

### Example 6:

Raw materials were weighed and taken according to the following formula, to prepare 100 g of composition by basically following the method in example 1.

| | |
|---|---|
| Pimelic acid | 2.50 g |
| P-hydroxybenzoic acid | 0.20 g |
| Lactic acid | 0.75 g |
| Cinnamyl alcohol | 0.03 g |
| Add purified water to reach the total weight of Adjust pH to 3.8 | 100 g |

### Example 7:

Raw materials were weighed and taken according to the following formula, to prepare 100 g of composition by basically following the method in example 1.

| | |
|---|---|
| Adipic acid | 0.10 g |
| Benzoic acid | 0.25 g |
| Caprylic acid | 0.01 g |
| Cinnamyl alcohol | 0.05 g |
| Malic acid | 0.70 g |
| Xanthan gum | 2.15 g |
| Add purified water to reach the total weight of Adjust pH to 3.8 | 100 g |

### Example 8:

Raw materials were weighed and taken according to the following formula, to prepare 100 g of composition by basically following the method in example 1.

| | |
|---|---|
| Benzoic acid | 0.25 g |
| Capric acid | 0.002 g |
| Succinic acid | 0.60 g |
| Palatinose | 1.00 g |
| Add purified water to reach the total weight of Adjust pH to 3.8 | 100 g |

### Example 9:

Raw materials were weighed and taken according to the following formula, to prepare 100 g of composition by basically following the method in example 1.

| | |
|---|---|
| Benzoic acid | 1.00 g |
| Phenethyl alcohol | 0.50 g |
| Add purified water to reach the total weight of Adjust pH to 4.8 | 100 g |

### Example 10:

Raw materials were weighed and taken according to the following formula, to prepare 100 g of the composition by basically following the method in example 1.

| | |
|---|---|
| Adipic acid | 1.50 g |
| Cinnamic acid | 0.05 g |
| Lauric acid | 0.001 g |
| Phenethyl alcohol | 0.70 g |
| Add purified water to reach the total weight of | 100 g |
| Adjust pH to | 3.8 |

### Example 11:

Raw materials were weighed and taken according to the following formula, to prepare 100 g of composition by basically following the method in example 1.

| | |
|---|---|
| Glutaric acid | 2.50 g |
| Benzoic acid | 0.50 g |
| Hexanoic acid | 0.15 g |
| Phenoxyethanol | 0.30 g |
| Add purified water to reach the total weight of | 100 g |
| Adjust pH to | 4.6 |

### Example 12:

Raw materials were weighed and taken according to the following formula, to prepare 100 g of the composition by basically following the method in example 1.

| | |
|---|---|
| Pimelic acid | 0.05 g |
| Cinnamic acid | 0.08 g |
| Phenethyl alcohol | 0.60 g |
| Undecylic acid | 0.001 g |
| Add purified water to reach the total weight of | 100 g |
| Adjust pH to | 3.6 |

### Example 13:

Raw materials were weighed and taken according to the following formula, to prepare 100 g of composition by basically following the method in example 1.

| | |
|---|---|
| Adipic acid | 1.50 g |
| Benzoic acid | 0.50 g |
| Propionic acid | 0.50 g |
| Cinnamyl alcohol | 0.08 g |
| Add purified water to reach the total weight of | 100 g |
| Adjust pH to | 4.4 |

### Example 14:

Raw materials were weighed and taken according to the following formula, to prepare 100 g of the composition by basically following the method in example 1.
Adipic acid 1.50 g, benzoic acid 0.20 g, propionic acid 1.25 g;
Glutamic acid 0.76 g, glutamine 0.34 g, aspartic acid 0.60 g, asparagine 0.98 g, isoleucine 0.31 g, methionine 0.35 g, phenylalanine 0.20 g, valine 0.46 g, leucine 0.78 g, proline 0.89 g;
Xanthan gum 2.50 g;
add purified water to reach the total weight of 100 g, and adjust pH to 4.0

### Example 15:

Raw materials were weighed and taken according to the following formula, to prepare 100 g of the composition by basically following the method in example 1.

| | |
|---|---|
| Adipic acid | 0.50 g |
| P-methoxybenzoic acid | 0.25 g |
| Acetic acid | 3.00 g |
| Glucose | 1.00 g |
| Xanthan gum | 2.15 g |
| Add purified water to reach the total weight of | 100 g |
| Adjust pH to | 3.6 |

### Example 16

A tablet containing 60 mg of adipic acid, 5 mg of sodium benzoate, 30 mg of propionic acid, and 60 mg of sucrose was prepared basically following the method described in Reference 1.

### Example 17

A vaginal suppository containing 60 mg of adipic acid, 5 mg of sodium benzoate, 30 mg of propionic acid, and 60 mg of maltose was prepared basically following the method described in Reference 3.

### Embodiment 2

This embodiment is used to validate the efficacy of the composition. The examples were designed to study the effect of each ingredient and combination of ingredients for illustrative purposes. Only the compositions comprising the at least four ingredients as defined in independent claim 1, or comprising the at least two ingredients as defined for the vaginal bacteriostatic composition of independent claim 9, are to be considered compositions according to the invention.

### In vitro experiment I

According to the method described in Appendix C of GB 15979-2002 *Hygienic Standard for Disposable Sanitary Products,* aqueous solutions containing different ingredients were studied for their inhibition rates of 20 minutes of action against *Candida albicans* ATCC 10231. The percentage concentration of each ingredient therein was the weight percentage concentration % (w/w), and the pH value of each group was 3.8. The experimental results are shown in Table 1.

**Table 1 Bacteriostatic Effect of Different Solutions Against Candida albicans ATCC 10231**

| Serial number | Propionic acid (%) | Adipic acid (%) | Benzyl alcohol (%) | Sodium benzoate (%) | Inhibition rate (%) |
|---|---|---|---|---|---|
| 1 | 0.50 | 1.50 | - | 0.20 | 61.29 |
| 2 | 0.50 | 1.50 | 0.50 | 0.20 | 81.52 |
| 3 | 0.50 | 1.50 | 0.70 | 0.20 | 99.27 |

Results:
1. It was evident in the result of Group 1 in Table 1 that, when the pH value was 3.8, the solution containing "0.50% propionic acid, 1.50% adipic acid, and 0.20% sodium benzoate" had a bacteriostatic effect against *Candida albicans,* and the inhibition rate was 61.29%;
2. It was evident in the results of Groups 2-3 in Table 1 that, when the pH value was 3.8, the combinations of "0.50% propionic acid, 1.50% adipic acid, and 0.20% sodium benzoate" and benzyl alcohol of two different concentrations (0.50%, 0.70%) respectively, had a bacteriostatic effect or a strong bacteriostatic effect against *Candida albicans,* and the inhibition rates were 81.52% and 99.27%, respectively.

In summary, it could be seen from the comparison of experimental results of Groups 2-3 and Group 1 that, when the pH value was 3.8, 0.50% and 0.70% benzyl alcohol respectively affected the bacteriostatic effect of the solution containing "0.50% propionic acid, 1.50% adipic acid, and 0.20% sodium benzoate" against *Candida albicans,* and could enhance the bacteriostatic effect.

### In vitro experiment II

According to the method described in Appendix C of GB 15979-2002 *Hygienic Standard for Disposable Sanitary Products,* aqueous solutions containing different ingredients were studied for their inhibition rates of 20 minutes of action against *Candida albicans* ATCC 10231. The percentage concentration of each ingredient therein was the weight percentage concentration % (w/w), and the pH value of each group was 4.0. The experimental results are shown in Table 2.

**Table 2 Bacteriostatic Effect of Different Solutions Against Candida albicans ATCC 10231**

| Serial number | Propionic acid (%) | Adipic acid (%) | Phenethyl alcohol (%) | Benzoic acid (%) | Inhibition rate (%) |
|---|---|---|---|---|---|
| 1 | 1.50 | - | 0.35 | 0.20 | 15.91 |
| 2 | 1.50 | - | 0.35 | 0.25 | 46.09 |
| 3 | 1.50 | 1.50 | 0.35 | 0.20 | 67.39 |
| 4 | 1.50 | 1.50 | 0.35 | 0.25 | 92.17 |

Results:
1. It was evident in the result of Group 1 in Table 2 that, when the pH value was 4.0, the inhibition rate of the solution containing "1.50% propionic acid, 0.35% phenethyl alcohol, and 0.20% benzoic acid" against *Candida albicans* was 15.91%;
2. It was evident in the result of Group 2 in Table 2 that, when the pH value was 4.0, the inhibition rate of the solution containing "1.50% propionic acid, 0.35% phenethyl alcohol, and 0.25% benzoic acid" against *Candida albicans* was 46.09%;
3. It was evident in the result of Group 3 in Table 2 that, when the pH value was 4.0, the solution containing "1.50% propionic acid, 1.50% adipic acid, 0.35% phenethyl alcohol, and 0.20% benzoic acid" had a bacteriostatic effect against *Candida albicans,* and the inhibition rate was 67.39%;
4. It was evident in the result of Group 4 in Table 2 that, when the pH value was 4.0, the solution containing "1.50% propionic acid, 1.50% adipic acid, 0.35% phenethyl alcohol, and 0.25% benzoic acid" had a strong bacteriostatic effect against *Candida albicans,* and the inhibition rate was 92.17%.

In summary,
1. It was evident in the comparison of experimental results of Groups 1 and 2, and the result of Groups 3 and 4 that, when the pH value was 4.0, the increase in the concentration of benzoic acid from 0.20% to 0.25% affected or significantly affected the bacteriostatic effects of the solution containing "1.50% propionic acid and 0.35% phenethyl alcohol" and the solution containing "1.50% propionic acid, 1.50% adipic acid, and 0.35% phenethyl alcohol" against *Candida albicans,* and enhanced the bacteriostatic effect;
2. It was evident in the comparison of experimental results of Groups 1 and 3, and Groups 2 and 4 that, when the pH value was 4.0, 1.50% adipic acid significantly affected the bacteriostatic effect of the solution containing "1.50% propionic acid, 0.35% phenethyl alcohol, and 0.20% benzoic acid" and the solution containing "1.50% propionic acid, 0.35% phenethyl alcohol, and 0.25% benzoic acid" against *Candida albicans,* and enhanced the bacteriostatic effect.

### In vitro experiment III

According to the method described in Appendix C of GB 15979-2002 *Hygienic Standard for Disposable Sanitary Products,* aqueous solutions containing different ingredients were studied for their inhibition rates of 20 minutes of action against *Candida albicans* ATCC 10231, the percentage concentration of each ingredient therein was the weight percentage concentration % (w/w), and the pH value of each group was 3.8. The experimental results are shown in Table 3.

**Table 3 Bacteriostatic Effect of Different Solutions Against Candida albicans ATCC 10231**

| Serial number | Propionic acid (%) | Adipic acid (%) | Benzyl alcohol (%) | Sodium benzoate (%) | Inhibition rate (%) |
|---|---|---|---|---|---|
| 1 | - | 1.35 | 0.25 | 0.20 | 45.12 |
| 2 | 0.50 | 1.35 | 0.25 | 0.20 | 61.60 |
| 3 | 1.25 | 1.35 | 0.25 | 0.20 | 89.72 |
| 4 | 1.50 | 1.35 | 0.25 | 0.20 | 94.42 |
| 5 | 3.00 | 1.35 | 0.25 | 0.20 | 100.00 |

Results:
1. It was evident in the result of Group 1 in Table 3 that, when the pH value was 3.8, the inhibition rate of the solution containing "1.35% adipic acid, 0.25% benzyl alcohol, and 0.20% sodium benzoate" against *Candida albicans* was 45.12%;
2. It was evident in the results of Groups 2-5 in Table 3 that, when the pH value was 3.8, the combinations of propionic acid of four different concentrations respectively (0.50%, 1.25%, 1.50%, 3.00%) and "1.35% adipic acid, 0.25% benzyl alcohol, and 0.20% sodium benzoate" had a bacteriostatic effect or a strong bacteriostatic effect against *Candida albicans,* and the inhibition rates were 61.60%, 89.72%, 94.42%, and 100.00%, respectively.

In summary, it could be seen from the comparison of experimental results of Groups 2-5 and Group 1 that, when the pH value was 3.8, propionic acid affected or significantly affected the bacteriostatic effect of the solution containing "1.35% adipic acid, 0.25% benzyl alcohol, and 0.20% sodium benzoate" against *Candida albicans,* and enhanced the bacteriostatic effect. The higher the concentration of propionic acid was, the stronger the bacteriostatic effect against *Candida albicans* became.

### In vitro experiment IV

According to the method described in Appendix C of GB 15979-2002 *Hygienic Standard for Disposable Sanitary Products,* aqueous solutions containing different ingredients were studied for their inhibition rates of 20 minutes of action against *Candida albicans* ATCC 10231, the percentage concentration of each ingredient therein was the weight percentage concentration % (w/w), and the pH value of each group was 4.3. The experimental results are shown in Table 4.

**Table 4 Bacteriostatic Effect of Different Solutions Against Candida albicans ATCC 10231**

| Serial number | Propionic acid (%) | Adipic acid (%) | Phenethyl alcohol (%) | Benzoic acid (%) | Inhibition rate (%) |
|---|---|---|---|---|---|
| 1 | 0.54 | 1.50 | 0.30 | - | 26.05 |
| 2 | 0.54 | 1.50 | 0.30 | 0.50 | 64.80 |
| 3 | 0.54 | 1.50 | 0.30 | 1.00 | 100.00 |

Results:
1. It was evident in the result of Group 1 in Table 4 that when the pH value was 4.3, the inhibition rate of the solution containing "0.54% propionic acid, 1.50% adipic acid, and 0.30% phenethyl alcohol" against *Candida albicans* was 26.05%;
2. It was evident in the results of Groups 2 and 3 in Table 4 that when the pH value was 4.3, the combinations of "0.54% propionic acid, 1.50% adipic acid, and 0.30% phenethyl alcohol" with 0.50% or 1.00% benzoic acid respectively had a bacteriostatic effect or a strong bacteriostatic effect against *Candida albicans,* and the inhibition rates were 64.80% and 100.00% respectively.

In summary, when the pH value was 4.3, 0.50% and 1.00% benzoic acid significantly affected the bacteriostatic effect of the solution containing "0.54% propionic acid, 1.50% adipic acid, and 0.30% phenethyl alcohol" against *Candida albicans,* and enhanced the bacteriostatic effect. The higher the concentration of benzoic acid was, the stronger the bacteriostatic effect against *Candida albicans* became.

### In vitro experiment V

According to the method described in Appendix C of GB 15979-2002 *Hygienic Standard for Disposable Sanitary Products,* aqueous solutions containing different ingredients were studied for their inhibition rates of 20 minutes of action against *Candida albicans* ATCC 10231, the percentage concentration of each ingredient therein was the weight percentage concentration % (w/w), and the pH value of each group was 4.6. The experimental results are shown in Table 5.

**Table 5 Bacteriostatic Effect of Different Solutions Against Candida albicans ATCC 10231**

| Serial number | Propionic acid (%) | Adipic acid (%) | Phenethyl alcohol (%) | Sodium benzoate (%) | Inhibition rate (%) |
|---|---|---|---|---|---|
| 1 | - | 1.50 | 0.30 | 1.18 | 84.12 |
| 2 | 2.00 | 1.50 | 0.30 | 1.18 | 100.00 |

Results:
1. It was evident in the result of Group 1 in Table 5 that when the pH value was 4.6, the solution containing "1.50% adipic acid, 0.30% phenethyl alcohol, and 1.18% sodium benzoate" had a bacteriostatic effect against *Candida albicans,* and the inhibition rate was 84.12%;
2. It was evident from the result of Group 2 in Table 5 that, when the pH value was 4.6, the solution containing "2.00% propionic acid, 1.50% adipic acid, 0.30% phenethyl alcohol, and 1.18% sodium benzoate" had a strong bacteriostatic effect against *Candida albicans,* and the inhibition rate was100.00%.

In summary, it could be seen from the comparison of experimental results of Group 1 and Group 2 that, when the pH value was 4.6, 2.00% propionic acid affected the bacteriostatic effect of the solution containing "1.50% adipic acid, 0.30% phenethyl alcohol, and 1.18% sodium benzoate" against *Candida albicans,* and enhanced the bacteriostatic effect.

### In vitro experiment VI

According to the method described in Appendix C of GB 15979-2002 *Hygienic Standard for Disposable Sanitary Products,* aqueous solutions containing different ingredients were studied for their inhibition rates of 20 minutes of action against *Candida albicans* ATCC 10231, the percentage concentration of each ingredient therein was the weight percentage concentration % (w/w), and the pH value of each group was 3.8. The experimental results are shown in Table 6.

**Table 6 Bacteriostatic Effect of Different Solutions Against Candida albicans ATCC 10231**

| Serial number | Butyric acid (%) | Hexanoic acid (%) | Adipic acid (%) | Phenethyl alcohol (%) | Phenoxyethanol (%) | Sodium benzoate (%) | Inhibition rate (%) |
|---|---|---|---|---|---|---|---|
| 1 | 0.88 | - | - | 0.40 | - | 0.20 | 71.52 |
| 2 | - | 0.12 | - | 0.40 | - | 0.20 | 70.65 |
| 3 | 0.88 | - | - | - | 0.40 | 0.20 | 56.75 |
| 4 | - | 0.12 | - | - | 0.40 | 0.20 | 35.88 |
| 5 | 0.88 | - | 1.46 | 0.40 | - | 0.20 | 100.00 |
| 6 | - | 0.12 | 1.46 | 0.40 | - | 0.20 | 100.00 |
| 7 | 0.88 | - | 1.46 | - | 0.40 | 0.20 | 100.00 |
| 8 | - | 0.12 | 1.46 | - | 0.40 | 0.20 | 100.00 |

Results:
1. It was evident in the results of Groups 1-2 in Table 6 that when the pH value was 3.8, the combinations of "0.20% sodium benzoate and 0.40% phenethyl alcohol" and two fatty acids of different concentrations (0.88% butyric acid, 0.12% hexanoic acid) respectively had a bacteriostatic effect against *Candida albicans,* and the inhibition rates were 71.52% and 70.65% respectively;
2. It was evident in the results of Groups 3-4 in Table 6 that when the pH value was 3.8, the solution containing "0.20% sodium benzoate, 0.40% phenoxyethanol, and 0.88% butyric acid" had a bacteriostatic effect against *Candida albicans,* and the inhibition rate was 56.75%; the solution containing "0.20% sodium benzoate, 0.40% phenoxyethanol, and 0.12% hexanoic acid" had an inhibition rate of 35.88% against *Candida albicans;*
3. It was evident in the results of Groups 5-6 in Table 6 that, when the pH value was 3.8, the combinations of "1.46% adipic acid, 0.20% sodium benzoate, and 0.40% phenethyl alcohol" and two fatty acids of different concentrations (0.88% butyric acid, 0.12% hexanoic acid) respectively had a strong bacteriostatic effect against *Candida albicans,* and the inhibition rates were 100%;
4. It was evident in the results of Groups 7-8 in Table 6 that when the pH value was 3.8, the combinations of "1.46% adipic acid, 0.20% sodium benzoate, and 0.40% phenoxyethanol" and two fatty acids of different concentrations (0.88% butyric acid, 0.12% hexanoic acid) respectively had a strong bacteriostatic effect against *Candida albicans,* and the inhibition rates were 100%.

In summary,
1. It was evident in the comparison of experimental results of Groups 5-6 and Groups 1-2 in Table 6 that, when the pH value was 3.8, 1.46% adipic acid affected the bacteriostatic effect of the solution containing "0.20% sodium benzoate, 0.40% phenethyl alcohol, and 0.88% butyric acid" and the solution containing "0.20% sodium benzoate, 0.40% phenethyl alcohol, and 0.12% hexanoic acid" against *Candida albicans* respectively, and enhanced the bacteriostatic effect;
2. It was evident in the comparison of experimental results of Groups 7-8 and Groups 3-4 in Table 6 that, when the pH value was 3.8, 1.46% adipic acid significantly affected the bacteriostatic effect of the solution containing "0.20% sodium benzoate, 0.40% phenoxyethanol, and 0.88% butyric acid" and the solution containing "0.20% sodium benzoate, 0.40% phenoxyethanol, and 0.12% hexanoic acid" against *Candida albicans* respectively, and enhanced the bacteriostatic effect.

### In vitro experiment VII

According to the method described in Appendix C of GB 15979-2002 *Hygienic Standard for Disposable Sanitary Products,* aqueous solutions containing different ingredients were studied for their inhibition rates of 20 minutes of action against *Candida albicans* ATCC 10231, the percentage concentration of each ingredient therein was the weight percentage concentration % (w/w), and the pH value of each group was 3.8. The experimental results are shown in Table 7.

**Table 7 Bacteriostatic Effect of Different Solutions Against Candida albicans ATCC 10231**

| Serial number | Propionic acid (%) | Pimelic acid (%) | Malic acid (%) | Phenethyl alcohol (%) | P-hydroxybenzoic acid (%) | Inhibition rate (%) |
|---|---|---|---|---|---|---|
| 1 | 2.50 | - | - | 0.06 | 0.21 | 62.76 |
| 2 | 2.50 | 1.60 | - | 0.06 | 0.21 | 94.84 |
| 3 | 2.50 | - | 1.34 | 0.06 | 0.21 | 65.68 |

Results:
1. It was evident in the result of Group 1 in Table 7 that, when the pH value was 3.8, the solution containing "0.21% p-hydroxybenzoic acid, 0.06% phenethyl alcohol, and 2.50% propionic acid" had a bacteriostatic effect against *Candida albicans,* and the inhibition rate was 62.76%;
2. It was evident in the result of Group 2 in Table 7 that, when the pH value was 3.8, the combination of 1.60% pimelic acid and "0.21% p-hydroxybenzoic acid, 0.06% phenethyl alcohol, and 2.50% propionic acid" had a strong bacteriostatic effect against *Candida albicans,* and the inhibition rate was 94.84%;
3. It was evident in the result of Group 3 in Table 7 that, when the pH value was 3.8, the combination of 1.34% malic acid and "0.21% p-hydroxybenzoic acid, 0.06% phenethyl alcohol, and 2.50% propionic acid" had a bacteriostatic effect against *Candida albicans,* and the inhibition rate was 65.68%;

In summary, it could be seen from the comparison of experimental results of Groups 1 and 2, and Groups 1 and 3 that, when the pH value was 3.8, 1.60% pimelic acid significantly affected the bacteriostatic effect of the solution containing "0.21% p-hydroxybenzoic acid, 0.06% phenethyl alcohol, and 2.50% propionic acid" against *Candida albicans,* and enhanced the bacteriostatic effect, while 1.34% malic acid did not affect the bacteriostatic effect of the solution containing "0.21% p-hydroxybenzoic acid, 0.06% phenethyl alcohol, and 2.50% propionic acid" against *Candida albicans.*

### In vitro experiment VIII

According to the method described in Appendix C of GB 15979-2002 *Hygienic Standard for Disposable Sanitary Products,* aqueous solutions containing different ingredients were studied for their bacteriostatic effects of 20 minutes of action against *Candida albicans* ATCC 10231, the percentage concentration of each ingredient therein was the weight percentage concentration % (w/w), and the pH value of each group was 3.9. The experimental results are shown in Table 8.

**Table 8 Bacteriostatic Effect of Different Solutions Against Candida albicans ATCC 10231**

| Serial number | Adipic acid (%) | Butyric acid (%) | Sodium benzoate (%) | Phenethyl alcohol (%) | Inhibition rate (%) |
|---|---|---|---|---|---|
| 1 | 1.46 | - | - | - | 11.52 |
| 2 | - | 0.60 | - | - | 1.57 |
| 3 | - | - | 0.14 | - | 19.90 |
| 4 | - | - | - | 0.40 | -1.05 |
| 5 | 1.46 | 0.60 | - | - | 13.61 |
| 6 | - | 0.60 | 0.14 | - | 17.80 |
| 7 | - | 0.60 | - | 0.40 | 8.90 |
| 8 | 1.46 | 0.60 | 0.14 | - | 27.23 |
| 9 | 1.46 | 0.60 | - | 0.40 | 25.65 |
| 10 | - | 0.60 | 0.14 | 0.40 | 2.62 |
| 11 | 1.46 | - | 0.14 | 0.40 | 42.41 |
| 12 | 1.46 | 0.60 | 0.14 | 0.40 | 89.53 |

Results:
1. It was evident in the results of Groups 1-4 in Table 8 that, when the pH value was 3.9, four solutions containing 1.46% adipic acid, 0.60% butyric acid, 0.14% sodium benzoate, and 0.40% phenethyl alcohol respectively had inhibition rates of 11.52%, 1.57%, 19.90%, and -1.05% respectively against *Candida albicans;*
2. It was evident in the result of Group 5 in Table 8 that, when the pH value was 3.9, the solution containing "1.46% adipic acid and 0.60% butyric acid" had an inhibition rate of 13.61% against *Candida albicans;*
3. It was evident in the result of Group 6 in Table 8 that, when the pH value was 3.9, the solution containing "0.60% butyric acid and 0.14% sodium benzoate" had an inhibition rate of 17.80% against *Candida albicans;*
4. It was evident in the result of Group 7 in Table 8 that, when the pH value was 3.9, the solution containing "0.60% butyric acid and 0.40% phenethyl alcohol" had an inhibition rate of 8.90% against *Candida albicans;*
5. It was evident in the result of Group 8 in Table 8 that, when the pH value was 3.9, the solution containing "1.46% adipic acid, 0.60% butyric acid, and 0.14% sodium benzoate" had an inhibition rate of 27.23% against *Candida albicans;*
6. It was evident in the result of Group 9 in Table 8 that, when the pH value was 3.9, the solution containing "1.46% adipic acid, 0.60% butyric acid, and 0.40% phenethyl alcohol" had an inhibition rate of 25.65% against *Candida albicans;*
7. It was evident in the result of Group 10 in Table 8 that, when the pH value was 3.9, the solution containing "0.60% butyric acid, 0.40% phenethyl alcohol, and 0.14% sodium benzoate" had an inhibition rate of 2.62% against *Candida albicans;*
8. It was evident in the result of Group 11 in Table 8 that, when the pH value was 3.9, the solution containing "1.46% adipic acid, 0.14% sodium benzoate, and 0.40% phenethyl alcohol" had an inhibition rate of 42.41% against *Candida albicans;*
9. It was evident in the result of Group 12 in Table 8 that, when the pH value was 3.9, the solution containing "1.46% adipic acid, 0.60% butyric acid, 0.14% sodium benzoate, and 0.40% phenethyl alcohol" had a bacteriostatic effect against *Candida albicans,* and the inhibition rate was 89.53%;

In summary, it could be seen that when the pH value was 3.9, the inhibition rate of the combination of 1.46% adipic acid, 0.60% butyric acid, 0.14% sodium benzoate, and 0.40% phenethyl alcohol against *Candida albicans* was much higher than that of each of the ingredients or the combination of any two or three of these ingredients, indicating the combination of these four ingredients had a synergistic bacteriostatic effect against *Candida albicans.*

### In vitro experiment IX

According to the method described in Appendix C of GB 15979-2002 *Hygienic Standard for Disposable Sanitary Products,* aqueous solutions containing different ingredients were studied for their bacteriostatic effects of 20 minutes of action against *Candida albicans* ATCC 10231, the percentage concentration of each ingredient therein was the weight percentage concentration % (w/w), and the pH value of each group was 4.3. The experimental results are shown in Table 9.

**Table 9 Bacteriostatic Effect of Different Solutions Against Candida albicans ATCC 10231**

| Serial number | Hexanoic acid (%) | Adipic acid (%) | Sodium benzoate (%) | Phenethyl alcohol (%) | Inhibition rate (%) |
|---|---|---|---|---|---|
| 1 | 0.18 | - | - | - | 2.82 |
| 2 | - | 1.35 | - | - | 5.81 |
| 3 | - | - | 0.20 | - | 6.95 |
| 4 | - | - | - | 0.30 | 0.59 |
| 5 | 0.18 | 1.35 | 0.20 | 0.30 | 58.86 |
| 6 | 0.18 | 1.35 | 0.20 | - | 25.17 |
| 7 | 0.18 | 1.35 | - | 0.30 | 9.33 |
| 8 | - | 1.35 | 0.20 | 0.30 | 15.37 |
| 9 | 0.18 | - | 0.20 | 0.30 | 28.78 |

Results:
1. It was evident in the results of Groups 1-4 in Table 9 that, when the pH value was 4.3, the inhibition rates against *Candida albicans* of four solutions containing 1.35% adipic acid, 0.20% sodium benzoate, 0.18% hexanoic acid, and 0.30% phenethyl alcohol respectively were all <10%;
2. It was evident in the result of Group 5 in Table 9 that, when the pH value was 4.3, the solution containing "1.35% adipic acid, 0.20% sodium benzoate, 0.18% hexanoic acid, and 0.30% phenethyl alcohol" had a bacteriostatic effect against *Candida albicans,* and the inhibition rate was 58.86%;
3. It was evident from the results of Groups 6-9 in Table 9 that, when the pH value was 4.3, the combination of any three ingredients selecting from1.35% adipic acid, 0.20% sodium benzoate, 0.18% hexanoic acid, and 0.30% phenethyl alcohol, had an inhibition rate of less than 50% against *Candida albicans.*

In summary, it could be seen that the inhibition rate of the combination of 1.35% adipic acid, 0.20% sodium benzoate, 0.18% hexanoic acid, and 0.30% phenethyl alcohol, against *Candida albicans was* significantly higher than that of the combination of any three of these four ingredients, and also significantly higher than the sum of the inhibition rate of each ingredient, indicating that the combination of these four ingredients had a synergistic bacteriostatic effect against *Candida albicans.*

### In vitro experiment X

According to the method described in Appendix C of GB 15979-2002 *Hygienic Standard for Disposable Sanitary Products,* aqueous solutions containing different ingredients were studied for their bacteriostatic effects of 20 minutes of action against *Candida albicans* ATCC 10231, the percentage concentration of each ingredient therein was the weight percentage concentration % (w/w), and the pH value of each group was 3.8. The experimental results are shown in Table 10.

**Table 10 Bacteriostatic Effect of Different Solutions Against Candida albicans ATCC 10231**

| Serial number | Propionic acid (%) | Adipic acid (%) | Cinnamic acid (%) | Phenethyl alcohol (%) | Salicylic acid (%) | Inhibition rate (%) |
|---|---|---|---|---|---|---|
| 1 | 1.50 | 1.50 | - | 0.45 | - | 30.96 |
| 2 | 1.50 | 1.50 | 0.03 | 0.45 | - | 59.04 |
| 3 | 1.50 | 1.50 | 0.05 | 0.45 | - | 71.19 |
| 4 | 1.50 | 1.50 | 0.08 | 0.45 | - | 96.38 |
| 5 | 1.50 | 1.50 | 0.10 | 0.45 | - | 100.00 |
| 6 | 1.50 | 1.50 | - | 0.45 | 0.03 | 37.40 |
| 7 | 1.50 | 1.50 | - | 0.45 | 0.05 | 55.58 |
| 8 | 1.50 | 1.50 | - | 0.45 | 0.08 | 86.96 |
| 9 | 1.50 | 1.50 | - | 0.45 | 0.10 | 94.81 |

Results:
1. It was evident in the result of Group 1 in Table 10 that, when the pH value was 3.8, the solution containing 1.50% adipic acid, 0.45% phenethyl alcohol, and 1.50% propionic acid had an inhibition rate of 30.96% against *Candida albicans;*
2. It was evident in the results of Groups 2-5 in Table 10 that, when the pH value was 3.8, the solution combining cinnamic acids of four different concentrations (0.03%, 0.05%, 0.08%, 0.10%) respectively with the solution containing "1.50% adipic acid, 0.45% phenethyl alcohol, and 1.50% propionic acid" had a bacteriostatic effect or a strong bacteriostatic effect against *Candida albicans,* and the inhibition rates were 59.04%, 71.19%, 96.38%, and 100.00% respectively;
3. It was evident in the results of Groups 6-9 in Table 10 that, when the pH value was 3.8, the combination of 0.03% salicylic acid with "1.50% adipic acid, 0.45% phenethyl alcohol, and 1.50% propionic acid" had an inhibition rate of 37.40% against *Candida albicans,* while the combination of salicylic acid of three higher concentrations (0.05%, 0.08%, 0.10%) respectively with "1.50% adipic acid, 0.45% phenethyl alcohol, and 1.50% propionic acid" had a bacteriostatic effect or a strong bacteriostatic effect against *Candida albicans,* and the inhibition rates were 55.58%, 86.96%, and 94.81%, respectively.

In summary, it could be seen that 0.03-0.10% cinnamic acid and 0.05-0.10% salicylic acid affected or significantly affected the bacteriostatic effect of the solution containing "1.50% adipic acid, 0.45% phenethyl alcohol, and 1.50% propionic acid", against *Candida albicans.* The higher the concentration of cinnamic acid or salicylic acid was, the further enhanced the bacteriostatic effect became.

### In vitro experiment XI

According to the method described in Appendix C of GB 15979-2002 *Hygienic Standard for Disposable Sanitary Products,* aqueous solutions containing different ingredients were studied for their bacteriostatic effects of 20 minutes of action against *Candida albicans* ATCC 10231, the percentage concentration of each ingredient therein was the weight percentage concentration % (w/w), and the pH value of each group was 3.8. The experimental results are shown in Table 11.

**Table 11 Bacteriostatic Effect of Different Solutions Against Candida albicans ATCC 10231**

| Serial number | Undecylic acid (%) | Undecylenic acid (%) | Lauric acid (%) | Adipic acid (%) | Phenethyl alcohol (%) | Sodium benzoate (%) | Inhibition rate (%) |
|---|---|---|---|---|---|---|---|
| 1 | 0.001 | | | - | | | 8.04 |
| 2 | 0.002 | | | - | | | 10.01 |
| 3 | - | 0.001 | - | - | - | - | 11.26 |
| 4 | - | 0.002 | - | - | - | - | 11.10 |
| 5 | - | - | 0.001 | - | - | - | 9.25 |
| 6 | - | - | 0.002 | - | - | - | 10.21 |
| 7 | - | - | - | 1.50 | 0.25 | 0.20 | 50.02 |
| 8 | 0.001 | - | - | 1.50 | 0.25 | 0.20 | 90.24 |
| 9 | 0.002 | - | - | 1.50 | 0.25 | 0.20 | 100.00 |
| 10 | - | 0.001 | - | 1.50 | 0.25 | 0.20 | 58.59 |
| 11 | - | 0.002 | - | 1.50 | 0.25 | 0.20 | 85.27 |
| 12 | - | - | 0.001 | 1.50 | 0.25 | 0.20 | 100.00 |
| 13 | - | - | 0.002 | 1.50 | 0.25 | 0.20 | 100.00 |

Results:
1. It was evident in the results of Groups 1-6 in Table 11 that, when the pH value was 3.8, each one of 0.001-0.002% undecylic acid, 0.001-0.002% undecylenic acid, or 0.001-0.002% lauric acid respectively had inhibition rates of <15% against *Candida albicans;*
2. It was evident in the result of Group 7 in Table 11 that, when the pH value was 3.8, the solution containing "0.20% sodium benzoate, 0.25% phenethyl alcohol, and 1.50% adipic acid" had a bacteriostatic effect against *Candida albicans,* and the inhibition rate was 50.02%;
3. It was evident in the results of Groups 8-9 in Table 11 that, when the pH value was 3.8, the combinations of "0.20% sodium benzoate, 0.25% phenethyl alcohol, and 1.50% adipic acid" and undecylic acid of two different concentrations (0.001%, 0.002%) respectively had a strong bacteriostatic effect against *Candida albicans,* and the inhibition rates were higher than 90%;
4. It was evident in the results of Groups 10-11 in Table 11 that, when the pH value was 3.8, the combinations of "0.20% sodium benzoate, 0.25% phenethyl alcohol, and 1.50% adipic acid" and undecylenic acid of two different concentrations (0.001%, 0.002%) respectively had a bacteriostatic effect against *Candida albicans,* and the inhibition rates were higher than 50%;
5. It was evident in the results of Groups 12-13 in Table 11 that, when the pH value was 3.8, the combinations of "0.20% sodium benzoate, 0.25% phenethyl alcohol, and 1.50% adipic acid" and lauric acid of two different concentrations (0.001%, 0.002%) respectively had a strong bacteriostatic effect against *Candida albicans,* and the inhibition rates were 100%.

In summary, it could be seen that 0.001-0.002% undecylic acid, 0.002% undecylenic acid, and 0.001-0.002% lauric acid significantly affected the bacteriostatic effect of the solution containing "0.20% sodium benzoate, 0.25% phenethyl alcohol, and 1.50% adipic acid" against *Candida albicans* respectively, and enhanced the bacteriostatic effect.

### In vitro experiment XII

According to the method described in Appendix C of GB 15979-2002 *Hygienic Standard for Disposable Sanitary Products,* aqueous solutions containing different ingredients were studied for their bacteriostatic effects of 20 minutes of action against *Candida albicans* ATCC 10231, and the percentage concentration of each ingredient therein was the weight percentage concentration % (w/w). The experimental results are shown in Tables 12-14.

**Table 12 Bacteriostatic Effect of Each One of Different Solutions Against Candida albicans ATCC 10231**

| pH | Propionic acid (%) | Butyric acid (%) | Valeric acid (%) | Hexanoic acid (%) | Heptanoic acid (%) | Caprylic acid (%) | Nonanoic acid (%) | Capric acid (%) | Undecylic acid (%) | Lauric acid (%) | Sodium benzoate (%) | Inhibition rate (%) |
|---|---|---|---|---|---|---|---|---|---|---|---|---|
| 3.6 | 2.00 | | | | | | - | | | | | 18.22 |
| 3.8 | - | 2.20 | | | | | - | | | | | 3.78 |
| 3.8 | - | - | 0.60 | | | | | - | | | | -0.51 |
| 3.8 | - | - | - | 0.20 | | | | - | | | | -0.31 |
| 3.8 | | | | | 0.07 | | | | - | | | 2.31 |
| 3.8 | | | - | | | 0.02 | | | - | | | 9.96 |
| 3.8 | | | - | | | | 0.009 | - | - | - | - | 17.04 |
| 3.8 | | | | - | | | | 0.002 | - | - | - | 6.12 |
| 3.8 | | | | - | | | | | 0.001 | - | - | -14.39 |
| 3.8 | | | | | - | | | | | 0.001 | - | 10.65 |
| 3.8 | | | | | | - | | | | | 0.25 | 3.73 |

**Table 13 Bacteriostatic Effect of Solutions Containing Two Different Ingredients Against Candida albicans ATCC 10231**

| pH | Propionic acid (%) | Butyric acid (%) | Valeric acid (%) | Hexanoic acid (%) | Heptanoic acid (%) | Caprylic acid (%) | Nonanoic acid (%) | Capric acid (%) | Undecylic acid (%) | Lauric acid (%) | Sodium benzoate (%) | Inhibition rate (%) |
|---|---|---|---|---|---|---|---|---|---|---|---|---|
| 3.6 | 2.00 | | | | | - | | | | | 0.25 | 57.20 |
| 3.8 | - | 2.20 | | | | | - | | | | 0.25 | 66.39 |
| 3.8 | - | - | 0.60 | | | | - | | | | 0.25 | 70.52 |
| 3.8 | | - | | 0.20 | | | | - | | | 0.25 | 56.02 |
| 3.8 | | - | | | 0.07 | | | - | | | 0.25 | 79.51 |
| 3.8 | | | - | | | 0.02 | - | - | - | - | 0.20 | 59.09 |
| 3.8 | | | - | | | | 0.009 | - | - | - | 0.20 | 58.85 |
| 3.8 | | | | - | | | | 0.002 | - | - | 0.20 | 50.55 |
| 3.8 | | | | | - | | | | 0.001 | - | 0.20 | 61.65 |
| 3.8 | | | | | - | | | | | 0.001 | 0.20 | 73.69 |

**Table 14 Bacteriostatic Effect of Solutions Containing Three Different Ingredients Against Candida albicans ATCC 10231**

| PH | Propionic acid (%) | Butyric acid (%) | Valeric acid (%) | Hexanoic acid (%) | Heptanoic acid (%) | Caprylic acid (%) | Nonanoic acid (%) | Capric acid (%) | Undecylic acid (%) | Lauric acid (%) | Phenethyl alcohol (%) | Sodium benzoate (%) | Inhibition rate (%) |
|---|---|---|---|---|---|---|---|---|---|---|---|---|---|
| 3.6 | 1.11 | | | | | - | | | | | 0.40 | 0.20 | 55.62 |
| 3.8 | - | 0.88 | | | | | - | | | | 0.40 | 0.20 | 61.90 |
| 3.8 | - | - | 0.40 | - | - | - | - | - | - | - | 0.40 | 0.20 | 81.89 |
| 3.8 | - | - | - | 0.15 | - | - | - | - | - | - | 0.40 | 0.20 | 82.82 |
| 3.8 | | - | | | 0.05 | - | - | - | - | - | 0.40 | 0.20 | 75.89 |
| 3.8 | | | - | | | 0.02 | - | - | - | - | 0.40 | 0.20 | 88.31 |
| 3.8 | | | - | | | | 0.005 | - | - | - | 0.40 | 0.20 | 77.98 |
| 3.8 | | | | - | | | | 0.002 | - | - | 0.40 | 0.20 | 82.58 |
| 3.8 | | | | | - | | | | 0.001 | - | 0.40 | 0.20 | 96.94 |
| 3.8 | | | | | - | | | | | 0.001 | 0.40 | 0.20 | 100.00 |

Results:
1. It was evident in the results in Table 12 that, when the pH value was 3.6-3.8, each one of the ten fatty acids of different concentrations (2.00% propionic acid, 2.20% butyric acid, 0.60% valeric acid, 0.20% hexanoic acid, 0.07% heptanoic acid, 0.02% caprylic acid, 0.009% nonanoic acid, 0.002% capric acid, 0.001% undecylic acid, 0.001% lauric acid) or 0.25% sodium benzoate respectively had inhibition rates of <20% against *Candida albicans;*
2. It was evident in the results in Table 13 that, when the pH value the 3.6-3.8, the combinations of 0.20-0.25% sodium benzoate with each one of the ten fatty acids of different concentrations (2.00% propionic acid, 2.20% butyric acid, 0.60% valeric acid, 0.20% hexanoic acid, 0.07% heptanoic acid, 0.02% caprylic acid, 0.009% nonanoic acid, 0.002% capric acid, 0.001% undecylic acid, 0.001% lauric acid) respectively had a bacteriostatic effect against *Candida albicans,* and the inhibition rates were >50%, indicating that the combination of these two ingredients had a synergistic bacteriostatic effect against *Candida albicans,* and enhanced the bacteriostatic effect;
3. It was evident in the results in Table 14 that, when the pH value was 3.6-3.8, the combinations of "0.20% sodium benzoate and 0.40% phenethyl alcohol" with each one of the ten fatty acids of different concentrations (2.00% propionic acid, 2.20% butyric acid, 0.60% valeric acid, 0.20% hexanoic acid, 0.07% heptanoic acid, 0.02% caprylic acid, 0.009% nonanoic acid, 0.002% capric acid, 0.001% undecylic acid, 0.001% lauric acid) respectively had a bacteriostatic effect or a strong bacteriostatic effect against *Candida albicans,* and the inhibition rates were 50%-90% or >90%, indicating that the combination of these three ingredients had a synergistic bacteriostatic effect against *Candida albicans,* and enhanced the bacteriostatic effect;

### In vitro experiment XIII

According to the method described in Appendix C of GB 15979-2002 *Hygienic Standard for Disposable Sanitary Products,* aqueous solutions containing different ingredients were studied for their bacteriostatic effects of 20 minutes of action against *Candida albicans* ATCC 10231, the percentage concentration of each ingredient therein was the weight percentage concentration % (w/w), and the pH value of each group was 3.8. The experimental results are shown in Table 15.

**Table 15 Bacteriostatic Effect of Different Solutions Against Candida albicans ATCC 10231**

| Serial number | Propionic acid (%) | Butyric acid (%) | Valeric acid (%) | Hexanoic acid (%) | Heptanoic acid (%) | Caprylic acid (%) | Capric acid (%) | Lauric acid (%) | Undecylic acid (%) | Cinnamyl alcohol (%) | Inhibition rate (%) |
|---|---|---|---|---|---|---|---|---|---|---|---|
| 1 | 2.00 | | | | | - | | | | | 12.31 |
| 2 | - | 2.20 | | | | | - | | | | 10.76 |
| 3 | - | - | 0.60 | | | | - | | | | 19.70 |
| 4 | | - | | 0.20 | | | | - | | | 5.27 |
| 5 | | | - | | 0.07 | | | - | | | 8.25 |
| 6 | | | - | | | 0.02 | | | - | | 7.93 |
| 7 | | | - | | | | 0.005 | | - | | 22.00 |
| 8 | | | | - | | | | 0.003 | - | - | 20.00 |
| 9 | | | | | - | | | | 0.003 | - | 20.00 |
| 10 | | | | | - | | | | | 0.30 | 7.38 |
| 11 | 2.00 | | | | | - | | | | 0.30 | 81.66 |
| 12 | - | 2.20 | | | | - | | | | 0.30 | 100.00 |
| 13 | - | - | 0.60 | | | | - | | | 0.30 | 100.00 |
| 14 | | - | | 0.20 | | | - | | | 0.30 | 100.00 |
| 15 | | | - | | 0.07 | | | - | | 0.30 | 100.00 |
| 16 | | | - | | | 0.02 | | - | | 0.30 | 100.00 |
| 17 | | | - | | | | 0.005 | - | - | 0.30 | 98.40 |
| 18 | | | | - | | | | 0.003 | - | 0.30 | 54.40 |
| 19 | | | | | - | | | | 0.003 | 0.30 | 81.60 |

Results:
1. It was evident in the results of Groups 1-10 in Table 15 that, when the pH value was 3.8, each one of the nine fatty acids of different concentrations (2.00% propionic acid, 2.20% butyric acid, 0.60% valeric acid, 0.20% hexanoic acid, 0.07% heptanoic acid, 0.02% caprylic acid, 0.005% capric acid, 0.003% lauric acid, 0.003% undecylic acid) or 0.30% cinnamyl alcohol respectively had inhibition rates of <25% against *Candida albicans;*
2. It was evident in the results of Groups 11-19 in Table 15 that, when the pH value was 3.8, the combinations of each one of the nine fatty acids of different concentrations (2.00% propionic acid, 2.2% butyric acid, 0.60% valeric acid, 0.20% hexanoic acid, 0.07% heptanoic acid, 0.02% caprylic acid, 0.005% capric acid, 0.003% lauric acid, 0.003% undecylic acid) respectively with 0.30% cinnamyl alcohol had a bacteriostatic effect or a strong bacteriostatic effect against *Candida albicans,* and the inhibition rates were 50%-90% or >90%, indicating that the combination of these two ingredients had a synergistic bacteriostatic effect against *Candida albicans,* and enhanced the bacteriostatic effect.

### In vitro experiment XIV

According to the method described in Appendix C of GB 15979-2002 *Hygienic Standard for Disposable Sanitary Products,* aqueous solutions containing different ingredients were studied for their inhibition rates of 20 minutes of action against *Candida albicans* ATCC 10231, the percentage concentration of each ingredient therein was the weight percentage concentration % (w/w), and the pH value of each group was 3.8. The experimental results are shown in Table 16.

**Table 16 Bacteriostatic Effect of Different Solutions Against Candida albicans ATCC 10231**

| Serial number | Butyric acid (%) | Pimelic acid (%) | Phenethyl alcohol (%) | Inhibition rate (%) |
|---|---|---|---|---|
| 1 | 1.00 | - | - | 1.22 |
| 2 | - | 1.00 | - | 2.09 |
| 3 | - | - | 0.60 | 4.98 |
| 4 | 1.00 | - | 0.60 | 11.09 |
| 5 | 1.00 | 1.00 | 0.60 | 65.22 |

Results:
1. It was evident in the results of Groups 1-3 in Table 16 that, when the pH value was 3.8, each one of 1.00% butyric acid, 1.00% pimelic acid, or 0.60% phenethyl alcohol respectively had inhibition rates of <10% against *Candida albicans;*
2. It was evident in the result of Group 4 in Table 16 that, when the pH value was 3.8, the inhibition rate of the solution containing 1.00% butyric acid and 0.60% phenethyl alcohol against *Candida albicans* was 11.09%;
3. It was evident in the result of Group 5 in Table 16 that, when the pH value was 3.8, the solution containing 1.00% butyric acid, 1.00% pimelic acid, and 0.60% phenethyl alcohol had a bacteriostatic effect against *Candida albicans,* and the inhibition rate was 65.22%.

In summary, it could be seen that the solution containing 1.00% butyric acid, 1.00% pimelic acid, and 0.60% phenethyl alcohol had a synergistic bacteriostatic effect against *Candida albicans,* and enhanced the bacteriostatic effect.

### Experimental example XV

According to the method described in Appendix C of GB 15979-2002 *Hygienic Standard for Disposable Sanitary Products,* aqueous solutions containing sodium propionate and dicarboxylic acid or tricarboxylic acid were studied for their bacteriostatic effects of 20 minutes of action against *Candida albicans* ATCC 10231, the percentage concentration of each ingredient therein was the weight percentage concentration % (w/w), and the pH value of each group was 3.6. The experimental results are shown in Table 17.

**Table 17 Bacteriostatic Effect of Aqueous Solutions Containing Different Ingredients Against Candida albicans ATCC 10231**

| Serial number | Sodium propionate (%) | Adipic acid (%) | Succinic acid (%) | Malic acid (%) | Tartaric acid (%) | Maleic acid (%) | Citric acid (%) | Fumaric acid (%) | Inhibition rate (%) |
|---|---|---|---|---|---|---|---|---|---|
| 1 | 2.88 | | | | - | | | | 19.32 |
| 2 | - | 4.38 | | | - | | | | 17.87 |
| 3 | - | - | 3.54 | | | - | | | 10.14 |
| 4 | - | - | - | 4.02 | | | - | | 20.29 |
| 5 | - | - | - | - | 4.50 | - | - | - | 8.70 |
| 6 | | | - | | | 3.50 | - | - | 18.36 |
| 7 | | | - | | | | 5.76 | - | 20.29 |
| 8 | | | | - | | | | 3.50 | 11.11 |
| 9 | 1.25 | 4.38 | | | - | | | | 72.46 |
| 10 | 1.25 | - | 3.54 | | | - | | | 10.14 |
| 11 | 1.25 | - | - | 4.02 | | | - | | 7.73 |
| 12 | 1.25 | - | - | - | 4.05 | - | - | - | 1.45 |
| 13 | 1.25 | | - | | | 3.50 | - | - | 9.66 |
| 14 | 1.25 | | | - | | | 5.00 | - | 7.25 |
| 15 | 1.25 | | | - | | | | 3.50 | 15.94 |

Results:
1. It was evident in the results of Groups 1-8 in Table 17 that, when the pH value was 3.6, each one of the 2.88% sodium propionate and seven dicarboxylic acids or polybasic carboxylic acids of different concentrations (4.38% adipic acid, 3.54% succinic acid, 4.02% malic acid, 4.50% tartaric acid, 3.50% maleic acid, 5.76% citric acid, 3.50% fumaric acid) respectively had inhibition rates of <25% against *Candida albicans;*
2. It was evident in the result of Group 9 in Table 17 that, when the pH value was 3.6, the solution containing 1.25% sodium propionate and 4.38% adipic acid had a bacteriostatic effect against *Candida albicans,* and the inhibition rate was 72.46%;
3. It was evident in the results of Groups 10-15 in Table 17 that, when the pH value was 3.6, the combinations of 1.25% sodium propionate and each one of the six dicarboxylic acids or polybasic carboxylic acids of different concentrations (3.54% succinic acid, 4.02% malic acid, 4.50% tartaric acid, 3.50% maleic acid, 5.76% citric acid, 3.50% fumaric acid) respectively had inhibition rates of <25% against *Candida albicans.*

In summary, it could be seen that the combination of adipic acid and sodium propionate had a synergistic bacteriostatic effect against *Candida albicans.*

### Experimental example XVI

According to the method described in Appendix C of GB 15979-2002 *Hygienic Standard for Disposable Sanitary Products,* the combination of citric acid, malic acid, or succinic acid respectively with a basic bacteriostatic solution containing "1.46% (w/w) adipic acid + 0.16% (w/w) sodium benzoate + 0.62% (w/w) propionic acid + 0.35% (w/w) phenethyl alcohol" was studied for the bacteriostatic effect of 20 minutes of action against *Candida albicans* ATCC 10231, the percentage concentration of each ingredient therein was the weight percentage concentration % (w/w), and the pH value of the basic bacteriostatic solution was 3.6. The experimental results are shown in Table 18.

**Table 18 Effects of Citric Acid, Malic Acid, and Succinic Acid on the Bacteriostatic Effect**

| Serial number | Adipic acid (%) | Sodium benzoate (%) | Propionic acid (%) | Phenethyl alcohol (%) | Citric acid (%) | Malic acid (%) | Succinic acid (%) | Inhibition rate (%) |
|---|---|---|---|---|---|---|---|---|
| 1 | 1.46 | 0.16 | 0.62 | 0.35 | - | - | - | 53.77 |
| 2 | 1.46 | 0.16 | 0.62 | 0.35 | 1.00 | - | - | 49.25 |
| 3 | 1.46 | 0.16 | 0.62 | 0.35 | - | 0.70 | - | 54.77 |
| 4 | 1.46 | 0.16 | 0.62 | 0.35 | - | - | 0.61 | 53.27 |

Results:
1. The basic bacteriostatic solution had a bacteriostatic effect against *Candida albicans,* and the inhibition rate was 53.77%;
2. The combinations of basic bacteriostatic solution with 1.00% citric acid, 0.70% malic acid, or 0.61% succinic acid respectively had inhibition rates of 49.25%, 54.77%, and 53.27% against *Candida albicans* respectively.

In summary, it could be seen that citric acid, malic acid, or succinic acid did not affect the bacteriostatic effect of the basic bacteriostatic solution containing "1.46% (w/w) adipic acid + 0.16% (w/w) sodium benzoate + 0.62% (w/w) propionic acid + 0.35% (w/w) phenethyl alcohol" against *Candida albicans.*

### In vitro experiment XVII

According to the method described in Appendix C of GB 15979-2002 *Hygienic Standard for Disposable Sanitary Products,* aqueous solutions containing different ingredients were studied for their inhibition rates of 20 minutes of action against *Candida albicans* ATCC 10231, the percentage concentration of each ingredient therein was the weight percentage concentration % (w/w), and the pH value of each group was 3.8. The experimental results are shown in Table 19.

**Table 19 Bacteriostatic Effect of Different Solutions Against Candida albicans ATCC 10231**

| Serial number | Butyric acid (%) | Valeric acid (%) | Hexanoic acid (%) | Heptanoic acid (%) | Caprylic acid (%) | Nonanoic acid (%) | Capric acid (%) | Adipic acid (%) | Pimelic acid (%) | Inhibition rate (%) |
|---|---|---|---|---|---|---|---|---|---|---|
| 1 | 1.32 | | | | | - | | | | 2.86 |
| 2 | - | 0.70 | | | | - | | | | -3.56 |
| 3 | - | - | 0.20 | | | | - | | | 10.25 |
| 4 | - | - | - | 0.12 | | | - | | | 34.00 |
| 5 | | - | | | 0.03 | | | - | | 15.28 |
| 6 | | | - | | | 0.0075 | - | - | - | 21.36 |
| 7 | | | - | | | | 0.005 | - | - | 16.25 |
| 8 | | | | - | | | | 1.50 | - | 0.30 |
| 9 | | | | | - | | | | 2.00 | 2.41 |
| 10 | 1.32 | | | | - | | | 1.50 | - | 51.28 |
| 11 | - | 0.70 | | | - | | | 1.50 | - | 71.25 |
| 12 | - | - | 0.20 | | | - | | 1.50 | - | 50.12 |
| 13 | - | - | - | 0.12 | - | - | - | 1.50 | - | 70.66 |
| 14 | | - | | | 0.03 | - | - | 1.50 | - | 62.26 |
| 15 | | - | | | | 0.0075 | - | 1.50 | - | 58.49 |
| 16 | | | - | | | | 0.005 | 1.50 | - | 97.92 |
| 17 | 1.32 | | | | - | | | | 2.00 | 81.33 |
| 18 | - | 0.70 | | | | - | | | 2.00 | 90.26 |
| 19 | - | - | 0.20 | | | - | | | 2.00 | 70.25 |
| 20 | - | - | - | 0.12 | | - | | | 2.00 | 97.14 |
| 21 | | - | | | 0.03 | - | - | - | 2.00 | 80.70 |
| 22 | | | - | | | 0.0075 | - | - | 2.00 | 62.54 |
| 23 | | | - | | | | 0.005 | - | 2.00 | 98.25 |

Results:
1. It was evident in the results of Groups 1-9 in Table 19 that, when the pH value was 3.8, each one of the seven fatty acids of different concentrations (1.32% butyric acid, 0.70% valeric acid, 0.20% hexanoic acid, 0.12% heptanoic acid, 0.03% caprylic acid, 0.0075% nonanoic acid, 0.005% capric acid) and two dicarboxylic acids of different concentrations (1.50% adipic acid, 2.00% pimelic acid) respectively had inhibition rates of <35% against *Candida albicans;*
2. It was evident in the results of Groups 10-16 in Table 19 that, when the pH value was 3.8, the combinations of each one of the seven fatty acids of different concentrations (1.32% butyric acid, 0.70% valeric acid, 0.20% hexanoic acid, 0.12% heptanoic acid, 0.03% caprylic acid, 0.0075% nonanoic acid, 0.005% capric acid) respectively with 1.50% adipic acid had a bacteriostatic effect or a strong bacteriostatic effect against *Candida albicans,* and the inhibition rates were 50%-90% or >90%;
3. It was evident in the results of Groups 17-23 in Table 19 that, when the pH value was 3.8, the combinations of each one of the seven fatty acids of different concentrations (1.32% butyric acid, 0.70% valeric acid, 0.20% hexanoic acid, 0.12% heptanoic acid, 0.03% caprylic acid, 0.0075% nonanoic acid, 0.005% capric acid) respectively with 2.00% pimelic acid had a bacteriostatic effect or a strong bacteriostatic effect against *Candida albicans,* and the inhibition rates were 50%-90% or >90%.

In summary, it could be seen that, when the pH value was 3.8, the combinations of each one of the seven fatty acids of different concentrations (1.32% butyric acid, 0.70% valeric acid, 0.20% hexanoic acid, 0.12% heptanoic acid, 0.03% caprylic acid, 0.0075% nonanoic acid, 0.005% capric acid) respectively and 1.50% adipic acid or 2.00% pimelic acid respectively had a synergistic bacteriostatic effect against *Candida albicans,* and enhanced the bacteriostatic effect.

### In vitro experiment XVIII

According to the method described in Appendix C of GB 15979-2002 *Hygienic Standard for Disposable Sanitary Products,* aqueous solutions containing different ingredients were studied for their inhibition rates of 20 minutes of action against *Escherichia coli* ATCC 25922, *Staphylococcus aureus* ATCC 6538, and *Candida albicans* ATCC 10231, the percentage concentration of each ingredient therein was the weight percentage concentration % (w/w), and the pH value of each group was 3.8. The experimental results are shown in Table 20.

**Table 20 Bacteriostatic Effect of Different Solutions Against Escherichia coli, Staphylococcus aureus and Candida albicans**

| Serial number | Butyric acid (%) | Hexanoic acid (%) | Heptanoic acid (%) | Caprylic acid (%) | Nonanoic acid (%) | Capric acid (%) | Undecylic acid (%) | Undecylenic acid (%) | Lauric acid (%) | Pimelic acid (%) | Inhibition rate (%) | | |
|---|---|---|---|---|---|---|---|---|---|---|---|---|---|
| | | | | | | | | | | | *Candida albicans* | *Escherichia coli* | *Staphylococcus aureus* |
| 1 | 3.00 | | | | | - | | | | | 55.00 | 100.00 | 100.00 |
| 2 | - | 0.45 | | | | | - | | | | 100.00 | 100.00 | 100.00 |
| 3 | - | - | 0.30 | | | | - | | | | 100.00 | 100.00 | 100.00 |
| 4 | - | - | - | 0.05 | | | | - | | | 100.00 | 100.00 | 100.00 |
| 5 | | | | | 0.03 | | | - | | | 100.00 | 100.00 | 100.00 |
| 6 | | | - | | | 0.008 | - | - | - | - | 100.00 | 100.00 | 100.00 |
| 7 | | | | | | | 0.006 | - | - | - | 78.49 | 100.00 | 100.00 |
| 8 | | | | - | | | | 0.006 | - | - | 71.68 | 100.00 | 100.00 |
| 9 | | | | | - | | | | 0.006 | - | 50.28 | 100.00 | 100.00 |
| 10 | | | | | - | | | | | 5.00 | 52.23 | 100.00 | 100.00 |

It was evident in the results in Table 20 that, when the pH value was 3.8, each one of the nine fatty acids of different concentrations (3.00% butyric acid, 0.45% hexanoic acid, 0.30% heptanoic acid, 0.05% caprylic acid, 0.03% nonanoic acid, 0.008% capric acid, 0.006% undecylic acid, 0.006% undecylenic acid, 0.006% lauric acid) respectively and 5.00% pimelic acid had a bacteriostatic effect or a strong bacteriostatic effect against *Candida albicans,* and the inhibition rates were 50%-90% or >90%, and had a strong bacteriostatic effect against *Escherichia coli and Staphylococcus aureus,* and the inhibition rates were 100.00%.

### In vitro experiment XIX

According to the method described in Appendix C of GB 15979-2002 *Hygienic Standard for Disposable Sanitary Products,* aqueous solutions containing different ingredients were studied for their bacteriostatic effects of 20 minutes of action against *Candida albicans* ATCC 10231, the percentage concentration of each ingredient therein was the weight percentage concentration % (w/w), and the pH value of each group was 3.8. The experimental results are shown in Table 21.

**Table 21 Bacteriostatic Effect of Different Solutions Against Candida albicans ATCC 10231**

| Serial number | Propionic acid (%) | Pimelic acid (%) | Cinnamyl alcohol (%) | Sodium benzoate (%) | Inhibition rate (%) |
|---|---|---|---|---|---|
| 1 | 2.00 | 1.50 | - | 0.20 | 41.25 |
| 2 | 2.00 | 1.50 | 0.03 | 0.20 | 72.15 |
| 3 | 2.00 | 1.50 | 0.05 | 0.20 | 84.54 |
| 4 | 2.00 | 1.50 | 0.08 | 0.20 | 95.21 |

Results:
1. It was evident in the result of Group 1 in Table 21 that, when the pH value was 3.8, the solution containing 1.50% pimelic acid, 0.20% sodium benzoate, and 2.00% propionic acid had an inhibition rate of 41.25% against *Candida albicans;*
2. It was evident in the results of Groups 2-4 in Table 21 that, when the pH value was 3.8, the combinations of cinnamyl alcohol of 0.03%, 0.05%, or 0.08% respectively with "1.50% pimelic acid, 0.20% sodium benzoate, and 2.00% propionic acid" had a bacteriostatic effect or a strong bacteriostatic effect against *Candida albicans,* and the inhibition rates were 50%-90% or >90% respectively.

In summary, it could be seen that each one of the cinnamyl alcohol of 0.03, 0.05%, or 0.08% significantly affected the bacteriostatic effect of the solution containing "1.50% pimelic acid, 0.20% sodium benzoate, and 2.00% propionic acid" against *Candida albicans* respectively, and enhanced the bacteriostatic effect.

### In vitro experiment XX

According to the method described in Appendix C of GB 15979-2002 *Hygienic Standard for Disposable Sanitary Products,* aqueous solutions containing different ingredients were studied for their bacteriostatic effects of 20 minutes of action against *Candida albicans* ATCC 10231, the percentage concentration of each ingredient therein was the weight percentage concentration % (w/w), and the pH value of each group was 4.8. The experimental results are shown in Table 22.

**Table 22 Bacteriostatic Effect of Different Solutions Against Candida albicans ATCC 10231**

| Serial number | Propionic acid (%) | Pimelic acid (%) | Sodium benzoate (%) | Inhibition rate (%) |
|---|---|---|---|---|
| 1 | - | 5.00 | - | 15.42 |
| 2 | - | - | 1.18 | 15.92 |
| 3 | 0.10 | - | - | 2.90 |
| 4 | 0.10 | 5.00 | 1.18 | 61.02 |

Results:
1. It was evident in the results of Groups 1-3 in Table 22 that, when the pH value was 4.8, each one of 5.00% pimelic acid, 1.18% sodium benzoate, or 0.10% propionic acid respectively had inhibition rates of less than 20% against *Candida albicans;*
2. It was evident in the result of Group 4 in Table 22 that, when the pH value was 4.8, the solution containing "5.00% pimelic acid, 1.18% sodium benzoate, and 0.10% propionic acid" had a bacteriostatic effect against *Candida albicans,* and the inhibition rate was 61.02%.

In summary, it could be seen that, when the pH value was 4.8, the solution containing 5.00% pimelic acid, 1.18% sodium benzoate, and 0.10% propionic acid had a synergistic bacteriostatic effect against *Candida albicans,* and enhanced the bacteriostatic effect.

### In vitro experiment XXI

According to the method described in Appendix C of GB 15979-2002 *Hygienic Standard for Disposable Sanitary Products,* aqueous solutions containing different ingredients were studied for their inhibition rates of 20 minutes of action against *Escherichia coli* ATCC 25922 and *Staphylococcus aureus* ATCC 6538, the percentage concentration of each ingredient therein was the weight percentage concentration % (w/w), and the pH value of each group was 4.0. The experimental results are shown in Table 23.

**Table 23 Bacteriostatic Effect of Different Solutions Against Escherichia coli ATCC 25922 and Staphylococcus aureus ATCC 6538**

| Serial number | Propionic acid (%) | Butyric acid (%) | Hexanoic acid (%) | Adipic acid (%) | Phenethyl alcohol (%) | Sodium benzoate (%) | Inhibition rate (%) | |
|---|---|---|---|---|---|---|---|---|
| | | | | | | | *Escherichia coli* | *Staphylococcus aureus* |
| 1 | | | - | | 0.30 | 0.06 | 64.80 | 80.00 |
| 2 | 0.50 | | | - | | 0.06 | 53.43 | 56.27 |
| 3 | - | 0.45 | | - | | 0.06 | 51.86 | 77.30 |
| 4 | - | - | 0.06 | - | - | 0.06 | 67.84 | 68.11 |
| 5 | 0.50 | | - | | 0.30 | - | 50.53 | 65.95 |
| 6 | - | 0.45 | - | - | 0.30 | - | 88.24 | 80.00 |
| 7 | - | - | 0.06 | - | 0.30 | - | 50.00 | 72.97 |
| 8 | 0.35 | | - | | 0.30 | 0.045 | 74.51 | 83.24 |
| 9 | - | 0.30 | - | - | 0.30 | 0.045 | 97.55 | 98.38 |
| 10 | - | - | 0.06 | - | 0.30 | 0.045 | 90.69 | 97.30 |
| 11 | 0.35 | - | - | 1.50 | 0.30 | 0.045 | 97.06 | 71.89 |
| 12 | - | 0.30 | - | 1.50 | 0.30 | 0.045 | 99.02 | 78.92 |
| 13 | - | - | 0.06 | 1.50 | 0.30 | 0.045 | 99.02 | 88.65 |

Results:
1. It was evident in the results of Groups 1-4 in Table 23 that, when the pH value was 4.0, the combinations of 0.06% sodium benzoate and each one of 0.30% phenethyl alcohol, 0.50% propionic acid, 0.45% butyric acid, or 0.06% hexanoic acid respectively had a bacteriostatic effect against *Escherichia coli* and *Staphylococcus aureus,* and the inhibition rates were higher than 50%;
2. It was evident in the results of Groups 5-7 in Table 23 that, when the pH value was 4.0, the combinations of 0.30% phenethyl alcohol and each one of 0.50% propionic acid, 0.45% butyric acid, or 0.06% hexanoic acid respectively had a bacteriostatic effect against *Escherichia coli* and *Staphylococcus aureus,* and the inhibition rates were higher than 50%;
3. It was evident in the results of Groups 8-10 in Table 23 that, when the pH value was 4.0, the combination of "0.045% sodium benzoate, 0.30% phenethyl alcohol" and 0.35% propionic acid had a bacteriostatic effect against *Escherichia coli* and *Staphylococcus aureus,* and the inhibition rates were higher than 50%; the combinations of "0.045% sodium benzoate, 0.30% phenethyl alcohol" and 0.30% butyric acid or 0.06% hexanoic acid respectively had a strong bacteriostatic effect against *Escherichia coli* and *Staphylococcus aureus,* and the inhibition rates were higher than 90%;
4. It was evident in the results of Groups 11-13 in Table 23 that, when the pH value was 4.0, the combinations of "1.50% adipic acid, 0.045% sodium benzoate, and 0.30% phenethyl alcohol" and each one of the three fatty acids of different concentrations (0.35% propionic acid, 0.30% butyric acid, 0.06% hexanoic acid) respectively had a strong bacteriostatic effect against *Escherichia coli,* and the inhibition rates were higher than 90%; and had a bacteriostatic effect against *Staphylococcus aureus,* and the inhibition rates were 50%-90%.

### In vitro experiment XXII

According to the method described in Appendix C of GB 15979-2002 *Hygienic Standard for Disposable Sanitary Products,* aqueous solutions containing different ingredients were studied for their bacteriostatic effects of 20 minutes of action against *Candida albicans* ATCC 10231, and the percentage concentration of each ingredient therein was the weight percentage concentration % (w/w). The experimental results are shown in Table 24.

**Table 24 Bacteriostatic Effect of Different Solutions Against Candida albicans ATCC 10231**

| Serial number | pH | Propionic acid (%) | Butyric acid (%) | Citric acid (%) | Adipic acid (%) | Phenethyl alcohol (%) | Sodium benzoate (%) | Inhibition rate (%) |
|---|---|---|---|---|---|---|---|---|
| 1 | 4.0 | 0.20 | 0.60 | 0.06 | 1.17 | 0.40 | 0.20 | 86.56 |
| 2 | 4.2 | 0.20 | 0.60 | 0.06 | 1.17 | 0.40 | 0.20 | 66.74 |
| 3 | 4.4 | 0.20 | 0.60 | 0.06 | 1.17 | 0.40 | 0.20 | 37.61 |

### Results:

It was evident in the results of Groups 1-3 in Table 24 that, when the pH value was 4.0 and 4.2 respectively, the solution containing "0.06% citric acid, 1.17% adipic acid, 0.20% propionic acid, 0.60% butyric acid, 0.20% sodium benzoate, and 0.40% phenethyl alcohol" had a bacteriostatic effect against *Candida albicans,* and the inhibition rates were 86.56% and 66.74% respectively. When the pH value was 4.4, the solution containing these ingredients had an inhibition rate of 37.61% against *Candida albicans.*

In summary, it could be seen that the lower the pH value of the solution containing "0.06% citric acid, 1.17% adipic acid, 0.2.0% propionic acid, 0.60% butyric acid, 0.20% sodium benzoate, and 0.40% phenethyl alcohol" was, the stronger the bacteriostatic effect of the solution against *Candida albicans* became.

### In vitro experiment XXIII

According to the method described in Appendix C of GB 15979-2002 *Hygienic Standard for Disposable Sanitary Products,* aqueous solutions containing different ingredients were studied for their bacteriostatic effects of 20 minutes of action against *Candida albicans* ATCC 10231, the percentage concentration of each ingredient therein was the weight percentage concentration % (w/w), and the pH value of each group was 3.86. The experimental results are shown in Table 25.

**Table 25 Bacteriostatic Effect of Different Solutions Against Candida albicans ATCC 10231**

| Serial number | Palatinose (%) | Propionic acid (%) | Butyric acid (%) | Adipic acid (%) | Malic acid (%) | Phenethyl alcohol (%) | Sodium benzoate (%) | Puerarin (%) | Inhibition rate (%) |
|---|---|---|---|---|---|---|---|---|---|
| 1 | 1.80 | 0.20 | 0.45 | 1.46 | 0.20 | 0.30 | 0.12 | - | 75.14 |
| 2 | 1.80 | 0.20 | 0.45 | 1.46 | 0.20 | 0.30 | 0.12 | 0.04 | 78.45 |
| 3 | 1.80 | 0.20 | 0.45 | 1.46 | 0.20 | 0.30 | 0.12 | 0.06 | 76.24 |

Results:
1. It was evident in the result of Group 1 in Table 25 that, when the pH value was 3.86, the solution containing "1.46% adipic acid, 0.20% malic acid, 0.12% sodium benzoate, 0.20% propionic acid, 0.45% butyric acid, 0.30% phenethyl alcohol, and 1.80% palatinose" had a bacteriostatic effect against *Candida albicans,* and the inhibition rate was 75.14%;
2. It was evident in the result of Group 2 in Table 25 that, when the pH value was 3.86, the solution containing "1.46% adipic acid, 0.20% malic acid, 0.12% sodium benzoate, 0.20% propionic acid, 0.45% butyric acid, 0.30% phenethyl alcohol, 1.80% palatinose, and 0.04% puerarin" had a bacteriostatic effect against *Candida albicans,* and the inhibition rate was 78.45%;
3. It was evident in the result of Group 3 in Table 25 that, when the pH value was 3.86, the solution containing "1.46% adipic acid, 0.20% malic acid, 0.12% sodium benzoate, 0.20% propionic acid, 0.45% butyric acid, 0.30% phenethyl alcohol, 1.80% palatinose, and 0.06% puerarin" had a bacteriostatic effect against *Candida albicans,* and the inhibition rate was 76.24%.

In summary, it could be seen that when the pH value of the solution was 3.86, the 0.04% and 0.06% puerarin contained in the solution respectively did not affect the inhibitory effect of the solution against *Candida albicans,* and did not weaken the bacteriostatic effect.

Understandably, other estrogens such as diethylstilbestrol, hexoestrol, estradiol, estrone, estriol, nilestriol, ethinyloestradiol, quinestrol, mestranol, and promestriene, and other phytoestrogens such as daidzin, daidzein, glycitein, puerarin, coumestrol, genistein, equol, apigenin, genistin, genisteol, biochanin, coumestrol, formononetin, resveratrol, secoisolariciresinol, and lignan are similar to puerarin in property and action principle. Therefore, it can be inferred that said estrogens and phytoestrogens will not significantly weaken the inhibitory effect of the compositions against *Candida albicans.*

### In vitro experiment XXIV

According to the method described in Appendix C of GB 15979-2002 *Hygienic Standard for Disposable Sanitary Products,* aqueous solutions containing different ingredients were studied for their bacteriostatic effects of 20 minutes of action against *Candida albicans* ATCC 10231, the percentage concentration of each ingredient therein was the weight percentage concentration %

(w/w), and the pH value of each group was 4.0. The experimental results are shown in Table 26.

**Table 26 Bacteriostatic Effect of Different Solutions Against Candida albicans ATCC 10231**

| Serial number | Propionic acid (%) | Malic acid (%) | Adipic acid (%) | Phenethyl alcohol (%) | Sodium benzoate (%) | Glutamic acid (%) | Inhibition rate (%) |
|---|---|---|---|---|---|---|---|
| 1 | 1.67 | 0.14 | 1.46 | 0.40 | 0.20 | 0.55 | 55.23 |
| 2 | 1.67 | 0.14 | 1.46 | 0.40 | 0.20 | - | 51.68 |

### Results:

It was evident in the comparison of experimental results of Group 1 to Group 2 in Table 26 that, when the pH value was 4.0, 0.55% glutamic acid did not affect the bacteriostatic effect of the solution containing "0.14% malic acid, 1.46% adipic acid, 0.20% sodium benzoate, 1.67% propionic acid, and 0.40% phenethyl alcohol" against *Candida albicans.*

Understandably, other appropriate amino acids such as glutamine, L-aspartic acid, asparagine, leucine, isoleucine, phenylalanine, valine, proline, and threonine are similar to glutamic acid in property and action principle. Therefore, it can be inferred that said amino acids will not affect the inhibitory effect of the compositions against *Candida albicans.*

### In vitro experiment XXV

According to the method described in Appendix C of GB 15979-2002 *Hygienic Standard for Disposable Sanitary Products,* aqueous solutions containing different ingredients were studied for their bacteriostatic effects of 20 minutes of action against *Escherichia coli* ATCC 25922 and *Staphylococcus aureus* ATCC 6538, the percentage concentration of each ingredient therein was the weight percentage concentration % (w/w), and the pH value of each group was 3.8. The experimental results are shown in Table 27.

**Table 27 Bacteriostatic Effect of Different Solutions Against Escherichia coli ATCC 25922 and Staphylococcus aureus ATCC 6538**

| Serial number | Adipic acid (%) | Propionic acid (%) | Butyric acid (%) | Valeric acid (%) | Hexanoic acid (%) | Cinnamyl alcohol (%) | Cinnamic acid (%) | Inhibition rate (%) | |
|---|---|---|---|---|---|---|---|---|---|
| | | | | | | | | *Escherichia coli* | *Staphylococcus aureus* |
| 1 | 1.75 | 1.50 | | | - | | | 100.00 | 68.57 |
| 2 | 1.75 | - | 1.30 | | - | | | 100.00 | 90.00 |
| 3 | 1.75 | - | - | 0.50 | | - | | 100.00 | 94.29 |
| 4 | 1.75 | | - | | 0.20 | - | - | 100.00 | 91.43 |
| 5 | 1.75 | 1.50 | | - | | 0.03 | - | 100.00 | 100.00 |
| 6 | 1.75 | - | 1.30 | - | - | 0.03 | - | 100.00 | 100.00 |
| 7 | 1.75 | - | - | 0.50 | - | 0.03 | - | 100.00 | 100.00 |
| 8 | 1.75 | | - | | 0.20 | 0.03 | - | 100.00 | 100.00 |
| 9 | 1.75 | 1.50 | | - | | | 0.03 | 100.00 | 100.00 |
| 10 | 1.75 | - | 1.30 | | - | | 0.03 | 100.00 | 100.00 |
| 11 | 1.75 | - | - | 0.50 | - | - | 0.03 | 100.00 | 100.00 |
| 12 | 1.75 | | - | | 0.20 | - | 0.03 | 100.00 | 100.00 |

Results:
1. It was evident in the results of Groups 1-4 in Table 27 that, when the pH value was 3.8, the combinations of 1.75% adipic acid and each one of 1.50% propionic acid, 1.30% butyric acid, 0.50% valeric acid, or 0.20% hexanoic acid respectively had a strong bacteriostatic effect against *Escherichia coli,* and the inhibition rates were 100.00%, and had a bacteriostatic effect or a strong bacteriostatic effect against *Staphylococcus aureus,* and the inhibition rates were 50%-90% or >90%;
2. It was evident in the results of Groups 5-8 in Table 27 that, when the pH value was 3.8, the combinations of "0.03% cinnamyl alcohol and 1.75% adipic acid" and each one of 1.50% propionic acid, 1.30% butyric acid, 0.50% valeric acid, and 0.20% hexanoic acid respectively had a strong bacteriostatic effect against *Escherichia coli* and *Staphylococcus aureus,* and the inhibition rates were 100.00%;
3. It was evident in the results of Groups 9-12 in Table 27 that, when the pH value was 3.8, the combinations of "0.03% cinnamic acid and 1.75% adipic acid" and each one of 1.50% propionic acid, 1.30% butyric acid, 0.50% valeric acid, or 0.20% hexanoic acid respectively had a strong bacteriostatic effect against *Escherichia coli and Staphylococcus aureus,* and the inhibition rates were 100.00%.

### In vitro experiment XXVI

Basically by referring to the methods and results determination criteria in Volume IV 1121 Bacteriostatic Effectiveness Testing of the *Pharmacopoeia of the People's Republic of China* 2020, the bacteriostatic composition was studied for their bacteriostatic effects against *Staphylococcus aureus, Pseudomonas aeruginosa, Escherichia coli, Candida albicans,* and *Aspergillus niger.* The experimental results are shown in Table 28.

**Table 28 Antimicrobial Effectiveness Testing of a Composition¹**

| Test bacteria | pH of the composition pH | Initial bacterial count (cfu/mL) | Bacterial count (cfu/mL) | | Ig value reduced² | |
|---|---|---|---|---|---|---|
| | | | Day 14 | Day 28 | Day 14 | Day 28 |
| *Staphylococcus aureus* | 4.2 | 2.8×10⁵ | 0 | 0 | 5 | NI³ |
| | 4.4 | 2.4×10⁵ | 0 | 0 | 5 | NI |
| *Pseudomonas aeruginosa* | 4.2 | 2.1 × 10⁵ | 0 | 0 | 5 | NI |
| | 4.4 | 2.1×10⁵ | 0 | 0 | 5 | NI |
| *Escherichia coli* | 4.2 | 1.5×10⁵ | 0 | 0 | 5 | NI |
| | 4.4 | 1.6×10⁵ | 0 | 0 | 5 | NI |
| *Candida albicans* | 4.2 | 1.1×10⁵ | 0 | 0 | 5 | NI |
| | 4.4 | 1.1×10⁵ | 1.1×10⁴ | 1.1×10³ | 1 | NI |
| *Aspergillus niger* | 4.2 | 1.0×10⁵ | 0 | 0 | 5 | NI |
| | 4.4 | 1.0×10⁵ | 1.0×10⁵ | 1.0×10³ | 0 | NI |

| | | | | | | |
|---|---|---|---|---|---|---|
| Note: 1. The composition comprised: 1.50% (w/w) adipic acid, 0.14% (w/w) sodium benzoate, 0.32% (w/w) phenethyl alcohol, 0.54% (w/w) propionic acid, 0.20% (w/w) hexanoic acid, 0.20% (w/w) palatinose, 0.60% (w/w) maltose, 0.0005% (w/w) rose essential oil, and 2.15% (w/w) xanthan gum. 2. The difference between the Ig value of bacteria count measured at each interval and the Ig value of the count of bacteria inoculated in 1 mL (g) of the test sample. 3. No increase (NI) is defined as not more than 0.5 Ig in the test bacteria count compared to the previous measurement time | | | | | | |

It was evident in the results in Table 28 that, when the pH value was 4.2, the reduced logarithm of the bacterial concentration was 5 on day 14 relative to day 0, and did not increase on day 28 relative to day 14 for all test bacteria, which met the requirements. It could be seen that the composition with a pH value of 4.2 in the test had an antiseptic effect.

It should be noted that even if the experimental conditions and methods are identical, the results of the in vitro bacteriostatic experiments, such as inhibition rate, may still differ due to the existence of experimental errors when the experiments are repeated, but such differences are reasonable and are understandable to those skilled in the art. Therefore, if there is any inconsistency between the in vitro experimental data in this disclosure and the data in the previous application documents, the data in this disclosure shall prevail.

### In vivo experiment I

Three groups of gels containing different ingredients respectively were vaginally administered to Cynomolgus Monkeys, 0.5 mL once a day, for five consecutive days. Vaginal swabs were collected for the test of pH value and for smear staining and microscopic examination to observe the effects of the gels on the pH value of vaginal secretions and on the vaginal flora of Cynomolgus Monkeys. The experimental results are shown in Table 29:

**Table 29 Effects of Bacteriostatic Gels Containing Different Ingredients on Vaginal Acidity and Vaginal Flora of Cynomolgus Monkeys**

| Serial number | Animal No. | Pre-administration | | Single administration | | Three administrations | | Five administrations | |
|---|---|---|---|---|---|---|---|---|---|
| | | pH | Nugent score⁴ | pH | Nugent score | pH | Nugent score | pH | Nugent score |
| B¹ | C1406056 | 5.4 | >7 | 3.8 | 4-6 | 3.8 | 0-3 | 3.8 | 0-3 |
| | 1501042 | 5.4 | >7 | 4.4 | 4-6 | 4.1 | 0-3 | 3.8 | 0-3 |
| | C1309138 | 5.4 | >7 | 4.1 | 4-6 | 3.8 | 0-3 | 3.8 | 0-3 |
| C² | 1510080 | 5.4 | >7 | 3.8 | 4-6 | 3.8 | 0-3 | 3.8 | 0-3 |
| | 1508068 | 5.4 | >7 | 5.4 | >7 | 3.8 | 4-6 | 3.8 | 4-6 |
| | C1405048 | 5.4 | >7 | 3.8 | 4-6 | 3.8 | 4-6 | 3.8 | 4-6 |
| 2³ | 1508188 | 5.4 | >7 | 5.4 | >7 | 3.8 | 0-3 | 3.8 | 0-3 |
| | 1508154 | 5.4 | >7 | 4.1 | 4-6 | 4.1 | 0-3 | 3.8 | 0-3 |
| | 1512032 | 5.4 | >7 | 5.4 | 4-6 | 5.4 | >7 | Menstruation | / |
| | 1501120 | 5.4 | >7 | 4.4 | 4-6 | 3.8 | 4-6 | 3.8 | 0-3 |
| | 1510080 | 5.4 | >7 | 4.1 | 4-6 | 5.4 | 4-6 | 4.4 | >7 |

| | | | | | | | | | |
|---|---|---|---|---|---|---|---|---|---|
| Note: 1: The gel of Group B comprised 2.50% (w/w) maltose, 0.20% (w/w) isomaltulose, 0.16% (w/w) sodium benzoate, 0.60% (w/w) sodium propionate, 0.35% (w/w) phenethyl alcohol, 1.00% (w/w) citric acid, 1.168% (w/w) adipic acid, and 2.15% (w/w) xanthan gum, with pH value adjusted to 3.6 2: The gel of Group C comprised 2.50% (w/w) maltose, 0.20% (w/w) isomaltulose, 0.16% (w/w) sodium benzoate, 0.60% (w/w) sodium propionate, 0.35% (w/w) phenethyl alcohol, 0.61% (w/w) succinic acid, 1.168% (w/w) adipic acid, and 2.15% (w/w) xanthan gum, with pH value adjusted to 3.6 3: The gel of Group 2 comprised 1.15% (w/w) sodium propionate, 0.45% (w/w) phenethyl alcohol, 0.20% (w/w) sodium benzoate, 1.50% (w/w) maltose, 1.00% (w/w) citric acid, and 2.15% (w/w) xanthan gum, with pH value adjusted to 3.6 4: Nugent score refers to the scoring based on the staining, morphology, and quantity of bacteria under microscopic examination of vaginal secretions after smearing and Gram staining, specified as follows: ≥7: Vaginal flora is mainly gram-negative bacilli, and/or negative cocci and positive cocci, with no or few gross gram-positive bacilli; 4-6: Vaginal flora is mainly gram-negative bacilli, and/or negative cocci and positive cocci, with few gross gram-positive bacilli; 0-3: Vaginal flora is normal, with mainly gross gram-positive bacilli. | | | | | | | | | |

Results:
1. It was evident in the results of Group B in Table 29 that, after five administrations of the gel that had a pH value of 3.6 and contained "2.50% (w/w) maltose, 0.20% (w/w) isomaltulose, 0.16% (w/w) sodium benzoate, 0.60% (w/w) sodium propionate, 0.35% (w/w) phenethyl alcohol, 1.00% (w/w) citric acid, 1.168% (w/w) adipic acid, and 2.15% (w/w) xanthan gum", the pH values of the vaginal secretions decreased to 3.8 and the Nugent scores of the vaginal flora decreased to 0-3 in all the three Cynomolgus Monkeys;
2. It was evident in the results of Group C in Table 29 that, after five administrations of the gel that had a pH value of 3.6 and contained "2.50% (w/w) maltose, 0.20 (w/w) isomaltulose, 0.16% (w/w) sodium benzoate, 0.60% (w/w) sodium propionate, 0.35% (w/w) phenethyl alcohol, 0.61% (w/w) succinic acid, 1.168% (w/w) adipic acid, and 2.15% (w/w) xanthan gum", the pH values of the vaginal secretions decreased to 3.8 in all the three Cynomolgus Monkeys, and the Nugent scores of the vaginal flora decreased to 0-3 in one Cynomolgus Monkey and to 4-6 in two Cynomolgus Monkeys;
3. It was evident in the results of Group 2 in Table 29 that, after five administrations of the gel that had a pH value of 3.6 and contained "1.15% (w/w) sodium propionate, 0.45% (w/w) phenethyl alcohol, 0.20% (w/w) sodium benzoate, 1.50% (w/w) maltose, 1.00% (w/w) citric acid, and 2.15% (w/w) xanthan gum", the pH values of the vaginal secretions decreased to 3.8 in three of the five Cynomolgus Monkeys and to 4.4 in one Cynomolgus Monkey, and one terminated due to menstruation; and the Nugent scores of the vaginal flora decreased to 0-3 in three Cynomolgus Monkeys, one remained >7, and one terminated due to menstruation.

In summary, it could be seen that all the three groups of gels, which had a pH value of 3.6, had the efficacies of inhibiting abnormal vaginal flora, restoring vaginal acidity, and restoring vaginal lactobacilli. Between the gels of Groups B and C, which both contained adipic acid, and contained either citric acid or succinic acid respectively, Group B had better efficacy than Group C and Group 2, the latter did not contain adipic acid. The gel of Group 2 containing no adipic acid had the weakest efficacy in inhibiting abnormal vaginal flora, in restoring vaginal acidity, and in restoring vaginal lactobacilli among the three groups of gels.

### In vivo experiment II

Three groups of gels containing different ingredients respectively were vaginally administered to Rhesus Monkeys, 0.5 ml once a day, for five consecutive days. Vaginal secretions were collected for the test of pH value and for smear staining and microscopic examination, to observe the effects of the gels on the pH values of vaginal secretions and on the vaginal flora of Rhesus Monkeys. The experimental results are shown in Table 30:

**Table 30 Effects of Bacteriostatic Gels Containing Different Ingredients on Vaginal Acidity and Vaginal Flora of Rhesus Monkeys**

| Serial number | Animal No. | Pre-administration | | Single administration | | Three administrations | | Five administrations | |
|---|---|---|---|---|---|---|---|---|---|
| | | pH | Nugent score⁴ | pH | Nugent score | pH | Nugent score | pH | Nugent score |
| A¹ | 15208 | 5.4 | >7 | 5.4 | >7 | 4.1 | 4-6 | 5.4 | 4-6 |
| | 15196 | 5.4 | >7 | 3.8 | 4-6 | 3.8 | 0-3 | 3.8 | 0-3 |
| | 16152 | 5.4 | >7 | 5.4 | >7 | 5.4 | 4-6 | 3.8 | 0-3 |
| | 16286 | 5.4 | >7 | 5.4 | 4-6 | 3.8 | 4-6 | 3.8 | 0-3 |
| B² | 15176 | 5.4 | >7 | 5.4 | >7 | 3.8 | 4-6 | 5.4 | 4-6 |
| | 17036 | 5.4 | >7 | 5.4 | >7 | 4.1 | 4-6 | 4.1 | 4-6 |
| | 1310354 | 5.4 | >7 | 5.4 | 4-6 | 3.8 | 0-3 | 3.8 | 0-3 |
| | 1306108 | 5.4 | >7 | 3.8 | 0-3 | 3.8 | 0-3 | 3.8 | 0-3 |
| | 1-4± | 5.4 | >7 | 5.4 | 4-6 | 3.8 | 0-3 | 3.8 | 0-3 |
| C³ | 1401040 | 5.4 | >7 | 5.4 | 4-6 | 3.8 | 0-3 | 3.8 | 0-3 |
| | 1308498 | 5.4 | >7 | 4.1 | 4-6 | 5.4 | 4-6 | 4.1 | 4-6 |

| | | | | | | | | | |
|---|---|---|---|---|---|---|---|---|---|
| Note: 1: The gel of Group A comprised 2.00% (w/w) isomaltulose, 0.12% (w/w) sodium benzoate, 0.60% (w/w) butyric acid, 1.31% (w/w) adipic acid, 0.30% (w/w) phenethyl alcohol, 0.35% (w/w) malic acid, and 2.15% (w/w) xanthan gum, with pH value adjusted to 4.1 2: The gel of Group B comprised 2.00% (w/w) isomaltulose, 0.12% (w/w) sodium benzoate, 0.60% (w/w) butyric acid, 0.0010% (w/w) capric acid, 1.31% (w/w) adipic acid, 0.30% (w/w) phenethyl alcohol, 0.35% (w/w) malic acid, and 2.15% (w/w) xanthan gum, with pH value adjusted to 4.1 3: The gel of Group C comprised 2.00% (w/w) isomaltulose, 0.12% (w/w) sodium benzoate, 0.60% (w/w) butyric acid, 0.0015% (w/w) capric acid, 1.31% (w/w) adipic acid, 0.30% (w/w) phenethyl alcohol, 0.35% (w/w) malic acid, and 2.15% (w/w) xanthan gum, with pH value adjusted to 4.1 4: Same as Note 4 in in vivo experiment I | | | | | | | | | |

Results:
1. It was evident in the results of Group A that, after five administrations of the gel, which had a pH value of 4.1 and contained "1.31% (w/w) adipic acid, 0.35% (w/w) malic acid, 0.30% (w/w) phenethyl alcohol, 0.12% (w/w) sodium benzoate, and 0.60% (w/w) butyric acid", as well as 2.00% (w/w) isomaltulose and 2.15% (w/w) xanthan gum, the pH values of vaginal secretions decreased to 3.8 in three of the four Rhesus Monkeys, while their Nugent scores of the vaginal flora decreased to 0-3; the pH value of vaginal secretion remained 5.4 in one Rhesus Monkey, and the Nugent score of vaginal flora decreased to 4-6.
2. It was evident in the results of Group B that, after five administrations of the gel, which had a pH value of 4.1 and contained "1.31% (w/w) adipic acid, 0.35% (w/w) malic acid, 0.30% (w/w) phenethyl alcohol, 0.12% (w/w) sodium benzoate, 0.60% (w/w) butyric acid, and 0.0010% (w/w) capric acid", as well as 2.00% (w/w) isomaltulose and 2.15% (w/w) xanthan gum, the pH values of vaginal secretions decreased to 3.8 in three of the five Rhesus Monkeys, while their Nugent scores of the vaginal flora decreased to 0-3; the pH value decreased to 4.1 in one of the rest, while the Nugent score decreased to 4-6; and the pH value remained 5.4 in another one of the rest, while the Nugent score decreased to 4-6.
3. It was evident in the results of Group C that, after five administrations of the gel, which had a pH value of 4.1 and contained "1.31% (w/w) adipic acid, 0.35% (w/w) malic acid, 0.30% (w/w) phenethyl alcohol, 0.12% (w/w) sodium benzoate, 0.60% (w/w) butyric acid, and 0.0015% (w/w) capric acid", as well as 2.0% (w/w) isomaltulose and 2.15% (w/w) xanthan gum, the pH values of vaginal secretions decreased to 3.8 in one of the two Rhesus Monkeys, while the Nugent score of the vaginal flora decreased to 0-3; and the pH value in another Rhesus Monkeys decreased to 4.1, while the Nugent score decreased to 4-6.

In summary, it could be seen that the three groups of gels containing 0.60% (w/w) butyric acid, 0.00125% (w/w) capric acid, or 0.0015% (w/w) capric acid respectively in the experiment had the effect of inhibiting abnormal vaginal flora, modulating vaginal flora, restoring vaginal lactobacilli, and restoring vaginal acidity to normal.

### In vivo experiment III

Five groups of gels containing different ingredients respectively were vaginally administered to Cynomolgus Monkeys, 0.5 mL once a day, for five consecutive days. Vaginal swabs were collected for the test of pH value and for smear staining and microscopic examination to observe the effects of the gels on the pH values of vaginal secretions and on the vaginal flora of Cynomolgus Monkeys. The experimental results are shown in Table 31:

**Table 31 Effects of Bacteriostatic Gels Containing Different Ingredients on Vaginal Acidity and Vaginal Flora of Cynomolgus Monkeys**

| Serial number | Animal No. | Pre-administration | | Single administration | | Three administrations | | Five administrations | |
|---|---|---|---|---|---|---|---|---|---|
| | | pH | Nugent score⁶ | pH | Nugent score | pH | Nugent score | pH | Nugent score |
| 1¹ | 175458c | 5.4 | >7 | 4.1 | >7 | 4.1 | 4-6 | 3.8 | 4-6 |
| | 1506228 | 5.4 | >7 | 5.4 | 4-6 | 4.1 | 4-6 | 4.1 | 4-6 |
| | 1508188 | 5.4 | >7 | 3.8 | 4-6 | 4.1 | 4-6 | 3.8 | 0-3 |
| | 1504422 | 5.4 | >7 | 3.8 | 4-6 | 4.1 | 4-6 | 4.1 | 0-3 |
| 2² | 1300048 | 5.4 | >7 | 3.8 | 4-6 | 3.8 | 0-3 | 3.8 | 0-3 |
| | 1601022 | 5.4 | >7 | 3.8 | 0-3 | 3.8 | 0-3 | 3.8 | 0-3 |
| 3³ | 1512032 | 5.4 | >7 | 5.4 | 4-6 | 4.1 | 4-6 | 3.8 | 4-6 |
| | 1508068 | 5.4 | >7 | 4.1 | 0-3 | 3.8 | 0-3 | 4.1 | 0-3 |
| | 1502116 | 5.4 | >7 | 3.8 | 0-3 | 3.8 | 0-3 | 4.1 | 0-3 |
| 4⁴ | 1512078 | 5.4 | >7 | 4.1 | 4-6 | 3.8 | 0-3 | 3.8 | 0-3 |
| | 1104018 | 5.4 | >7 | 3.8 | 0-3 | 3.8 | 0-3 | 3.8 | 4-6 |
| 5⁵ | 1105022 | 5.4 | >7 | 3.8 | 4-6 | 3.8 | 0-3 | 3.8 | 0-3 |
| | 1501024 | 5.4 | >7 | 4.1 | 4-6 | 3.8 | 0-3 | 3.8 | 0-3 |

| | | | | | | | | | |
|---|---|---|---|---|---|---|---|---|---|
| Note: 1. The gel of Group 1 comprised 0.14% (w/w) sodium benzoate, 0.54% (w/w) propionic acid, 1.46% (w/w) adipic acid, 0.30% (w/w) phenethyl alcohol, 0.35% (w/w) malic acid, and 2.15% (w/w) xanthan gum, with pH value adjusted to 3.8 2: The gel of Group 2 comprised 2.00% (w/w) maltose, 0.14% (w/w) sodium benzoate, 0.54% (w/w) propionic acid, 1.46% (w/w) adipic acid, 0.30% (w/w) phenethyl alcohol, 0.35% (w/w) malic acid, and 2.15% (w/w) xanthan gum, with pH value adjusted to 3.8 3: The gel of Group 3 comprised 2.0% (w/w) isomaltulose, 0.14% (w/w) sodium benzoate, 0.54% (w/w) propionic acid, 1.46% (w/w) adipic acid, 0.30% (w/w) phenethyl alcohol, 0.35% (w/w) malic acid, and 2.15% (w/w) xanthan gum, with pH value adjusted to 3.8 4: The gel of Group 4 comprised 2.00% (w/w) isomaltulose, 0.12% (w/w) sodium benzoate, 0.38% (w/w) propionic acid, 1.31% (w/w) adipic acid, 0.0015% (w/w) capric acid, 0.30% (w/w) phenethyl alcohol, 0.35% (w/w) malic acid, and 2.15% (w/w) xanthan gum, with pH value adjusted to 3.9 5: The gel of Group 5 comprised 2.00% (w/w) isomaltulose, 0.12% (w/w) sodium benzoate, 0.38% (w/w) propionic acid, 1.31% (w/w) adipic acid, 0.01% (w/w) caprylic acid, 0.30% (w/w) phenethyl alcohol, 0.35% (w/w) malic acid, and 2.15% (w/w) xanthan gum, with pH value adjusted to 3.9 6: Same as Note 4 in in vivo experiment I | | | | | | | | | |

Results:
1. It was evident in the results of Groups 1-3 in Table 31 that all of the ingredients except saccharide of the gels of the three groups are identical. The gel of Group 1 did not contain any saccharide, the gel of Group 2 contained 2.00% (w/w) maltose, and the gel of Group 3 contained 2.00% (w/w) isomaltulose. The gels had the same pH value of 3.8, and all contained "0.14% (w/w) sodium benzoate, 0.54% (w/w) propionic acid, 1.46% (w/w) adipic acid, 0.30% (w/w) phenethyl alcohol, 0.35% (w/w) malic acid, and 2.15% (w/w) xanthan gum".
   After five administrations, the pH values of vaginal secretions decreased to 4.1 or below in all three groups; the Nugent scores of the vaginal flora in Group 1 without saccharide decreased to 4-6 in two of the four Cynomolgus Monkeys, and to 0-3 in the other two Cynomolgus Monkeys; The Nugent scores in Group 2 with maltose decreased to 0-3 in two Cynomolgus Monkeys. The Nugent scores in Group 3 with isomaltulose decreased to 0-3 in two of the three Cynomolgus Monkeys and to 4-6 in the other one Cynomolgus Monkey.
2. It was evident in the results of Group 4 in Table 31 that, after five administrations of the gel, which had a pH value of 3.9 and contained"2.00% (w/w) isomaltulose, 0.12% (w/w) sodium benzoate, 0.38% (w/w) propionic acid, 1.31% (w/w) adipic acid, 0.30% (w/w) phenethyl alcohol, 0.35% (w/w) malic acid, 0.0015% (w/w) capric acid, and 2.15% (w/w) xanthan gum", the pH values of vaginal secretions decreased to 3.8 in two Cynomolgus Monkeys, and the Nugent score of the vaginal flora decreased to 0-3 in one Cynomolgus Monkey and to 4-6 in another Cynomolgus Monkey.
3. It was evident in the results of Group 5 in Table 31 that, after three administrations of the gel, which had a pH value of 3.9 and contained "2.00% (w/w) isomaltulose, 0.12% (w/w) sodium benzoate, 0.38% (w/w) propionic acid, 1.31% (w/w) adipic acid, 0.30% (w/w) phenethyl alcohol, 0.35% (w/w) malic acid, 0.01% (w/w) caprylic acid, and 2.15% (w/w) xanthan gum", the pH values of vaginal secretions decreased to 3.8 and the Nugent score of the vaginal flora decreased to 0-3 in two Cynomolgus Monkeys.

In summary, it could be seen that:
1. The gel which had a pH value of 3.8 and contained "1.46% (w/w) adipic acid, 0.35% (w/w) malic acid, 0.54% (w/w) propionic acid, 0.30% (w/w) phenethyl alcohol, and 0.14% (w/w) sodium benzoate" but no saccharide had the efficacy of inhibiting abnormal vaginal flora, restoring vaginal acidity, and restoring vaginal lactobacilli, as shown in the results of Group 1. However, the gel of Group 2 which contains maltose further and the gel of Group 3 which contains isomaltulose further had a faster and stronger effect in restoring vaginal lactobacilli than the gel of Group 1 without saccharide, as shown in the results of Group 2 and Group 3.
2. The gels which contained "1.31% (w/w) adipic acid, 0.35% (w/w) malic acid, 0.38% (w/w) propionic acid, 0.30% (w/w) phenethyl alcohol, 0.12% (w/w) sodium benzoate, and 2.0% (w/w) isomaltulose", as well as 0.0015% (w/w) capric acid or 0.01% (w/w) caprylic acid respectively had the efficacy of inhibiting abnormal vaginal flora, restoring the vaginal acidity, and restoring vaginal lactobacilli, as shown in the results of Group 4 and Group 5.

### In vivo experiment IV

Three groups of gels that contained different ingredients were vaginally administered to Cynomolgus Monkeys, 0.5 ml once a day, for five consecutive days. Vaginal swabs were collected for the test of pH value, and for smear staining and microscopic examination to observe the effects of the gels on the pH values of vaginal secretions and on the vaginal flora of Cynomolgus Monkeys. The experimental results are shown in Table 32:

**Table 32 Effects of Bacteriostatic Gels Containing Different Ingredients on Vaginal Acidity and Vaginal Flora of Cynomolgus Monkeys**

| Serial number | Animal No. | Pre-administration | | Single administration | | Three | | Five administrations | |
|---|---|---|---|---|---|---|---|---|---|
| | | pH | Nugent score⁴ | pH | Nugent score | pH | Nugent score | pH | Nugent score |
| 1¹ | 151674 | 5.4 | >7 | 5.4 | 4-6 | 4.1 | 4-6 | 3.8 | 0-3 |
| 2² | 175456c | 5.4 | >7 | 4.1 | 4-6 | 3.8 | 4-6 | 3.8 | 0-3 |
| | 1612144c | 5.4 | >7 | 4.1 | 4-6 | 4.1 | 4-6 | 4.1 | 0-3 |
| 3³ | 1502004 | 5.4 | >7 | 5.4 | >7 | 5.4 | >7 | 5.4 | 0-3 |
| | 1503264 | 5.4 | >7 | 3.8 | 4-6 | 5.4 | 4-6 | 5.4 | 4-6 |

| | | | | | | | | | |
|---|---|---|---|---|---|---|---|---|---|
| Note: 1: The gel of Group 1 comprised 2.00% (w/w) isomaltulose, 0.12% (w/w) sodium benzoate, 0.38% (w/w) propionic acid, 1.31% (w/w) adipic acid, 0.04% (w/w) heptanoic acid, 0.30% (w/w) phenethyl alcohol, 0.35% (w/w) malic acid, and 2.15% (w/w) xanthan gum, with pH value adjusted to 3.9 Note 2: The gel of Group 2 comprised 2.00% (w/w) isomaltulose, 0.12% (w/w) sodium benzoate, 0.38% (w/w) propionic acid, 1.31% (w/w) adipic acid, 0.10% (w/w) hexanoic acid, 0.30% (w/w) phenethyl alcohol, 0.35% (w/w) malic acid, and 2.15% (w/w) xanthan gum, with pH value adjusted to 3.9 Note 3: The gel of Group 3 comprised 2.00% (w/w) isomaltulose, 0.12% (w/w) sodium benzoate, 0.38% (w/w) propionic acid, 1.31% (w/w) adipic acid, 0.20% (w/w) valeric acid, 0.30% (w/w) phenethyl alcohol, 0.35% (w/w) malic acid, and 2.15% (w/w) xanthan gum, with pH value adjusted to 3.9 Note 4: Same as Note 4 in in vivo experiment I | | | | | | | | | |

Results:
1. It was evident in the result of Group 1 in Table 32 that, after five administrations of the gel, which had a pH value of 3.9 and contained "2.00% (w/w) isomaltulose, 0.12% (w/w) sodium benzoate, 0.38% (w/w) propionic acid, 1.31% (w/w) adipic acid, 0.30% (w/w) phenethyl alcohol, 0.35% (w/w) malic acid, 0.04% (w/w) heptanoic acid, and 2.15% (w/w) xanthan gum", the pH values of vaginal secretion decreased to 3.8 and the Nugent score of the vaginal flora decreased to 0-3 in Cynomolgus Monkeys.
2. It was evident in the results of Group 2 in Table 32 that, after five administrations of the gel, which had a pH value of 3.9 and contained "2.00% (w/w) isomaltulose, 0.12% (w/w) sodium benzoate, 0.38% (w/w) propionic acid, 1.31% (w/w) adipic acid, 0.30% (w/w) phenethyl alcohol, 0.35% (w/w) malic acid, 0.10% (w/w) hexanoic acid, and 2.15% (w/w) xanthan gum", the pH values of vaginal secretions decreased to 4.1 or below, and the Nugent scores of the vaginal flora decreased to 0-3 in the two Cynomolgus Monkeys.
3. It was evident in the results of Group 3 in Table 32 that, after five administrations of the gel, which had a pH value of 3.9 and contained "2.00% (w/w) isomaltulose, 0.12% (w/w) sodium benzoate, 0.38% (w/w) propionic acid, 1.31% (w/w) adipic acid, 0.30% (w/w) phenethyl alcohol, 0.35% (w/w) malic acid, 0.20% (w/w) valeric acid, and 2.15% (w/w) xanthan gum", the pH values of vaginal secretions remained 5.4 in the two Cynomolgus Monkeys, and the Nugent scores of the vaginal flora decreased to 0-3 in one Cynomolgus Monkey and to 4-6 in another Cynomolgus Monkey.

In summary, it could be seen that the three groups of gels, which had a pH value of 3.9 and contained "2.00% (w/w) isomaltulose, 0.12% (w/w) sodium benzoate, 0.38% (w/w) propionic acid, 1.31% (w/w) adipic acid, 0.30% (w/w) phenethyl alcohol, 0.35% (w/w) malic acid, and 2.15% (w/w) xanthan gum ", as well as 0.04% (w/w) heptanoic acid, 0.10% (w/w) hexanoic acid, or 0.20% (w/w) valeric acid respectively had the efficacy of inhibiting abnormal vaginal flora and restoring vaginal lactobacilli. The two groups of gels containing 0.04% (w/w) heptanoic acid or 0.10% (w/w) hexanoic acid respectively also had the efficacy of restoring vaginal acidity.

### In vivo experiment V

Six groups of gels containing different ingredients were vaginally administered to Cynomolgus Monkeys, 0.5 mL once a day, for five consecutive days. The vaginal swabs were collected for the test of pH values and for smear staining and microscopic examination to observe the effects of the gels on the pH values of vaginal secretions and on the vaginal flora of Cynomolgus Monkeys. The experimental results are shown in Table 33:

**Table 33 Effects of Bacteriostatic Gels Containing Different Ingredients on Vaginal Acidity and Vaginal Flora of Cynomolgus Monkeys**

| Serial number | Animal No. | Pre-administration | | Single administration | | Three administrations | | Five administrations | |
|---|---|---|---|---|---|---|---|---|---|
| | | pH | Nugent score⁷ | pH | Nugent score | pH | Nugent score | pH | Nugent score |
| 1 | 1356026 | 5.4 | >7 | 5.4 | 4-6 | 5.4 | 4-6 | 5.4 | 0-3 |
| | 1524590 | 5.4 | >7 | 5.4 | >7 | 5.4 | >7 | 3.8 | 4-6 |
| | 1517032 | 5.4 | >7 | 5.4 | >7 | 5.4 | >7 | Menstruation | / |
| 2² | 1235012 | 5.4 | >7 | 5.4 | 4-6 | 5.4 | 4-6 | 5.4 | 4-6 |
| | 1430426 | 5.4 | >7 | 4.4 | 0-3 | 4.1 | 0-3 | 4.1 | 0-3 |
| 3³ | 1093752 | 5.4 | >7 | 3.8 | 0-3 | 3.8 | 0-3 | 3.8 | 0-3 |
| | 1490523 | 5.4 | >7 | 5.4 | >7 | 4.6 | 4-6 | 4.1 | 0-3 |
| 4⁴ | 1205990 | 5.4 | >7 | 5.4 | >7 | 4.6 | 4-6 | 4.6 | 4-6 |
| | 1434210 | 5.4 | >7 | 4.1 | 0-3 | 3.8 | 0-3 | 3.8 | 0-3 |
| 5⁵ | 1539203 | 5.4 | >7 | 5.4 | >7 | 5.4 | 4-6 | 4.6 | 4-6 |
| | 1434420 | 5.4 | >7 | 5.4 | >7 | 3.8 | 0-3 | 3.8 | 0-3 |
| 6⁶ | 1029995 | 5.4 | >7 | 4.6 | >7 | 3.8 | 0-3 | 3.8 | 0-3 |
| | 1488275 | 5.4 | >7 | 5.4 | >7 | 5.4 | >7 | 4.1 | 4-6 |

| | | | | | | | | | |
|---|---|---|---|---|---|---|---|---|---|
| Note: 1: The gel of Group 1 comprised 0.10% (w/w) hexanoic acid, 1.50% (w/w) adipic acid, 2.00% (w/w) maltose, and 2.15% (w/w) xanthan gum, with pH value adjusted to 3.1 2: The gel of Group 2 comprised 2.00% (w/w) propionic acid, 0.30% (w/w) cinnamyl alcohol, 0.60% (w/w) succinic acid, 2.00% (w/w) maltose, and 2.15% (w/w) xanthan gum, with pH value adjusted to 3.8 3: The gel of Group 3 comprised 0.02% (w/w) caprylic acid, 0.25% (w/w) benzoic acid, 1.00% (w/w) citric acid, 2.00% (w/w) maltose, and 2.15% (w/w) xanthan gum, with pH value adjusted to 3.8 4: The gel of Group 4 comprised 0.20% (w/w) benzoic acid, 0.25% (w/w) benzyl alcohol, 0.001% (w/w) undecylic acid, 1.00% (w/w) malic acid, 2.00% (w/w) maltose, and 2.15% (w/w) xanthan gum, with pH value adjusted to 3.80 5: The gel of Group 5 comprised 0.20% (w/w) benzoic acid, 0.50% (w/w) propionic acid, 1.50% (w/w) adipic acid, 2.00% (w/w) maltose, and 2.15% (w/w) xanthan gum, with pH value adjusted to 3.8 6: The gel of Group 6 comprised 1.00% (w/w) pimelic acid, 0.60% (w/w) phenethyl alcohol, 1.25% (w/w) butyric acid, 2.00% (w/w) maltose, and 2.15% (w/w) xanthan gum, with pH value adjusted to 3.8 7: Same as Note 4 in in vivo experiment I | | | | | | | | | |

Results:
1. It was evident in the results of Group 1 in Table 33 that, after five administrations of the gel, which had a pH value of 3.1 and contained "0.10% (w/w) hexanoic acid, 1.50% (w/w) adipic acid, 2.00% (w/w) maltose, and 2.15% (w/w) xanthan gum", the experiment was terminated in one of the three Cynomolgus Monkeys due to menstruation; the pH values of vaginal secretions remained 5.4 in one Cynomolgus Monkey, and decreased to 3.8 in another one; The Nugent scores of the vaginal flora decreased to 4-6 in one Cynomolgus Monkey and decreased to 0-3 in another one;
2. It was evident in the results of Group 2 in Table 33 that, after five administrations of the gels, which had a pH value of 3.8 and contained "2.00% (w/w) propionic acid, 0.30% (w/w) cinnamyl alcohol, 0.60% (w/w) succinic acid, 2.00% (w/w) maltose, and 2.15% (w/w) xanthan gum", the pH values of vaginal secretions decreased to 4.1 in one of the two Cynomolgus Monkeys and remained 5.4 in another one; The Nugent scores of the vaginal flora decreased to 4-6 in one Cynomolgus Monkey and to 0-3 in another one.
3. It was evident in the results of Group 3 in Table 33 that, after five administrations of the gel, which had a pH value of 3.8 and contained "0.02% (w/w) caprylic acid, 0.25% (w/w) benzoic acid, 1.00% (w/w) citric acid, 2.00% (w/w) maltose, and 2.15% (w/w) xanthan gum", the pH values of vaginal secretions decreased to 3.8 in one of the two Cynomolgus Monkeys and to 4.1 in another, and the Nugent scores of the vaginal flora decreased to 0-3 in the two Cynomolgus Monkeys;
4. It was evident in the results of Group 4 in Table 33 that, after five administrations of the gel, which had a pH value of 3.8 and contained"0.20% (w/w) benzoic acid, 0.25% (w/w) benzyl alcohol, 0.001% (w/w) undecylic acid, 1.00% (w/w) malic acid, 2.00% (w/w) maltose, and 2.15% (w/w) xanthan gum", the pH values of vaginal secretions decreased to 4.6 in one of the two Cynomolgus Monkeys and to 3.8 in another, and the Nugent score of the vaginal flora decreased to 4-6 in one Cynomolgus Monkey and to 0-3 in another;
5. It was evident in the results of Group 5 in Table 33 that, after five administrations of the gel, which had a pH value of 3.8 and contained "0.20% (w/w) benzoic acid, 0.50% (w/w) propionic acid, 1.50% (w/w) adipic acid, 2.00% (w/w) maltose, and 2.15% (w/w) xanthan gum", the pH value of vaginal secretions decreased to 4.6 in one of the two Cynomolgus Monkeys and to 3.8 in another, and the Nugent score of vaginal flora decreased to 4-6 in one Cynomolgus Monkey and to 0-3 in another;
6. It was evident in the results of Group 6 in Table 33 that, after five administrations of the gel, which had a pH value of 3.8 and contained "1.00% (w/w) pimelic acid, 0.60% (w/w) phenethyl alcohol, 1.25% (w/w) butyric acid, 2.00% (w/w) maltose, and 2.15% (w/w) xanthan gum", the pH value of vaginal secretions decreased to 3.8 in one of the two Cynomolgus Monkeys and to 4.1 in another, and the Nugent score of the vaginal flora decreased to 0-3 in one Cynomolgus Monkey and to 4-6 in another.

In summary, it could be seen that these 6 gel compositions had the effect of inhibiting abnormal vaginal flora, restoring vaginal acidity, and restoring vaginal lactobacilli.

### In vivo experiment VI

The gels containing adipic acid or fumaric acid were vaginally administered to Cynomolgus Monkeys, 0.5 mL once a day, for five consecutive days, and vaginal swabs were collected for the test of pH value and for smear staining and microscopic examination to observe the effects of the gels on the pH values of vaginal secretions and on the vaginal flora of Cynomolgus Monkeys. The experimental results are shown in Table 34:

**Table 34 Effects of Gels Containing Adipic Acid and Fumaric Acid on Vaginal Acidity and Vaginal Flora of Cynomolgus Monkeys**

| Serial number | Animal No. | Pre-administration | | Single administration | | Three administrations | | Five administrations | |
|---|---|---|---|---|---|---|---|---|---|
| | | pH | Nugent score³ | pH | Nugent score | pH | Nugent score | pH | Nugent score |
| 1¹ | 1038246 | 5.4 | >7 | 5.4 | >7 | 5.4 | >7 | 5.4 | >7 |
| | 1421211 | 5.4 | >7 | 5.4 | >7 | 5.4 | >7 | 5.4 | 4-6 |
| 2² | 1421363 | 5.4 | >7 | 4.6 | 4-6 | 4.4 | 0-3 | 4.1 | 0-3 |
| | 1102845 | 5.4 | >7 | 4.4 | 0-3 | 4.1 | 0-3 | 4.1 | 0-3 |

| | | | | | | | | | |
|---|---|---|---|---|---|---|---|---|---|
| Note: 1: The gel of Group 1 comprised 1.50% (w/w) fumaric acid, 1.00% (w/w) propionic acid, 0.08% (w/w) cinnamic acid, 0.25% (w/w) phenethyl alcohol, 1.40% (w/w) maltose, and 2.15 % (w/w) xanthan gum, with pH value adjusted to 3.8 2: The gel of Group 2 comprised 1.50% (w/w) adipic acid, 1.00% (w/w) propionic acid, 0.08% (w/w) cinnamic acid, 0.25% (w/w) phenethyl alcohol, 1.40% (w/w) maltose, and 2.15% (w/w) xanthan gum, with pH value adjusted to 3.8 3: Same as Note 4 in in vivo experiment I | | | | | | | | | |

Results:
1. It was evident in the results of Group 1 in Table 34 that, after five administrations of the gel, which had a pH value of 3.8 and contained "1.50% (w/w) fumaric acid, 1.00% (w/w) propionic acid, 0.08% (w/w) cinnamic acid, 0.25% (w/w) phenethyl alcohol, 1.40% (w/w) maltose, and 2.15% (w/w) xanthan gum", the pH values of vaginal secretions remained 5.4 in two Cynomolgus Monkeys, and the Nugent score of the vaginal flora remained >7 in one Cynomolgus Monkey and decreased to 4-6 in another.
2. It was evident from the results of Group 2 in Table 34 that, after five administrations of the gel, which had a pH value of 3.8 and contained "1.50% (w/w) adipic acid, 1.00% (w/w) propionic acid, 0.08% (w/w) cinnamic acid, 0.25% (w/w) phenethyl alcohol, 1,40% (w/w) maltose, and 2.15% (w/w) xanthan gum", the pH values of vaginal secretions decreased to 4.1 and the Nugent scores of the vaginal flora decreased to 0-3 in both of the two Cynomolgus Monkeys.

In summary, it could be seen that the gel containing 1.50% (w/w) adipic acid had the effect of inhibiting abnormal vaginal flora, restoring vaginal lactobacilli, and restoring vaginal acidity.

### Clinical observation I

The gel A was vaginally administered in 10 patients with bacterial vaginosis (BV) and 5 healthy volunteers, 4.5 g once a day, for 5 consecutive days, and vaginal swabs were collected on the third day of administration (V1) and 3 days after drug discontinuance (V2) for the test of pH value and for smear staining and microscopic examination to observe the effects of the gel on the pH values of vaginal secretions and on the vaginal flora. The experimental results are shown in Table 35:

**Table 35 Effects of Gel A on Vaginal Acidity and Vaginal Flora of Patients with BV and Healthy Volunteers**

| Subject | | | Pre-administration | | V1 | | V2 | |
|---|---|---|---|---|---|---|---|---|
| | | | pH² | Nugent score³ | pH | Nugent score | pH | Nugent score |
| Gel A¹ | Patients with BV | 1 | 4.6-5.4 | 8 | 3.8-4.1 | 8 | 3.8-4.1 | 1 |
| | | 2 | 4.6-5.4 | 8 | 3.8-4.1 | 5 | 3.8-4.1 | 2 |
| | | 3 | 4.6-5.4 | 7 | 3.8-4.1 | 2 | 3.8-4.1 | 0 |
| | | 4 | 4.6-5.4 | 8 | 4.6-5.4 | 8 | 4.6-5.4 | 6 |
| | | 5 | 4.6-5.4 | 8 | 4.6-5.4 | 7 | 3.8-4.1 | 1 |
| | | 6 | 4.6-5.4 | 8 | 3.8-4.1 | 5 | 3.8-4.1 | 2 |
| | | 7 | 4.6-5.4 | 8 | 3.8-4.1 | 4 | 3.8-4.1 | 1 |
| | | 8 | 4.6-5.4 | 8 | 4.6-5.4 | 6 | 3.8-4.1 | 2 |
| | | 9 | 4.6-5.4 | 8 | 3.8-4.1 | 3 | 3.8-4.1 | 1(VVC) |
| | | 10 | 4.6-5.4 | 7 | 3.8-4.1 | 0 | 3.8-4.1 | 0 |
| | Healthy volunteers | 1 | 3.8-4.1 | 0 | 3.8-4.1 | 1 | 3.8-4.1 | 1 |
| | | 2 | 3.8-4.1 | 1 | 3.8-4.1 | 2 | 3.8-4.1 | 1 |
| | | 3 | 3.8-4.1 | 1 | 3.8-4.1 | 0 | 3.8-4.1 | 0 |
| | | 4 | 3.8-4.1 | 2 | 3.8-4.1 | 0 | 3.8-4.1 | 1 |
| | | 5 | 3.8-4.1 | 1 | 3.8-4.1 | 1 | 3.8-4.1 | 1 |

| | | | | | | | | |
|---|---|---|---|---|---|---|---|---|
| Note 1: Gel A comprised 1.46% (w/w) adipic acid, 0.70% (w/w) sodium propionate, 0.14% (w/w) sodium benzoate, 0.32% (w/w) phenethyl alcohol, 1.00% (w/w) citric acid, 1.60% (w/w) maltose, 0.20% (w/w) palatinose, 0.0005% (w/w) rose essential oil, and 2.15% (w/w) xanthan gum, with pH value adjusted to 3.70 Note 2: pH value was measured using a pH range standard colorimetric card. The colorimetric card has two ranges: 3.8-4.1 indicates normal; 4.6-5.4 indicates abnormal Note 3: Same as Note 7 in in vivo experiment I | | | | | | | | |

Results:
1. It was evident in the results of BV patients in Table 35 that, after three administrations of the gel, which had a pH value of 3.70 and contained "1.46% (w/w) adipic acid, 0.70% (w/w) sodium propionate, 0.14% (w/w) sodium benzoate, 0.32% (w/w) phenethyl alcohol, 1.00% (w/w) citric acid, 1.60% (w/w) maltose, 0.20% (w/w) palatinose, 0.0005% (w/w) rose essential oil, and 2.15% (w/w) xanthan gum", the pH values decreased to 3.8-4.1 in seven of the ten BV patients, and the Nugent scores of the vaginal flora decreased to 3 or below in three BV patients and to 4-6 in four BV patients.
   After five administrations and an interval of 3 days after drug discontinuance, the pH values decreased to 3.8-4.1 in nine of the ten BV patients, and the Nugent scores of the vaginal flora decreased to lower than 3. In one patient, the pH value remained 4.6-5.4, and the Nugent score decreased to 6.
2. It was evident in the results of healthy volunteers in Table 35 that, three administrations of the gel, which had a pH value of 3.70 and contained "1.46% (w/w) adipic acid, 0.70% (w/w) sodium propionate, 0.14% (w/w) sodium benzoate, 0.32% (w/w) phenethyl alcohol, 1.00% (w/w) citric acid, 1.60% (w/w) maltose, 0.20% (w/w) palatinose, 0.0005% (w/w) rose essential oil, and 2.15% (w/w) xanthan gum", did not affect the pH values of vaginal secretions or the vaginal flora in healthy volunteers.

After five administrations and an interval of 3 days after drug discontinuance, the results showed that the administrations did not affect the pH value of vaginal secretions or the vaginal flora in healthy volunteers.

In summary, it could be seen in the experiment that, the gel, which had a pH value of 3.70 and contained"1.46% (w/w) adipic acid, 0.70% (w/w) sodium propionate, 0.14% (w/w) sodium benzoate, 0.32% (w/w) phenethyl alcohol, 1.00% (w/w) citric acid, 1.60% (w/w) maltose, 0.20% (w/w) palatinose, 0.0005% (w/w) rose essential oil, and 2.15% (w/w) xanthan gum", could decrease significantly the abnormal vaginal flora, increase significantly vaginal lactobacilli, and lowered the vaginal pH value in BV patients, but had no effect on the normal pH values of vaginal secretions or normal flora in healthy volunteers, indicating that the gel had the effect of inhibiting abnormal vaginal flora, restoring and/or maintaining vaginal lactobacilli, and restoring and/or maintaining normal vaginal acidity.

### References:

1. Fang Liang: Pharmaceutics, People's Medical Publishing House, eighth edition (2016), pages 243-252;
2. Fang Liang: Pharmaceutics, People's Medical Publishing House, eighth edition (2016), pages 211-228;
3. Fang Liang: Pharmaceutics, People's Medical Publishing House, eighth edition (2016), pages 234-237;
4. Fang Liang: Pharmaceutics, People's Medical Publishing House, eighth edition (2016), pages 280-284.

## Claims

1. A bacteriostatic composition, **characterized in that** it comprises:
(1) one or more of the fatty acids and/or salts thereof selected from the group consisting of acetic acid, propionic acid, butyric acid, isobutyric acid, valeric acid, hexanoic acid, heptanoic acid, caprylic acid, nonanoic acid, capric acid, undecylic acid, undecylenic acid, lauric acid, and salts thereof; the total content of the ingredients (1) as described, calculated as fatty acid, is in the range of 0.001-3.00% (w/w);
(2) one or more of the dicarboxylic acids and/or salts thereof selected from the group consisting of glutaric acid, adipic acid, pimelic acid, and salts thereof; the total content of the ingredients (2) as described, calculated as dicarboxylic acid, is in the range of 0.05-5.00% (w/w);
(3) one or more of the aromatic alcohols selected from the group consisting of benzyl alcohol, phenethyl alcohol, phenoxyethanol, and cinnamyl alcohol; the total content of the ingredients (3) as described is in the range of 0.03-1.00% (w/w);
(4) one or more of the aromatic carboxylic acids and/or salts thereof selected from the group consisting of benzoic acid, p-hydroxybenzoic acid, p-methoxybenzoic acid, salicylic acid, cinnamic acid, gentianic acid, caffeic acid, and salts thereof; the total content of the ingredients (4) as described, calculated as aromatic carboxylic acid, is in the range of 0.03-1.00% (w/w);
the bacteriostatic composition is in a dosage form selected from the group consisting of aqueous solutions, water-soluble gels, foams, sprays, ointments, powders, films, capsules, suppositories, and tablets.

2. The bacteriostatic composition according to Claim 1, **characterized in that** the total content of the ingredients (1) in the bacteriostatic composition as described is in the range of 0.001-2.00% (w/w); and/or
the total content of the ingredients (2) as described is in the range of 0.10-3.50% (w/w); and/or
the total content of the ingredients (3) as described is in the range of 0.03-0.70% (w/w); and/or
the total content of the ingredients (4) as described is in the range of 0.03-0.50% (w/w).

3. The bacteriostatic composition according to Claim 1, **characterized in that** the bacteriostatic composition as described further comprises one or more of the antibacterial drugs selected from the group consisting of metronidazole, tinidazole, ornidazole, gentamicin, tobramycin, amikacin, sisomicin, netilmicin, ciprofloxacin, ofloxacin, levofloxacin, nifuratel, nifuroxime, furacilin, furazolidone, furantoin, silver sulfadiazine, sodium sulfacetamide, clotrimazole, fluconazole, miconazole, ketoconazole, naftifine, terbinafine, amphotericin B, nystatin, levorin, and natamycin.

4. The bacteriostatic composition according to Claim 1, **characterized in that** the bacteriostatic composition as described is in a dosage form selected from the group consisting of aqueous solutions, water-soluble gels, foams, sprays, or ointments; the pH value of which is in the range of 3.1-4.8, or 3.6-4.6, or 3.8-4.4.

5. The bacteriostatic composition according to Claim 1, **characterized in that** the bacteriostatic composition as described can be a therapeutic product, an active ingredient thereof, and an antiseptic thereof; the therapeutic product form is one of the following group: drugs, disinfectants, antibacterial agents, bacteriostatic agents, topical microbicides, flora modulators, microecological modulators, microenvironment modulators, microbial modulators, disposable medical supplies, and the like, or components of medical devices, components of pharmaceutical devices, components of disinfection devices, and components of devices for vagina use; and/or the bacteriostatic composition as described can be a non-therapeutic product, an active ingredient thereof, and an antiseptic thereof; the non-therapeutic product form is one of the following group: health care products, hygiene products, personal cleaning and care products, cosmetics, disposable hygiene products, cleaning products, daily necessities, microecological care products, deodorants, lubricants, humectants, lotions, cleaning agents, body care products, antipruritic agents, and refreshing agents, or the components of sanitary napkins, sanitary pads, and tampons.

6. The bacteriostatic composition according to Claim 1, **characterized in that** the bacteriostatic composition as described is a vaginal bacteriostatic composition, wherein the vaginal bacteriostatic composition comprises the following ingredients:
(1) one or more of the fatty acids and/or salts thereof selected from the group consisting of acetic acid, propionic acid, butyric acid, valeric acid, hexanoic acid, heptanoic acid, caprylic acid, nonanoic acid, capric acid, undecylic acid, undecylenic acid, lauric acid, and salts thereof; the total content of the ingredients (1), calculated as fatty acid, is in the range of 0.001-2.00% (w/w);
(2) one or more of the dicarboxylic acids and/or salts thereof selected from the group consisting of glutaric acid, adipic acid, pimelic acid, and salts thereof; the total content of the ingredients (2), calculated as dicarboxylic acid, is in the range of 0.50-2.50% (w/w);
(3) one or more of the aromatic alcohols selected from the group consisting of benzyl alcohol, phenethyl alcohol, phenoxyethanol, and cinnamyl alcohol; the total content of the ingredients (3) is in the range of 0.05-0.60% (w/w);
(4) one or more of the aromatic carboxylic acids and/or salts thereof selected from the group consisting of benzoic acid, p-hydroxybenzoic acid, p-methoxybenzoic acid, salicylic acid, cinnamic acid, gentianic acid, caffeic acid, and salts thereof; the total content of the ingredients (4), calculated as aromatic carboxylic acid, is in the range of 0.05-0.25% (w/w); the vaginal bacteriostatic composition is in a dosage form selected from the group consisting of aqueous solutions, water-soluble gels, foams, sprays, ointments, powders, films, capsules, suppositories, and tablets.

7. A method for inhibiting harmful microorganisms outside the body, **characterized in that** it includes the steps of using a bacteriostatic composition as described in Claims 1-5.

8. The method according to Claim 7, **characterized in that** the harmful microorganisms as described refer to one or more of *Candida, Staphylococcus aureus, Escherichia coli, Pseudomonas aeruginosa, Gardnerella, Prevotella, Mobiluncus,* and *Aspergillus niger.*

9. A vaginal bacteriostatic composition for use in the treatment and/or prevention of vaginal infections by modulation of vaginal flora, **characterized in that** the vaginal bacteriostatic composition comprises the following ingredients:
(1) one or more of the fatty acids and/or salts thereof selected from the group consisting of propionic acid, butyric acid, valeric acid, hexanoic acid, heptanoic acid, caprylic acid, nonanoic acid, capric acid, undecylic acid, undecylenic acid, lauric acid, and salts thereof; the total content of which, calculated as fatty acid, is in the range of 0.001-2.00% (w/w); and
(2) one or more of the dicarboxylic acids and/or salts thereof selected from the group consisting of glutaric acid, adipic acid, pimelic acid, and salts thereof; the total content of which, calculated as dicarboxylic acid, is in the range of 0.50-2.50% (w/w);
wherein the vaginal bacteriostatic composition is in a dosage form selected from the group consisting of aqueous solutions, water-soluble gels, foams, sprays, ointments, powders, films, capsules, suppositories, and tablets;
wherein the modulation of vaginal flora refers to at least one of the following: inhibiting abnormal vaginal flora, restoring and/or maintaining vaginal lactobacilli.

10. The vaginal bacteriostatic composition according to Claim 9, **characterized in that** the vaginal bacteriostatic composition is used for inhibiting abnormal vaginal flora, wherein the inhibition of abnormal vaginal flora refers to inhibiting one or more of *Candida, Staphylococcus, Streptococcus, Gardnerella, Escherichia coli, Veillonella parvula, Prevotella,* and *Mobiluncus.*

11. The vaginal bacteriostatic composition according to Claim 9, **characterized in that** the vaginal bacteriostatic composition further comprises one or more of the aromatic carboxylic acids and/or salts thereof selected from the group consisting of benzoic acid, p-hydroxybenzoic acid, p-methoxybenzoic acid, salicylic acid, cinnamic acid, gentianic acid, caffeic acid, and salts thereof; the total content of which, calculated as aromatic carboxylic acid, is in the range of 0.05-0.25% (w/w).

12. The vaginal bacteriostatic composition according to Claim 9, **characterized in that** the vaginal bacteriostatic composition further comprises one or more of the estrogens or phytoestrogens selected from the group consisting of diethylstilbestrol, hexoestrol, estradiol, estrone, estriol, nilestriol, ethinyloestradiol, quinestrol, mestranol, promestriene, daidzin, daidzein, glycitein, puerarin, coumestrol, genistein, equol, apigenin, genistin, genisteol, biochanin, coumestrol, formononetin, resveratrol, secoisolariciresinol, and lignan; the total content of which is in the range of 0.001-1.00% (w/w).

13. The vaginal bacteriostatic composition according to Claim 9, **characterized in that** the vaginal bacteriostatic composition further comprises one or more of the saccharides selected from the group consisting of glucose, fructose, mannose, galactose, maltose, isomaltose, sucrose, isomaltulose, lactose, lactulose, trehalose, cellobiose, melibiose, gentiobiose, 1-kestose, nystose, 1F-fructofuranosylnystose, isomaltotriose, isomaltotetraose, isomaltopentaose, gentiooligosaccharide, raffinose, panose, maltooligosaccharide, palatinose-oligosaccharide, oligofructose, glucomannan, galactooligosaccharide, dextrin, starch, and glycogen; the total content of which is in the range of 0.01-20.00% (w/w) or 0.10-2.00% (w/w).

14. The vaginal bacteriostatic composition according to Claim 9, **characterized in that** the vaginal bacteriostatic composition is used to restore and/or maintain normal vaginal flora, and/or to restore and/or maintain normal vaginal microecology, and/or to restore and/or maintain normal vaginal acidity, and/or to clean and take care of the vagina and/or vulva, and/or to reduce and/or eliminate vaginal pruritus, soreness, dryness, irritation, and dyspareunia, and/or to reduce and/or eliminate abnormal vaginal discharge, and unpleasant odor of vaginal discharge.

15. The vaginal bacteriostatic composition according to Claim 9, **characterized in that** the vaginal bacteriostatic composition as described is used to prevent and/or treat the imbalance of vaginal flora, bacterial vaginosis, aerobic vaginitis, cytolytic vaginosis, vulvovaginal Candidiasis, and/or atrophic vaginitis.

## Patentansprüche

1. Bakteriostatische Zusammensetzung, **dadurch gekennzeichnet, dass** sie Folgendes umfasst:
(1) eine oder mehrere der Fettsäuren und/oder deren Salze, ausgewählt aus der Gruppe bestehend aus Essigsäure, Propionsäure, Buttersäure, Isobuttersäure, Valeriansäure, Hexansäure, Heptansäure, Caprylsäure, Nonansäure, Caprinsäure, Undecylsäure, Undecylensäure, Laurinsäure und deren Salze; der Gesamtgehalt der Inhaltsstoffe (1) wie beschrieben, berechnet als Fettsäure, liegt im Bereich von 0,001-3,00 % (w/w);
(2) eine oder mehrere der Dicarboxylsäuren und/oder deren Salze, ausgewählt aus der Gruppe bestehend aus Glutarsäure, Adipinsäure, Pimelicsäure und deren Salze; der Gesamtgehalt der Inhaltsstoffe (2) wie beschrieben, berechnet als Dicarboxylsäure, liegt im Bereich von 0,05-5,00 % (w/w);
(3) einen oder mehrere der aromatischen Alkohole, ausgewählt aus der Gruppe bestehend aus Benzylalkohol, Phenethylalkohol, Phenoxyethanol und Zimtalkohol; der Gesamtgehalt der Inhaltsstoffe (3) wie beschrieben liegt im Bereich von 0,03-1,00 % (w/w);
(4) eine oder mehrere der aromatischen Karboxylsäuren und/oder deren Salze, ausgewählt aus der Gruppe bestehend aus Benzoesäure, p-Hydroxybenzoesäure, p-Methoxybenzoesäure, Salicylsäure, Zimtsäure, Gentiansäure, Koffeinsäure und deren Salze; der Gesamtgehalt der Inhaltsstoffe (4) wie beschrieben, berechnet als aromatische Karboxylsäure, liegt im Bereich von 0,03-1,00 % (w/w);
die bakteriostatische Zusammensetzung in einer Dosierungsform vorliegt, die aus der Gruppe bestehend aus wässrigen Lösungen, wasserlöslichen Gelen, Schäumen, Sprays, Salben, Pulvern, Filmen, Kapseln, Zäpfchen und Tabletten ausgewählt ist.

2. Die bakteriostatische Zusammensetzung nach Anspruch 1, **dadurch gekennzeichnet, dass** der Gesamtgehalt der Inhaltsstoffe (1) in der bakteriostatischen Zusammensetzung wie beschrieben im Bereich von 0,001-2,00 % (w/w) liegt; und/oder
der Gesamtgehalt der Inhaltsstoffe (2) wie beschrieben im Bereich von 0,10-3,50 % (w/w) liegt; und/oder
der Gesamtgehalt der Inhaltsstoffe (3) wie beschrieben im Bereich von 0,03-0,70 % (w/w) liegt; und/oder
der Gesamtgehalt der Inhaltsstoffe (4) wie beschrieben im Bereich von 0,03-0,50 % (w/w) liegt.

3. Bakteriostatische Zusammensetzung nach Anspruch 1, **dadurch gekennzeichnet, dass** die bakteriostatische Zusammensetzung wie beschrieben, ferner einen oder mehrere antibakterielle Wirkstoffe umfasst, ausgewählt aus der Gruppe bestehend aus Metronidazol, Tinidazol, Ornidazol, Gentamicin, Tobramycin, Amikacin, Sisomicin, Netilmicin, Ciprofloxacin, Ofloxacin, Levofloxacin, Nifuratel, Nifuroxim, Furacilin, Furazolidon, Furantoin, Silbersulfadiazin, Natriumsulfacetamid, Clotrimazol, Fluconazol, Miconazol, Ketoconazol, Naftifin, Terbinafin, Amphotericin B, Nystatin, Levorin und Natamycin.

4. Bakteriostatische Zusammensetzung nach Anspruch 1, **dadurch gekennzeichnet, dass** die bakteriostatische Zusammensetzung wie beschrieben in einer Dosierungsform vorliegt, die aus der Gruppe ausgewählt ist, bestehend aus wässrigen Lösungen, wasserlöslichen Gelen, Schäumen, Sprays oder Salben, deren pH-Wert im Bereich von 3,1-4,8, oder 3,6-4,6, oder 3,8-4,4 liegt.

5. Bakteriostatische Zusammensetzung nach Anspruch 1, **dadurch gekennzeichnet, dass** die bakteriostatische Zusammensetzung wie beschrieben ein therapeutisches Produkt, ein Wirkstoff davon, und ein Antiseptikum davon sein kann; die therapeutische Produktform ist eine der folgenden Gruppe: Medikamente, Desinfektionsmittel, antibakterielle Mittel, bakteriostatische Mittel, topische Mikrobizide, Floramodulatoren, mikroökologische Modulatoren, Mikroumgebungsmodulatoren, mikrobielle Modulatoren, medizinische Einwegartikel, und dergleichen, oder Komponenten von Medizingeräten, Bestandteile von pharmazeutischen Geräten, Bestandteile von Desinfektionsgeräten, und Bestandteile von Geräten zur vaginalen Anwendung; und/oder die bakteriostatische Zusammensetzung wie beschrieben kann ein nichttherapeutisches Produkt, ein Wirkstoff davon und ein Antiseptikum davon sein; die nichttherapeutische Produktform ist eine der folgenden Gruppe: Gesundheitsprodukte, Hygieneprodukte, Körperpflegeprodukte, Kosmetika, Einweg-Hygieneprodukte, Reinigungsprodukte, Alltagsutensilien, mikroökologische Pflegeprodukte, Deodorants, Gleitmittel, Feuchthaltemittel, Lotionen, Reinigungsmittel, Körperpflegemittel, Anti-Juckreizmittel und Auffrischungsmittel, oder die Bestandteile von Sanitärtüchern, Binden und Tampons.

6. Bakteriostatische Zusammensetzung nach Anspruch 1, **dadurch gekennzeichnet, dass** die bakteriostatische Zusammensetzung wie beschrieben eine vaginale bakteriostatische Zusammensetzung ist, wobei die vaginale bakteriostatische Zusammensetzung folgende Inhaltsstoffe umfasst:
(1) eine oder mehrere der Fettsäuren und/oder deren Salze, ausgewählt aus der Gruppe bestehend aus Essigsäure, Propionsäure, Buttersäure, Valeriansäure, Hexansäure, Heptansäure, Caprylsäure, Nonansäure, Caprinsäure, Undecylsäure, Undecylensäure, Laurinsäure und deren Salze; der Gesamtgehalt der Bestandteile (1), berechnet als Fettsäure, liegt im Bereich von 0,001-2,00 % (w/w);
(2) eine oder mehrere der Dicarbonsäuren und/oder deren Salze, ausgewählt aus der Gruppe bestehend aus Glutarsäure, Adipinsäure, Pimelinsäure und deren Salze; der Gesamtgehalt der Bestandteile (2), berechnet als Dicarbonsäure, liegt im Bereich von 0,50-2,50 % (w/w);
(3) einen oder mehrere der aromatischen Alkohole, ausgewählt aus der Gruppe bestehend aus Benzylalkohol, Phenethylalkohol, Phenoxyethanol und Zimtalkohol; der Gesamtgehalt der Bestandteile (3) liegt im Bereich von 0,05-0,60 % (w/w);
(4) eine oder mehrere der aromatischen Carbonsäuren und/oder deren Salze, ausgewählt aus der Gruppe bestehend aus Benzoesäure, p-Hydroxybenzoesäure, p-Methoxybenzoesäure, Salicylsäure, Zimtsäure, Gentiansäure, Kaffeesäure und deren Salze; der Gesamtgehalt der Bestandteile (4), berechnet als aromatische Carbonsäure, liegt im Bereich von 0,05-0,25 % (w/w); die vaginale bakteriostatische Zusammensetzung liegt in einer Dosierungsform vor, ausgewählt aus der Gruppe bestehend aus wässrigen Lösungen, wasserlöslichen Gelen, Schäumen, Sprays, Salben, Pulvern, Filmen, Kapseln, Zäpfchen und Tabletten.

7. Verfahren zur Hemmung schädlicher Mikroorganismen außerhalb des Körpers, **dadurch gekennzeichnet, dass** es die Schritte der Verwendung einer bakteriostatischen Zusammensetzung gemäß den Ansprüchen 1-5 beinhaltet.

8. Verfahren nach Anspruch 7, **dadurch gekennzeichnet, dass** sich die schädlichen Mikroorganismen wie beschrieben auf einen oder mehrere von *Candida, Staphylococcus aureus, Escherichia coli, Pseudomonas aeruginosa, Gardnerella, Prevotella, Mobiluncus,* und *Aspergillus niger* beziehen.

9. Vaginale bakteriostatische Zusammensetzung zur Verwendung bei der Behandlung und/oder Prävention von vaginalen Infektionen durch Modulation der Vaginalflora, **dadurch gekennzeichnet, dass** die vaginale bakteriostatische Zusammensetzung folgende Inhaltsstoffe umfasst:
(1) eine oder mehrere der Fettsäuren und/oder deren Salze, ausgewählt aus der Gruppe, bestehend aus Propionsäure, Buttersäure, Valeriansäure, Hexansäure, Heptansäure, Caprylsäure, Nonansäure, Caprinsäure, Undecylsäure, Undecylensäure, Laurinsäure und deren Salze, deren Gesamtgehalt, berechnet als Fettsäure, im Bereich von 0,001-2,00 % (w/w) liegt; und
(2) eine oder mehrere der Dicarboxylsäuren und/oder deren Salze, ausgewählt aus der Gruppe bestehend aus Glutarsäure, Adipinsäure, Pimelinsäure und deren Salze; deren Gesamtgehalt, berechnet als Dicarboxylsäure, im Bereich von 0,50-2,50 % (w/w) liegt;
wobei die vaginale bakteriostatische Zusammensetzung in einer Dosierungsform vorliegt, die aus der Gruppe bestehend aus wässrigen Lösungen, wasserlöslichen Gelen, Schäumen, Sprays, Salben, Pulvern, Filmen, Kapseln, Zäpfchen und Tabletten ausgewählt ist;
wobei sich die Modulation der Vaginalflora auf mindestens eines der folgenden bezieht: Hemmung der abnormalen Vaginalflora, Wiederherstellung und/oder Erhaltung der vaginalen Laktobazillen.

10. Vaginale bakteriostatische Zusammensetzung nach Anspruch 9, **dadurch gekennzeichnet, dass** die vaginale bakteriostatische Zusammensetzung zur Hemmung einer abnormalen Vaginalflora verwendet wird, wobei sich die Hemmung einer abnormalen Vaginalflora auf die Hemmung eines oder mehrerer der *Candida, Staphylokokken, Streptokokken, Gardnerella, Escherichia coli, Veillonella parvula, Prevotella* und *Mobiluncus* bezieht.

11. Vaginale bakteriostatische Zusammensetzung nach Anspruch 9, **dadurch gekennzeichnet, dass** die vaginale bakteriostatische Zusammensetzung ferner eine oder mehrere der aromatischen Carbonsäuren und/oder deren Salze umfasst, ausgewählt aus der Gruppe bestehend aus Benzoesäure, p-Hydroxybenzoesäure, p-Methoxybenzoesäure, Salicylsäure, Zimtsäure, Enziansäure, Kaffeesäure und deren Salzen; deren Gesamtgehalt, berechnet als aromatische Carbonsäure, im Bereich von 0,05-0,25 % (w/w) liegt.

12. Vaginale bakteriostatische Zusammensetzung nach Anspruch 9, **dadurch gekennzeichnet, dass** die vaginale bakteriostatische Zusammensetzung ferner eines oder mehrere der Östrogene oder Phytoöstrogene umfasst, ausgewählt aus der Gruppe bestehend aus Diethylstilbestrol, Hexoestrol, Estradiol, Östron, Estriol, Nilestriol, Ethinyloestradiol, Chinestrol, Mestranol, Promestrien, Daidzin, Daidzein, Glycitein, Puerarin, Coumestrol, Genistein, Equol, Apigenin, Genistin, Genisteol, Biochanin, Coumestrol, Formononetin, Resveratrol, Secoisolariciresinol und Lignan; wobei der Gesamtgehalt im Bereich von 0,001-1,00 % (w/w) liegt.

13. Bakteriostatische Vaginalzusammensetzung nach Anspruch 9, **dadurch gekennzeichnet, dass** die vaginale bakteriostatische Zusammensetzung ferner eines oder mehrere der Saccharide umfasst, ausgewählt aus der Gruppe bestehend aus Glucose, Fructose, Mannose, Galactose, Maltose, Isomaltose, Saccharose, Isomaltulose, Lactose, Lactulose, Trehalose, Cellobiose, Melibiose, Gentiobiose, 1-Kestose, Nystose, 1F-Fructofuranosylnystose, Isomaltotriose, Isomaltotetraose, Isomaltopentaose, Gentiooligosaccharid, Raffinose, Panose, Maltooligosaccharid, Palatinose-Oligosaccharid, Oligofructose, Glucomannan, Galactooligosaccharid, Dextrin, Stärke und Glykogen; wobei der Gesamtgehalt im Bereich von 0,01-20,00 % (w/w) oder 0,10-2,00 % (w/w) liegt.

14. Vaginale bakteriostatische Zusammensetzung nach Anspruch 9, **dadurch gekennzeichnet, dass** die vaginale bakteriostatische Zusammensetzung zur Wiederherstellung und/oder Aufrechterhaltung einer normalen Vaginalflora und/oder zur Wiederherstellung und/oder Aufrechterhaltung einer normalen Vaginalmikroökologie und/oder zur Wiederherstellung und/oder Aufrechterhaltung einer normalen Vaginalsäure, und/oder zur Reinigung und Pflege der Vagina und/oder Vulva und/oder zur Reduzierung und/oder Beseitigung von vaginalem Juckreiz, Schmerzen, Trockenheit, Irritation und Dyspareunie, und/oder zum Reduzieren und/oder Beseitigen von abnormalem Vaginalausfluss und unangenehmem Geruch des Vaginalausflusses, verwendet wird.

15. Vaginale bakteriostatische Zusammensetzung nach Anspruch 9, **dadurch gekennzeichnet, dass** die beschriebene vaginale bakteriostatische Zusammensetzung zur Vorbeugung und/oder Behandlung eines Ungleichgewichts der Vaginalflora, bakterieller Vaginose, aerober Vaginitis, zytolytischer Vaginose, vulvovaginaler Candidiasis und/oder atrophischer Vaginitis verwendet wird.

## Revendications

1. Composition bactériostatique, **caractérisée en ce qu'**elle comprend :
(1) un ou plusieurs des acides gras et/ou leurs sels choisis dans le groupe constitué par l'acide acétique, l'acide propionique, l'acide butyrique, l'acide isobutyrique, l'acide valérique, l'acide hexanoïque, l'acide heptanoïque, l'acide caprylique, l'acide nonanoïque, l'acide caprique, l'acide undécylique, l'acide undécylénique, l'acide laurique, et leurs sels ; la teneur totale des ingrédients (1) tels que décrits, calculée en acide gras, s'inscrit dans la plage de 0,001 à 3,00 % (p/p) ;
(2) un ou plusieurs des acides dicarboxyliques et/ou leurs sels choisis dans le groupe constitué par l'acide glutarique, l'acide adipique, l'acide pimélique et leurs sels ; la teneur totale des ingrédients (2) tels que décrits, calculée en acide dicarboxylique, s'inscrit dans la plage de 0,05 à 5,00 % (p/p) ;
(3) un ou plusieurs des alcools aromatiques choisis dans le groupe constitué par l'alcool benzylique, l'alcool phénéthylique, le phénoxyéthanol et l'alcool cinnamylique ; la teneur totale des ingrédients (3) tels que décrits s'inscrit dans la plage de 0,03 à 1,00 % (p/p) ;
(4) un ou plusieurs des acides carboxyliques aromatiques et/ou leurs sels choisis dans le groupe constitué par l'acide benzoïque, l'acide p-hydroxybenzoïque, l'acide p-méthoxybenzoïque, l'acide salicylique, l'acide cinnamique, l'acide gentianique, l'acide caféique et leurs sels ; la teneur totale des ingrédients (4) telle que décrite, calculée en acide carboxylique aromatique, s'inscrit dans la plage de 0,03 à 1,00 % (p/p) ;
la composition bactériostatique est sous une forme galénique choisie dans le groupe constitué par des solutions aqueuses, des gels solubles dans l'eau, des mousses, des sprays, des pommades, des poudres, des films, des gélules, des suppositoires et des comprimés.

2. Composition bactériostatique selon la revendication 1, **caractérisée en ce que** la teneur totale des ingrédients (1) dans la composition bactériostatique telle que décrite s'inscrit dans la plage de 0,001 à 2,00 % (p/p) ; et/ou la teneur totale des ingrédients (2) telle que décrite s'inscrit dans la plage de 0,10 à 3,50 % (p/p) ; et/ou la teneur totale des ingrédients (3) telle que décrite s'inscrit dans la plage de 0,03 à 0,70 % (p/p) ; et/ou la teneur totale des ingrédients (4) telle que décrite s'inscrit dans la plage de 0,03 à 0,50 % (p/p).

3. Composition bactériostatique selon la revendication 1, **caractérisée en ce que** la composition bactériostatique telle que décrite comprend en outre un ou plusieurs des médicaments antibactériens choisis dans le groupe constitué par le métronidazole, le tinidazole, l'ornidazole, la gentamicine, la tobramycine, l'amikacine, la sisomicine, la nétilmycine, la ciprofloxacine, l'ofloxacine, la lévofloxacine, le nifuratel, la nifuroxime, la furaciline, la furazolidone, la furantoïne, la sulfadiazine argentique, le sulfacetamide de sodium, le clotrimazole, le fluconazole, le miconazole, le kétoconazole, la naftifine, la terbinafine, l'amphotéricine B, la nystatine, la lévorine et la natamycine.

4. Composition bactériostatique selon la revendication 1, **caractérisée en ce que** la composition bactériostatique telle que décrite est sous une forme galénique choisie dans le groupe constitué par des solutions aqueuses, des gels solubles dans l'eau, des mousses, des sprays ou des pommades dont la valeur de pH s'inscrit dans la plage de 3,1 à 4,8, ou de 3,6 à 4,6, ou de 3,8 à 4,4.

5. Composition bactériostatique selon la revendication 1, **caractérisée en ce que** la composition bactériostatique telle que décrite peut être un produit thérapeutique, un ingrédient actif de celui-ci, et un antiseptique de celui-ci ; la forme de produit thérapeutique est l'un des groupes suivants : les médicaments, les désinfectants, les agents antibactériens, les agents bactériostatiques, les microbicides topiques, les modulateurs de flore, les modulateurs micro-écologiques, les modulateurs de micro-environnement, les modulateurs microbiens, les fournitures médicales jetables et similaires, ou des composants de dispositifs médicaux, des composants de dispositifs pharmaceutiques, des composants de dispositifs de désinfection et des composants de dispositifs à usage vaginal ; et/ou la composition bactériostatique telle que décrite peut être un produit non thérapeutique, un ingrédient actif de celui-ci, et un antiseptique de celui-ci ; la forme de produit non thérapeutique est l'un des groupes suivants : les produits de soins de santé, les produits d'hygiène, les produits de nettoyage et de soins personnels, les produits cosmétiques, les produits d'hygiène jetables, les produits de nettoyage, les produits d'usage quotidien, les produits de soin micro-écologiques, les déodorants, les lubrifiants, les humectants, les lotions, les agents nettoyants, les produits de soin corporel, les agents antipruritiques et les agents rafraîchissants, ou les composants de serviettes hygiéniques, de protections hygiéniques et de tampons.

6. Composition bactériostatique selon la revendication 1, **caractérisée en ce que** la composition bactériostatique telle que décrite est une composition bactériostatique vaginale, dans laquelle la composition bactériostatique vaginale comprend les ingrédients suivants :
(1) un ou plusieurs des acides gras et/ou leurs sels choisis dans le groupe constitué par l'acide acétique, l'acide propionique, l'acide butyrique, l'acide valérique, l'acide hexanoïque, l'acide heptanoïque, l'acide caprylique, l'acide non-anoïque, l'acide caprique, l'acide undécylique, l'acide undécylénique, l'acide laurique, et leurs sels ; la teneur totale des ingrédients (1), calculée en acide gras, s'inscrit dans la plage de 0,001 à 2,00 % (p/p) ;
(2) un ou plusieurs des acides dicarboxyliques et/ou leurs sels choisis dans le groupe constitué par l'acide glutarique, l'acide adipique, l'acide pimélique et leurs sels ; la teneur totale des ingrédients (2), calculée en acide dicarboxylique, s'inscrit dans la plage de 0,50 à 2,50 % (p/p) ;
(3) un ou plusieurs des alcools aromatiques choisis dans le groupe constitué par l'alcool benzylique, l'alcool phénéthylique, le phénoxyéthanol et l'alcool cinnamylique ; la teneur totale des ingrédients (3) s'inscrit dans la plage de 0,05 à 0,60 % (p/p) ;
(4) un ou plusieurs des acides carboxyliques aromatiques et/ou leurs sels choisis dans le groupe constitué par l'acide benzoïque, l'acide p-hydroxybenzoïque, l'acide p-méthoxybenzoïque, l'acide salicylique, l'acide cinnamique, l'acide gentianique, l'acide caféique et leurs sels ; la teneur totale des ingrédients (4), calculée en acide carboxylique aromatique, s'inscrit dans la plage de 0,05 à 0,25 % (p/p) ; la composition bactériostatique vaginale est sous une forme galénique choisie dans le groupe constitué par des solutions aqueuses, des gels solubles dans l'eau, des mousses, des sprays, des pommades, des poudres, des films, des gélules, des suppositoires et des comprimés.

7. Procédé d'inhibition de micro-organismes nocifs à l'extérieur de l'organisme, **caractérisé en ce qu'**il comporte les étapes d'utilisation d'une composition bactériostatique telle que décrite dans les revendications 1 à 5.

8. Procédé selon la revendication 7, **caractérisé en ce que** les micro-organismes nocifs tels que décrits font référence à un ou plusieurs parmi *Candida, Staphylococcus aureus, Escherichia coli, Pseudomonas aeruginosa, Gardnerella, Prevotella, Mobiluncus,* et *Aspergillus niger.*

9. Composition bactériostatique vaginale destinée à être utilisée dans le traitement et/ou la prévention d'infections vaginales par modulation de la flore vaginale, **caractérisée en ce que** la composition bactériostatique vaginale comprend les ingrédients suivants :
(1) un ou plusieurs des acides gras et/ou leurs sels choisis dans le groupe constitué par l'acide propionique, l'acide butyrique, l'acide valérique, l'acide hexanoïque, l'acide heptanoïque, l'acide caprylique, l'acide non-anoïque, l'acide caprique, l'acide undécylique, l'acide undécylénique, l'acide laurique, et leurs sels ; dont la teneur totale, calculée en acide gras, s'inscrit dans la plage de 0,001 à 2,00 % (p/p) ; et
(2) un ou plusieurs des acides dicarboxyliques et/ou leurs sels choisis dans le groupe constitué par l'acide glutarique, l'acide adipique, l'acide pimélique et leurs sels ; dont la teneur totale, calculée en acide dicarboxylique, s'inscrit dans la plage de 0,50 à 2,50 % (p/p) ;
dans laquelle la composition bactériostatique vaginale est sous une forme galénique choisie dans le groupe constitué par des solutions aqueuses, des gels solubles dans l'eau, des mousses, des sprays, des pommades, des poudres, des films, des gélules, des suppositoires et des comprimés ;
dans laquelle la modulation de la flore vaginale fait référence à au moins l'un des éléments suivants : inhibition de la flore vaginale anormale, restauration et/ou maintien des lactobacilles vaginaux.

10. Composition bactériostatique vaginale selon la revendication 9, **caractérisée en ce que** la composition bactériostatique vaginale est utilisée pour inhiber la flore vaginale anormale, dans laquelle l'inhibition de la flore vaginale anormale fait référence à l'inhibition d'un ou plusieurs de *Candida, Staphylococcus, Streptococcus, Gardnerella, Escherichia coli, Veillonella parvula, Prevotella* et *Mobiluncus.*

11. Composition bactériostatique vaginale selon la revendication 9, **caractérisée en ce que** la composition bactériostatique vaginale comprend en outre un ou plusieurs des acides carboxyliques aromatiques et/ou leurs sels choisis dans le groupe constitué par l'acide benzoïque, l'acide p-hydroxybenzoïque, l'acide p-méthoxybenzoïque, l'acide salicylique, l'acide cinnamique, l'acide gentianique, l'acide caféique, et leurs sels ; dont la teneur totale, calculée en acide carboxylique aromatique, s'inscrit dans la plage de 0,05 à 0,25 % (p/p).

12. Composition bactériostatique vaginale selon la revendication 9, **caractérisée en ce que** la composition bactériostatique vaginale comprend en outre un ou plusieurs des œstrogènes ou phytoœstrogènes choisis dans le groupe constitué par le diéthylstilbestrol, l'hexoestrol, l'estradiol, l'estrone, l'estriol, le nilestriol, l'éthinylœstradiol, le quinestrol, le mestranol, promestriène, la daidzine, la daidzéine, la glycitéine, la puérarine, le coumestrol, la génistéine, l'équol, l'apigénine, la génistéine, le génisteol, la biochanine, le coumestrol, la formonétine, le resvératrol, le sécoisolaricirésinol, et le lignane ; dont la teneur totale s'inscrit dans la plage de 0,001 à 1,00 % (p/p).

13. Composition bactériostatique vaginale selon la revendication 9, **caractérisée en ce que** la composition bactériostatique vaginale comprend en outre un ou plusieurs des saccharides choisis dans le groupe constitué par le glucose, le fructose, le mannose, le galactose, le maltose, l'isomaltose, le saccharose, l'isomaltulose, le lactose, le lactulose, le tréhalose, le cellobiose, le mélibiose, le gentiobiose, le 1-kestose, le nystose, le 1F-fructofuranosylnystose, l'isomaltotriose, l'isomaltotétraose, l'isomaltopentaose, le gentiooligosaccharide, le raffinose, le panose, le maltooligosaccharide, le palatinose-oligosaccharide, l'oligofructose, le glucomannane, le galactooligosaccharide, la dextrine, l'amidon et le glycogène ; dont la teneur totale s'inscrit dans la plage de 0,01 à 20,00 % (p/p) ou de 0,10 à 2,00 % (p/p).

14. Composition bactériostatique vaginale selon la revendication 9, **caractérisée en ce que** la composition bactériostatique vaginale est utilisée pour restaurer et/ou maintenir une flore vaginale normale, et/ou pour restaurer et/ou maintenir une microécologie vaginale normale, et/ou pour restaurer et/ou maintenir une acidité vaginale normale, et/ou pour nettoyer et prendre soin du vagin et/ou de la vulve, et/ou pour réduire et/ou éliminer le prurit, la douleur, la sécheresse, l'irritation et la dyspareunie, et/ou pour réduire et/ou éliminer les écoulements vaginaux anormaux et l'odeur désagréable des écoulements vaginaux.

15. Composition bactériostatique vaginale selon la revendication 9, **caractérisée en ce que** la composition bactériostatique vaginale telle que décrite est utilisée pour prévenir et/ou traiter le déséquilibre de la flore vaginale, la vaginose bactérienne, la vaginite aérobie, la vaginose cytolytique, la candidose vulvovaginale et/ou la vaginite atrophique.
